# EUROPEAN PATENT APPLICATION

(11) **EP 4 194 453 A1**
(43) Date of publication of application: **14.06.2023**
(21) Application number: 21213135.3
(22) Date of filing: 08.12.2021
(51) Int. Cl.: C07D 413/04, C07D 413/14, C07D 417/04, C07D 417/14, A01N 43/82

(54) **PYRAZINE COMPOUNDS FOR THE CONTROL OF INVERTEBRATE PESTS**

(71) Applicant: BASF SE, 67056 Ludwigshafen am Rhein (DE)
(72) Inventor: Pedroni, Julia, 67056 Ludwigshafen am Rhein (DE); Gilberg, Erik, 50672 Koeln (DE); Koerber, Karsten, 67056 Ludwigshafen am Rhein (DE); Huwyler, Nikolas, 67063 Ludwigshafen (DE)
(74) Representative: BASF IP Association

(57) **Abstract**

The invention relates to compounds of formula I 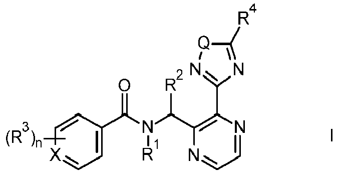 wherein the variables have the meanings as defined in the specification, to compositions comprising them, to active compound combinations comprising them, and to their use for protecting growing plants and animals from attack or infestation by invertebrate pests, furthermore, to seed comprising such compounds.

## Description

The invention relates to compounds of formula I 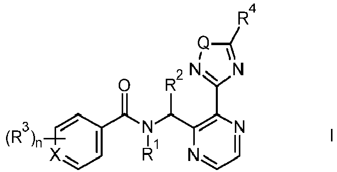 wherein
- R¹: is H, OH, C₁₋C₆-alkyl, C₁-C₆-haloalkyl, C₃-C₆-cycloalkyl, C₃-C₆-halocycloalkyl, C₁-C₅-alkoxy, C₁-C₄-alkyl-C₃-C₆-cycloalkyl, C₁-C₄-alkyl-C₃-C₆-halocycloalkyl, which groups are unsubstituted, or partially or fully substituted with R¹¹;
- R¹¹: is halogen, CN, NO₂, NR¹²R¹³, C(O)NH₂, C(S)NH₂, C(O)OH, OR¹⁴, Si(CH₃)₃; C₁-C₆-alkyl; C₁-C₆-haloalkyl; C₂-C₆-alkenyl; C₂-C₆-haloalkenyl; C₂-C₆-alkynyl; C₂-C₆-haloalkynyl; C₃-C₄-cycloalkyl-C₁-C₂-alkyl, which ring is unsubstituted or substituted with 1 or 2 halogen; 3- to 6-membered heterocyclyl, 5- or 6-membered hetaryl, or phenyl, which rings are unsubstituted or substituted with halogen, C₁-C₃-haloalkyl, and/or CN;
- R¹², R¹³: are independently from each other H, C₁-C₄-alkyl, C₁-C₄-alkoxy, C₁-C₄-haloalkoxy, C₁-C₄-haloalkyl, C₃-C₆-cycloalkyl, C(O)-C₁₋C₄-alkyl, C(O)-C₁-C₄-haloalkyl, C(O)-C₃-C₄-cycloalkyl, C(O)-C₃-C₄-halocycloalkyl, C(O)NH-C₁-C₄-alkyl, C(O)NH-C₁-C₄-haloalkyl, C(O)N(C₁-C₄-alkyl)-C₁-C₄-alkyl, C(O)N(C₁-C₄-haloalkyl)-C₁-C₄-alkyl, C(O)N(C₁-C₄-haloalkyl)-C₁-C₄-haloalkyl, C(O)NH-C₁-C₄-alkoxy, C(O)NH-C₁-C₄-haloalkoxy, C(O)NH-C₁-C₄-alkoxy-C₁-C₄-alkyl, C(O)NH-C₁-C₄-alkoxy-C₁-C₄-haloalkyl; C(O)NH-phenyl, C(O)NH-3-6-membered heterocyclyl or 5- or 6-membered hetaryl, C(O)NH-C₁-C₄-alkyl-phenyl, C(O)NH-C₁-C₄-alkyl-3-6-membered heterocyclyl or 5- or 6-membered hetaryl, which rings are unsubstituted or substituted with halogen, C₁-C₃-haloalkyl, and/or CN; S(O)ₘ-C₁-C₄-haloalkyl, S(O)ₘ-C₃-C₆-cycloalkyl, S(O)ₘ-C₃-C₃-halocycloalkyl; 3- to 6-membered heterocyclyl, 5- or 6-membered hetaryl, or phenyl, which rings are unsubstituted or substituted with halogen, C₁-C₃-haloalkyl, and/or CN;
- or R¹² and R¹³: together with the nitrogen atom they are bound to form a 3-6 membered saturated, partially or fully unsaturated heterocycle, which may contain 1 or 2 additional heteroatoms selected from N, O, and S, wherein S may be oxidized, and which heterocycle is unsubstituted or substituted with R³;
- or R¹² and R¹³: together with the nitrogen atom they are bound to form a group N=S(=O)R^{14a}R^{14b}, wherein R^{14a} and R^{14b} are defined as R¹⁴;
- R¹⁴: is H, C₁-C₄-alkyl, C₁-C₄-haloalkyl, C₃-C₆-cycloalkyl, C₃-C₆-halocycloalkyl, C₃-C₄-cycloalkyl-C₁-C₂-alkyl, C₃-C₄-halocycloalkyl-C₁-C₂-alkyl, C(O)-Cr₁₋C₄-alkyl, C(O)-C₁-C₄-haloalkyl, C(O)-C₃-C₄-cycloalkyl, C(O)-C₃-C₄-halocycloalkyl, or phenyl which is unsubstituted or partially or fully substituted with R³;
- R²: m is 0, 1, or 2; is H, CN, C₁-C₃-alkyl, C₁-C₃-haloalkyl, C₂-C₃-alkenyl, or C₂-C₃-alkynyl;
- R³: is halogen, CN, NO₂, C₁-C₄-alkyl, C₃-C₆-cycloalkyl, C₁-C₆-haloalkyl, C₁-C₆-halocycloalkyl, OR¹⁴, S(O)ₘ-R¹⁴; wherein rings are unsubstituted or substituted with R¹¹; n is 0, 1, 2, or 3;
- R⁴: is H, halogen, C₁-C₆-alkyl, C₃-C₆-cycloalkyl, C₁-C₆-haloalkyl, C₁-C₆-halocycloalkyl, C₂-C₄-alkenyl, C₂-C₄-haloalkenyl, C₂-C₄-alkynyl, each optionally substituted by R⁴¹; S(O)ₘ-C₁-C₄-alkyl, S(O)ₘ-C₁-C₄-haloalkyl, S(O)ₘ-C₃-C₆-cycloalkyl, S(O)ₘ-C₃-C₆-halocycloalkyl, S(O)₂NR¹²R¹³, NR¹²R¹³, C(O)NR¹²R¹³, C(O)OR¹⁴, 3- to 6-membered heterocyclyl, 5- or 6-membered hetaryl, or phenyl, which rings are unsubstituted or substituted with R³;
- R⁴¹ is H, OR¹⁵, NR¹²R¹³,: C₁-C₄-alkyl, C₁-C₄-haloalkyl, C₃-C₆-cycloalkyl, C(O)-Cr₁₋C₄-alkyl, C(O)-C₁-C₄-haloalkyl, C(O)-C₃-C₄-cycloalkyl, C(O)-C₃-C₄-halocycloalkyl, C(O)NH-C₁-C₄-alkyl, C(O)NH-C₁-C₄-haloalkyl, C(O)N(C₁-C₄-alkyl)-C₁-C₄-alkyl, C(O)N(C₁-C₄-haloalkyl)-C₁-C₄-alkyl, C(O)N(C₁-C₄-haloalkyl)-C₁-C₄-haloalkyl, C(O)NH-C₁-C₄-alkoxy, C(O)NH-C₁-C₄-haloalkoxy, C(O)NH-C₁-C₄-alkoxy-C₁-C₄-alkyl, C(O)NH-C₁-C₄-alkoxy-C₁-C₄-haloalkyl; C(O)NH-phenyl, C(O)NH-3-6-membered heterocyclyl or 5- or 6-membered hetaryl, C(O)NH-C₁-C₄-alkyl-phenyl, C(O)NH-C₁-C₄-alkyl-3-6-membered heterocyclyl or 5- or 6-membered hetaryl, which rings are unsubstituted or substituted with halogen, C₁-C₃-haloalkyl, and/or CN; S(O)ₘ-C₁-C₄-haloalkyl, S(O)ₘ-C₃-C₄-cycloalkyl, S(O)ₘ-C₃-C₄-halocycloalkyl; 3- to 6-membered heterocyclyl, 5- or 6-membered hetaryl, or phenyl, which rings are unsubstituted or substituted with halogen, C₁-C₃-haloalkyl, and/or CN;
- R¹⁵: is H, C₁-C₄-alkyl, or C₁-C₄-haloalkyl, C₃-C₆-cycloalkyl, C₁-C₆-halocycloalkyl, which carbon chains are unsubstituted or partially or fully substituted with R¹¹; or 3- to 6-membered heterocyclyl, 5- or 6-membered hetaryl, or phenyl, which rings are unsubstituted or substituted with R³;
- Q: is O or S;
and the N-oxides, stereoisomers, and agriculturally or veterinarily acceptable salts thereof.

The invention also provides agricultural compositions comprising at least one compound of formula I, a stereoisomer thereof and/or an agriculturally acceptable salt thereof and at least one liquid and/or solid carrier, especially at least one inert liquid and/or solid agriculturally acceptable carrier.

The invention also provides a veterinary composition comprising at least one compound of formula I, a stereoisomer thereof and/or a veterinarily acceptable salt thereof and at least one liquid and/or solid carrier, especially at least one inert veterinarily liquid and/or solid acceptable carrier.

The invention also provides a method for controlling invertebrate pests which method comprises treating the pests, their food supply, their habitat or their breeding ground or a cultivated plant, plant propagation materials (such as seed), soil, area, material or environment in which the pests are growing or may grow, or the materials, cultivated plants, plant propagation materials (such as seed), soils, surfaces or spaces to be protected from pest attack or infestation with a pesticidally effective amount of a compound of formula I or a salt thereof as defined herein.

The invention also relates to plant propagation material, in particular seed, comprising at least one compound of formula I and/or an agriculturally acceptable salt thereof.

The invention further relates to a method for treating or protecting an animal from infestation or infection by parasites which comprises bringing the animal in contact with a parasiticidally effective amount of a compound of formula I or a veterinarily acceptable salt thereof. Bringing the animal in contact with the compound I, its salt or the veterinary composition of the invention means applying or administering it to the animal.

WO2021068179, WO2021069575, and WO2021037614 describe structurally closely related active compounds. These compounds are mentioned to be useful for combating invertebrate pests.

Nevertheless, there remains a need for highly effective and versatile agents for combating invertebrate pests. It is therefore an object of the invention to provide compounds having a good pesticidal activity and showing a broad activity spectrum against a large number of different invertebrate pests, especially against difficult to control pests, such as insects.

It has been found that these objects can be achieved by compounds of formula I as depicted and defined below, and by their stereoisomers, salts, tautomers and N-oxides, in particular their agriculturally acceptable salts.

Compounds I with R⁴ being different from H can be obtained by alkylation of a compound II (corresponding to formula I with R¹ = H) with a suitable alkylating agent III (e.g. alkyl halide). In formula III R¹ has the meaning as in formula I, and Y is a nucleophilic leaving group, such as a halide, preferably Br or CI. The alkylation can be effected under standard conditions known from literature.

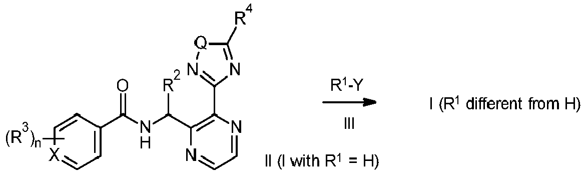

This transformation is usually carried out at temperatures of from -10°C to +110°C, preferably from 0°C to 25°C, in an inert solvent and in the presence of a base [cf. WO 2002100846, S. M. Somagond, Heterocycl. Commun. 2017, 317].

The starting materials are generally reacted with one another in equimolar amounts. In terms of yield, it may be advantageous to employ an excess of III, based on II.

Compounds I with Q=O (formula I.O) can be prepared from the corresponding amidoxime precursors IV by cyclisation with a suitable reagent, such as V or VI.

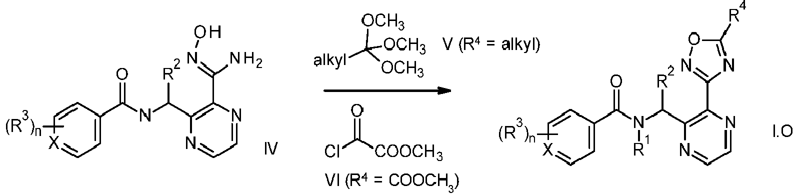

Reaction of compounds IV with an orthoester (V) leads to compounds I.O in which R⁴ is an alkyl group, e.g. as described in WO2009154557. This transformation is usually carried out neat in excess V and in the presence of 0.05 to 1.0 equivalents of an acid, such as p-toluenesulfonic acid (TsOH), ZnCl₂, or BF₃ diethyl etherate, and at temperatures between 0°C and 120°C, preferably between 25°C and 80°C. In terms of yield, it is generally advantageous to employ an excess of V based on IV.

Alternatively, reaction of compounds IV with alkyl 2-chloro-2-oxo-acetates, such as VI, delivers compounds I.O in which R⁴ is an alkyl ester moiety, e.g. as described in J. Med. Chem. 2008, 4392. This transformation is usually carried out in an inert solvent such as 1,4-dioxane, tetrahydrofuran (THF), 2-methyl tetrahydrofuran, chloroform, dichloroethane, dichloromethane, or in a mixture of the aforementioned solvents, and in the presence of 1 to 3 equivalents of a base, such as triethylamine, diisopropylethylamine, pyridine, 2,6-lutidine, 2,4,6-collidine, or alternatively using the base as the solvent, and at temperatures between 0°C and 170°C, preferably between 25°C and the boiling point of the solvent. In terms of yield, it is generally advantageous to employ an excess of VI based on IV.

Furthermore, compounds I in which R⁴ is C(O)NR¹²R¹³ can be obtained from compounds I in which R⁴ is C(O)OR¹⁴ by reaction with the corresponding amine HNR¹²R¹³. This transformation is usually carried out in a solvent such as ethanol, methanol, or water, or in a mixture of the aforementioned solvents, either using a stoichiometric amount of the amine HNR¹²R¹³ in the presence of 1 to 3 equivalents of a suitable base, such as triethylamine, diisopropylamine, or pyridine, or using 2 to 4 equivalents of the amine HNR¹²R¹³, and at temperatures between 10°C and 140°C, preferably between 25°C and the boiling point of the solvent, for example as described in J. Med. Chem. 2008, 4377.

Compounds IV can be prepared from compounds VII by reaction with hydroxylamine or a suitable salt thereof.

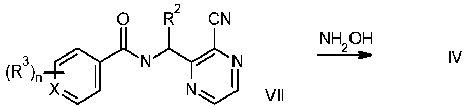

This transformation is usually carried out in a solvent such as (or solvent mixture composed of) methanol, ethanol, 2-propanol, and/or water, optionally in the presence of 1 to 2 equivalents of a base such as NaOH, KOH, Na₂CO₃, K₂CO₃, or CS₂CO₃, and at temperatures ranging from 0°C to the boiling point of the solvent or solvent mixture (cf. Tiemann, Chem. Ber. 1884, 17, 126; Gobis et al., Acta Pol Pharm (Drug Research) 2006, 39).

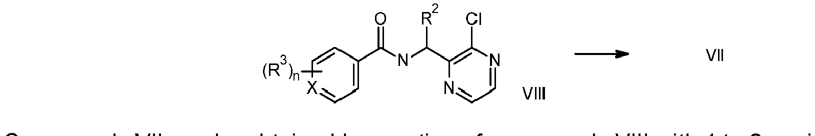

Compounds VII can be obtained by reaction or compounds VIII with 1 to 2 equivalents of Zn(CN)₂ in the presence of 0.01 to 0.2 equivalents of a transition-metal complex, such as palladium diacetate, palladium trifluoroacetate, tris(dibenzylideneacetone)dipalladium, tetrakis(triphenylphosphine)palladium, or NiCl₂, and 0.01 to 0.2 equivalents of a ligand, such as 2-[di(tert-butyl)phosphino]-1,1'-binaphthyl, 1,1-bis(diphenylphosphino)ferrocene, or BINAP, as catalysts, and 0.1 to 0.5 equivalents of zinc powder or zinc flakes as a cocatalyst, and optionally with 1 to 3 equivalents of a base, such as 4-(dimethylamino)pyridine or sodium tert-butoxide, in a solvent such as dimethylformamide (DMF), N,N-dimethylacetamide (DMA), or acetonitrile, at temperatures between 25°C and 150°C, preferably between 25°C and the boiling point of the solvent (cf. A. Littke et al., Org. Lett. 2007, 1711). Compounds VIII are known from WO2020070049.

Compounds I with Q=S can be prepared by acylation of the corresponding amines IX:

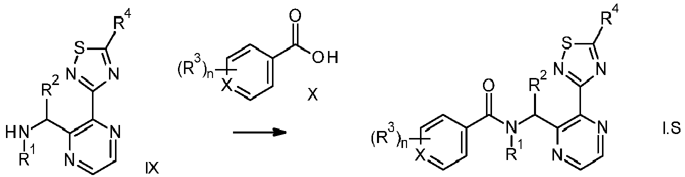

Such compounds of formula I correspond to formula I.S.

This transformation is usually carried out at temperatures of from -20°C to 50°C, preferably from 0°C to 25°C, in an inert solvent, in the presence of a peptide coupling reagent and optionally in the presence of a base [cf. A. EI-Faham, Chem. Rev. 2011, 6557], or in two steps by preparation of an intermediate acyl chloride from X under conditions known from literature, e.g. by reaction with SOCl₂ or oxalyl chloride in DMF (cf. Schaefer et al, Organic Syntheses 1929, 32), followed by reaction with IX in the presence of a base, optionally under Schotten-Baumann conditions (Baumann, Chem. Ber. 1886, 3218). Suitable peptide coupling reagents are, e.g., dicyclohexylcarbodiimide, diisopropylcarbodiimide, 1-ethyl-3-(3'-dimethylaminopropyl)carbodiimide hydrochloride, or chloro-N,N,N',N'-tetramethylformamidinium hexafluorophosphate, which are commonly used together with catalytic, stoichiometric, excess amounts of additives, such as 1-hydroxybenzotriazole, 1-hydroxy-7-aza-benzotriazole, 4-(dimethylamino)pyridine, and/or 1-methylimidazole.

Suitable solvents are halogenated hydrocarbons, such as dichloromethane (DCM) or 1,2-dichloroethane, ethers, such as diethylether, THF, or 1,4-dioxane, or high-boiling solvents such as dimethylformamide (DMF), preferably DCM or DMF, or in aqueous media.

Suitable bases are, in general, inorganic compounds, such as alkali metal and alkaline earth metal hydroxides, such as LiOH, NaOH, KOH, or Ca(OH)₂, alkali metal and alkaline earth metal carbonates, such as Na₂CO₃, K₂CO₃, or CS₂CO₃, alkali metal bicarbonates, such as NaHCO₃, or organic bases, for example tertiary amines, such as triethylamine, diisopropylethylamine, N-methylpiperidine, or basic aromatic rings, such as pyridine, 2,4,6-collidine, 2,6-lutidine, or 4-(dimethylamino)pyridine, or bicyclic amines, such as 1,8-diazabicylo[5.4.0]undec-7-ene (DBU), 1,5-diazabicyclo[4.3.0]non-5-ene (DBN), or 1,4-diazabicyclo[2.2.2]octane (DABCO). Particular preference is given to triethylamine, diisopropylethylamine, and NaOH. The bases are generally employed in stoichiometric or excess amounts; however, they can also be used in catalytic amounts or, if appropriate, as the solvent.

The starting materials are generally reacted with one another in equimolar amounts. In terms of yield, it may be advantageous to employ an excess of X based on IX.

Compounds IX can be prepared by reductive amination of a compound XI:

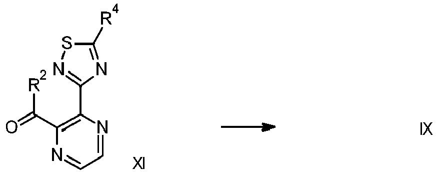

This transformation is usually carried out at temperatures of from 0°C to 130°C, preferably from 20°C to 70°C, generally in alcoholic and/or aqueous media and in the presence of a reagent and a reducing agent [cf. WO2021037614]. Suitable solvents are alcohols, such as methanol, ethanol, n-propanol, 2-propanol, or n-butanol, or water, preferably methanol. It is also possible to use mixtures of the aforementioned solvents. Suitable reagents are ammonium acetate (NH₄Ac), ammonium formate, NH₄OH, NH₄Cl, ammonia, or primary amines R¹NH₂. Suitable reducing agents are NaBH₃CN, sodium triacetoxyborohydride, or NaBH₄. Preference is given to ammonium acetate and NaBH₃CN, resp.

Compounds XI can be obtained by reaction of XII with an appropriately substituted nitrile XIII, in which W can be, for example, CH₂CO₂Et or CO₂Et, with subsequent conversion of W into the desired substituent R⁴ following standard methods known from literature.

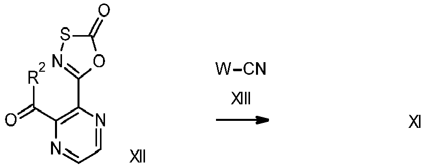

This transformation is generally carried out in a high-boiling hydrocarbon solvent, such as decaline, or aromatic solvent, such as 1,2-dichlorobenzene, in a sealed flask and at high temperatures ranging from 120°C to 200°C, as described in WO2013031694.

Compounds XII can be prepared from compounds XIV by cyclisation with (chlorocarbonyl)sulfenyl chloride (XV) as described in Bioorg. Med. Chem. Lett. 2013, 1424.

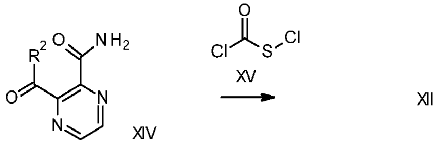

Compounds XIV can be prepared from compounds XVI by reaction with ammonia or an ammonium salt, whereas the latter are known from Bioorg. Med. Chem. Lett. 2014, 4490.

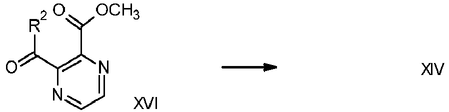

The reaction mixtures are worked up in a customary manner, for example by mixing with water, extracting with an appropriate organic solvent, separating the phases and, if appropriate, chromatographic purification of the crude products. Some of the intermediates and end products are obtained in the form of colourless or slightly brownish viscous oils which are purified or freed from volatile components under reduced pressure and at moderately elevated temperature. If the intermediates and end products are obtained as solids, purification can also be carried out by recrystallization or digestion.

If individual compounds I cannot be obtained by the routes described above, they can be prepared by derivatization of other compounds I.

However, if the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the pest to be controlled.

The organic moieties groups mentioned in the above definitions of the variables are - like the term halogen - collective terms for individual listings of the individual group members. The prefix Cₙ-Cₘ indicates in each case the possible number of carbon atoms in the group.

The term "partially or fully substituted" by a radical means that in general the group is substituted with same or different radicals.

The term "halogen" denotes in each case fluorine, bromine, chlorine, or iodine, in particular fluorine, chlorine, or bromine.

The term "alkyl" as used herein and in the alkyl moieties of alkylamino, alkylcarbonyl, alkylthio, alkylsulfinyl, alkylsulfonyl and alkoxyalkyl denotes in each case a straight-chain or branched alkyl group having usually from 1 to 10 carbon atoms, frequently from 1 to 6 carbon atoms, preferably 1 to 4 carbon atoms, more preferably from 1 to 3 carbon atoms. Examples of an alkyl group are methyl (Me), ethyl (Et), n-propyl (n-Pr), iso-propyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl.

The term "haloalkyl" as used herein and in the haloalkyl moieties of haloalkylcarbonyl, haloalkoxycarbonyl, haloalkylthio, haloalkylsulfonyl, haloalkylsulfinyl, haloalkoxy and haloalkoxyalkyl, denotes in each case a straight-chain or branched alkyl group having usually from 1 to 10 carbon atoms, frequently from 1 to 6 carbon atoms, preferably from 1 to 4 carbon atoms, wherein the hydrogen atoms of this group are partially or totally replaced with halogen atoms. Preferred haloalkyl moieties are selected from C₁-C₄-haloalkyl, more preferably from C₁-C₃-haloalkyl or C₁-C₂-haloalkyl, in particular from C₁-C₂-fluoroalkyl such as fluoromethyl, difluoromethyl, trifluoromethyl, 1-fluoroethyl, 2-fluoroethyl, 2,2-difluoroethyl, 2,2,2-trifluoroethyl, pentafluoroethyl, and the like.

The term "alkoxy" as used herein denotes in each case a straight-chain or branched alkyl group which is bonded via an oxygen atom and has usually from 1 to 10 carbon atoms, frequently from 1 to 6 carbon atoms, preferably 1 to 4 carbon atoms. Examples of an alkoxy group are methoxy, ethoxy, n-propoxy, iso-propoxy, n-butyloxy, 2-butyloxy, iso-butyloxy, tert.-butyloxy, and the like.

The term "alkoxyalkyl" as used herein refers to alkyl usually comprising 1 to 10, frequently 1 to 4, preferably 1 to 2 carbon atoms, wherein 1 carbon atom carries an alkoxy radical usually comprising 1 to 4, preferably 1 or 2 carbon atoms as defined above. Examples are CH₂OCH₃, CH₂-OC₂H₅, 2-(methoxy)ethyl, and 2-(ethoxy)ethyl.

The term "haloalkoxy" as used herein denotes in each case a straight-chain or branched alkoxy group having from 1 to 10 carbon atoms, frequently from 1 to 6 carbon atoms, preferably 1 to 4 carbon atoms, wherein the hydrogen atoms of this group are partially or totally replaced with halogen atoms, in particular fluorine atoms. Preferred haloalkoxy moieties include C₁-C₄-haloalkoxy, in particular C₁-C₂-fluoroalkoxy, such as fluoromethoxy, difluoromethoxy, trifluoromethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoro-ethoxy, 2,2dichloro-2-fluorethoxy, 2,2,2-trichloroethoxy, pentafluoroethoxy and the like.

The term "alkylthio "(alkylsulfanyl: S-alkyl)" as used herein refers to a straight-chain or branched saturated alkyl group having 1 to 10 carbon atoms, preferably 1 to 4 carbon atoms (= C₁-C₄-alkylthio), more preferably 1 to 3 carbon atoms, which is attached via a sulfur atom.

The term "haloalkylthio" as used herein refers to an alkylthio group as mentioned above wherein the hydrogen atoms are partially or fully substituted by fluorine, chlorine, bromine and/or iodine.

The term "alkylsulfinyl" (alkylsulfoxyl: S(=O)-alkyl), as used herein refers to a straight-chain or branched saturated alkyl group (as mentioned above) having 1 to 10 carbon atoms, preferably 1 to 4 carbon atoms (= C₁-C₄-alkylsulfinyl), more preferably 1 to 3 carbon atoms bonded through the sulfur atom of the sulfinyl group at any position in the alkyl group.

The term "haloalkylsulfinyl" as used herein refers to an alkylsulfinyl group as mentioned above wherein the hydrogen atoms are partially or fully substituted by fluorine, chlorine, bromine and/or iodine.

The term "alkylsulfonyl" (S(=O)₂-alkyl) as used herein refers to a straight-chain or branched saturated alkyl group having 1 to 10 carbon atoms, preferably 1 to 4 carbon atoms (= C₁-C₄-alkylsulfonyl), preferably 1 to 3 carbon atoms, which is bonded via the sulfur atom of the sulfonyl group at any position in the alkyl group.

The term "haloalkylsulfonyl" as used herein refers to an alkylsulfonyl group as mentioned above wherein the hydrogen atoms are partially or fully substituted by fluorine, chlorine, bromine and/or iodine.

The term "alkylcarbonyl" refers to an alkyl group as defined above, which is bonded via the carbon atom of a carbonyl group (C=O) to the remainder of the molecule.

The term "haloalkylcarbonyl" refers to an alkylcarbonyl group as mentioned above, wherein the hydrogen atoms are partially or fully substituted by fluorine, chlorine, bromine and/or iodine.

The term "alkoxycarbonyl" refers to an alkylcarbonyl group as defined above, which is bonded via an oxygen atom to the remainder of the molecule.

The term "haloalkoxycarbonyl" refers to an alkoxycarbonyl group as mentioned above, wherein the hydrogen atoms are partially or fully substituted by fluorine, chlorine, bromine and/or iodine.

The term "alkenyl" as used herein denotes in each case a singly unsaturated hydrocarbon radical having usually 2 to 10, frequently 2 to 6, preferably 2 to 4 carbon atoms, e.g. vinyl, allyl (2-propen-1-yl), 1-propen-1-yl, 2-propen-2-yl, methallyl (2-methylprop-2-en-1-yl), 2-buten-1-yl, 3-buten-1-yl, 2-penten-1-yl, 3-penten-1-yl, 4-penten-1-yl, 1-methylbut-2-en-1-yl, 2-ethylprop-2-en-1-yl and the like.

The term "haloalkenyl" as used herein refers to an alkenyl group as defined above, wherein the hydrogen atoms are partially or totally replaced with halogen atoms.

The term "alkynyl" as used herein denotes in each case a singly unsaturated hydrocarbon radical having usually 2 to 10, frequently 2 to 6, preferably 2 to 4 carbon atoms, e.g. ethynyl, propargyl (2-propyn-1-yl), 1-propyn-1-yl, 1-methylprop-2-yn-1-yl), 2-butyn-1-yl, 3-butyn-1-yl, 1-pentyn-1-yl, 3-pentyn-1-yl, 4-pentyn-1-yl, 1-methylbut-2-yn-1-yl, 1-ethylprop-2-yn-1-yl and the like.

The term "haloalkynyl" as used herein refers to an alkynyl group as defined above, wherein the hydrogen atoms are partially or totally replaced with halogen atoms.

The term "cycloalkyl" as used herein and in the cycloalkyl moieties of cycloalkoxy and cyclo-alkylthio denotes in each case a monocyclic cycloaliphatic radical having usually from 3 to 10 or from 3 to 6 carbon atoms, such as cyclopropyl (cC₃H₅), cyclobutyl (cC₄H₇), cyclopentyl (cC₅H₉), cyclohexyl (cC₆H₁₁), cycloheptyl, cyclooctyl, cyclononyl and cyclodecyl or cyclopropyl, cyclobutyl, cyclopentyl and cyclohexyl.

The term "halocycloalkyl" as used herein and in the halocycloalkyl moieties of halocycloalkoxy and halocycloalkylthio denotes in each case a monocyclic cycloaliphatic radical having usually from 3 to 10 C atoms or 3 to 6 C atoms, wherein at least one, e.g. 1, 2, 3, 4 or 5 of the hydrogen atoms, are replaced by halogen, in particular by fluorine or chlorine. Examples are 1- and 2-flu-orocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl and the like.

The term "halocycloalkenyl" as used herein and in the halocycloalkenyl moieties of halocycloalkenyloxy and halocycloalkenylthio denotes in each case a monocyclic singly unsaturated non-aromatic radical having usually from 3 to 10, e.g. 3 or 4 or from 5 to 10 carbon atoms, preferably from 3- to 8 carbon atoms, wherein at least one, e.g. 1, 2, 3, 4 or 5 of the hydrogen atoms, are replaced by halogen, in particular by fluorine or chlorine. Examples are 3,3-difluorocyclopropen-1-yl and 3,3-dichlorocyclopropen-1-yl.

The term "cycloalkenylalkyl" refers to a cycloalkenyl group as defined above which is bonded via an alkyl group, such as a C₁-C₅-alkyl group or a C₁-C₄-alkyl group, in particular a methyl group (= cycloalkenylmethyl), to the remainder of the molecule.

The term "carbocycle" or "carbocyclyl" includes in general a 3- to 12-membered, preferably a 3- to 8-membered or a 5- to 8-membered, more preferably a 5- or 6-membered mono-cyclic, non-aromatic ring comprising 3 to 12, preferably 3 to 8 or 5 to 8, more preferably 5 or 6 carbon atoms. Preferably, the term "carbocycle" covers cycloalkyl and cycloalkenyl groups as defined above.

The term "heterocycle" or "heterocyclyl" includes in general 3- to 12-membered, preferably 3-to 6-membered, in particular 6-membered monocyclic heterocyclic non-aromatic radicals. The heterocyclic non-aromatic radicals usually comprise 1, 2, 3, 4 or 5, preferably 1, 2 or 3 heteroatoms selected from N, O, and S as ring members, wherein S-atoms as ring members may be present as S, SO, or SO₂. Examples of 5- or 6-membered heterocyclic radicals comprise saturated or unsaturated, non-aromatic heterocyclic rings, such as oxiranyl, oxetanyl, thietanyl, thietanyl-S-oxid (S-oxothietanyl), thietanyl-S-dioxid (S-dioxothiethanyl), pyrrolidinyl, pyrrolinyl, pyrazolinyl, tetrahydrofuranyl, dihydrofuranyl, 1,3-dioxolanyl, thiolanyl, S-oxothiolanyl, S-dioxo-thiolanyl, dihydrothienyl, S-oxodihydrothienyl, S-dioxodihydrothienyl, oxazolidinyl, oxazolinyl, thiazolinyl, oxathiolanyl, piperidinyl, piperazinyl, pyranyl, dihydropyranyl, tetrahydropyranyl, 1,3- and 1,4-dioxanyl, thiopyranyl, S.oxothiopyranyl, S-dioxothiopyranyl, dihydrothiopyranyl, S-oxo-dihydrothiopyranyl, S-dioxodihydrothiopyranyl, tetrahydrothiopyranyl, S-oxotetra-hydrothiopyranyl, S-dioxotetrahydrothiopyranyl, morpholinyl, thiomorpholinyl, S-oxothiomorpholinyl, S-dioxothiomorpholinyl, thiazinyl and the like. Examples for heterocyclic ring also comprising 1 or 2 carbonyl groups as ring members comprise pyrrolidin-2-onyl, pyrrolidin-2,5-dionyl, imidazolidin-2-onyl, oxazolidin-2-onyl, thiazolidin-2-only, and the like.

The term "hetaryl" includes monocyclic 5- or 6-membered heteroaromatic radicals comprising as ring members 1, 2, 3 or 4 heteroatoms selected from N, O, and S. Examples of 5- or 6-membered heteroaromatic radicals include pyridyl, i.e. 2-, 3-, or 4-pyridyl, pyrimidinyl, i.e. 2-, 4- or 5-pyrimidinyl, pyrazinyl, pyridazinyl, i.e. 3- or 4-pyridazinyl, thienyl, i.e. 2- or 3-thienyl, furyl, i.e. 2- or 3-furyl, pyrrolyl, i.e. 2- or 3-pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5-isothiazolyl, pyrazolyl, i.e. 1-, 3-, 4- or 5-pyrazolyl, i.e. 1-, 2-, 4- or 5-imidazolyl, oxadiazolyl, e.g. 2- or 5-[1,3,4]oxadiazolyl, 4- or 5-(1,2,3-oxadiazol)yl, 3- or 5-(1,2,4-oxadiazol)yl, 2- or 5-(1,3,4-thiadiazol)yl, thiadiazolyl, e.g. 2- or 5-(1,3,4-thiadiazol)yl, 4- or 5-(1,2,3-thiadiazol)yl, 3- or 5-(1,2,4-thiadiazol)yl, triazolyl, e.g. 1H-, 2H- or 3H-1,2,3-triazol-4-yl, 2H-triazol-3-yl, 1H-, 2H-, or 4H-1,2,4-triazolyl and tetrazolyl, i.e. 1H- or 2H-tetrazolyl. The term "hetaryl" also includes bicyclic 8 to 10-membered heteroaromatic radicals comprising as ring members 1, 2 or 3 heteroatoms selected from N, O, and S, wherein a 5- or 6-membered heteroaromatic ring is fused to a phenyl ring or to a 5- or 6-membered heteroaromatic radical. Examples of a 5- or 6-membered heteroaromatic ring fused to a phenyl ring or to a 5- or 6-membered heteroaromatic radical include benzofuranyl, benzothienyl, indolyl, indazolyl, benzimidazolyl, benzoxathiazolyl, benzoxadiazolyl, benzothiadiazolyl, benzoxazinyl, chinolinyl, isochinolinyl, purinyl, 1,8-naphthyridyl, pteridyl, pyrido[3,2-d]pyrimidyl or pyridoimidazolyl and the like. These fused hetaryl radicals may be bonded to the remainder of the molecule via any ring atom of 5- or 6-membered heteroaromatic ring or via a carbon atom of the fused phenyl moiety.

The terms "heterocyclylalkyl" and "hetarylalkyl" refer to heterocyclyl or hetaryl, respectively, as defined above which are bonded via a C₁-C₅-alkyl group or a C₁-C₄-alkyl group, in particular a methyl group (= heterocyclylmethyl or hetarylmethyl, respectively), to the remainder of the molecule.

The term "arylalkyl" and "phenylalkyl" refer to aryl as defined above and phenyl, respectively, which are bonded via C₁-C₅-alkyl group or a C₁-C₄-alkyl group, in particular a methyl group (= arylmethyl or phenylmethyl), to the remainder of the molecule, examples including benzyl, 1-phenylethyl, 2-phenylethyl, 2-phenoxyethyl etc.

The terms "alkylene", "cycloalkylene", "heterocycloalkylene", "alkenylene", "cycloalkenylene", "heterocycloalkenylene" and "alkynylene" refer to alkyl, cycloalkyl, heterocycloalkyl, alkenyl, cycloalkenyl, heterocycloalkenyl and alkynyl as defined above, respectively, which are bonded to the remainder of the molecule, via two atoms, preferably via two carbon atoms, of the respective group, so that they represent a linker between two moieties of the molecule.

In a particular embodiment, the variables of the compounds of the formula I have the following meanings, these meanings, both on their own and in combination with one another, being particular embodiments of the compounds of the formula I.

Embodiments and preferred compounds of the invention for use in pesticidal methods and for insecticidal application purposes are outlined in the following paragraphs.

With respect to the variables, the particularly preferred embodiments of the intermediates correspond to those of the compounds of the formula I.

In a preferred embodiment, the compounds I are present in form of a mixture of compounds I.A and I.B, wherein compound I.A with S-configuration of the carbon atom neighboring the nitrogen is present in an amount of more than 50% by weight, in particular of at least 70% by weight, more particularly of at least 85% by weight, specifically of at least 90% by weight, based on the total weight of compounds I.A and I.B.

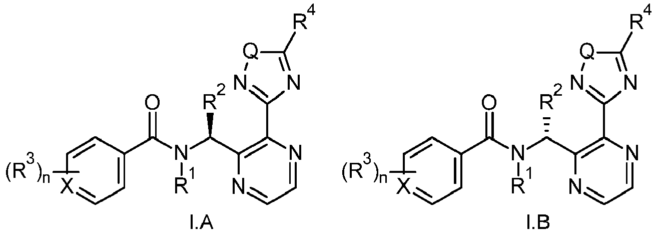

In one particularly preferred embodiment of the invention, the method comprises the step of contacting the plant, parts of it, its propagation material, the pests, their food supply, habitat or breeding grounds with a pesticidally effective amount of a compound of formula I.A.

Preferably R¹ is H, C₁-C₆-alkyl, C₃-C₆-alkynyl, C₃-C₆-cycloalkyl, or C₁-C₄-alkyl-C₃-C₆-cycloalkyl.

Preferably R² is CH₃.

X is preferably CH or CR³, particularly CH. Such compounds correspond to Formula 1.1

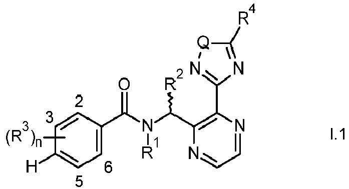

In another embodiment X is N. Such compounds correspond to formula 1.2.

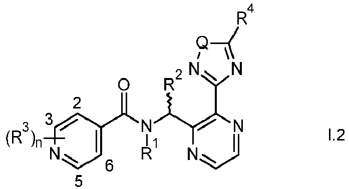

R³ is preferably halogen, CN, C₁-C₄-haloalkyl, C₁-C₄-haloalkoxy, C₃-C₄-cycloalkyl unsubstituted or substituted with one or more CN, C₃-C₄-halocycloalkyl, S(O)ₘ-C₁-C₄-alkyl, S(O)ₘ-C₁-C₄-haloalkyl, S(O)ₘ-C₃-C₄-cycloalkyl, S(O)ₘ-C₃-C₄-halocycloalkyl, S(O)ₘ-(substituted phenyl). Index m in R³ is preferably 2. Index n is preferably 2.

R³ groups stand preferably in positions 3 and 5.

In another embodiment R³ is preferably halogen, CN, C₁-C₄-haloalkyl, C₁-C₄-haloalkoxy, C₃-C₄-cycloalkyl, C₃-C₄-halocycloalkyl, S(O)ₘ-C₁-C₄-alkyl, S(O)M-C₁-C₄-haloalkyl, S(O)ₘ-C₃-C₄-cycloalkyl, S(O)m-C₃-C₄-halocycloalkyl, or
S(O)ₘ-R¹⁴, wherein R¹⁴ is phenyl, which is partially substituted with R¹¹
R⁴ is preferably H, C₁-C₄-alkyl, C₁-C₄-haloalkyl, C₁-C₄-alkoxy-C₁-C₄-alkyl, C(O)NH-C₁-C₆-alkyl, S(O)ₘ-C₁-C₄-alkyl, or phenyl unsubstituted or substituted with one or more groups R³.

In one embodiment of formula I, Q is O. Such compounds correspond to formula I.O

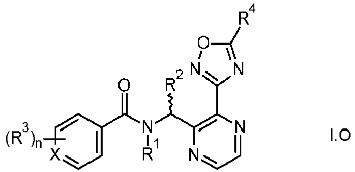

in another embodiment of formula I, Q is S. Such compounds correspond to formula I.S

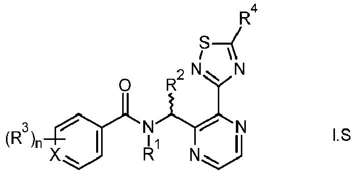

In particular with a view to their use, preference is given to the compounds of formula I compiled in the tables below. Each of the groups mentioned for a substituent in the tables is furthermore per se, independently of the combination in which it is mentioned, a particularly preferred aspect of the substituent in question.

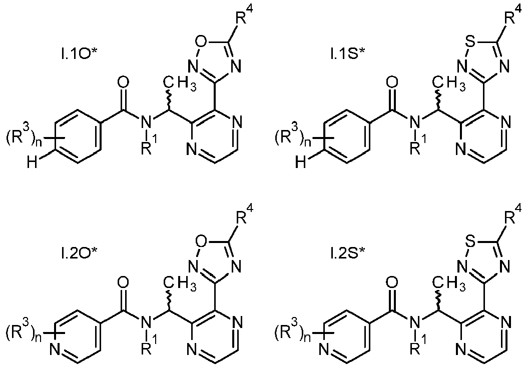

### Table 1

Compounds of formula I.1O* in which R⁴ is H, and the combination of R¹ and (R³)ₙ for a compound corresponds in each case to one row of Table A

### Table 2

Compounds of formula I.1O* in which R⁴ is CH₃, and the combination of R¹ and (R³)ₙ for a compound corresponds in each case to one row of Table A

### Table 3

Compounds of formula I.1O* in which R⁴ is CI, and the combination of R¹ and (R³)ₙ for a compound corresponds in each case to one row of Table A

### Table 4

Compounds of formula I.1O* in which R⁴ is C(O)N(CH₃)(C₂H₅), and the combination of R¹ and (R³)ₙ for a compound corresponds in each case to one row of Table A

### Table 5

Compounds of formula I.1O* in which R⁴ is C(O)NHCH₃, and the combination of R¹ and (R³)ₙ for a compound corresponds in each case to one row of Table A

### Table 6

Compounds of formula I.1S* in which R⁴ is H, and the combination of R¹ and (R³)ₙ for a compound corresponds in each case to one row of Table A

### Table 7

Compounds of formula I.1S* in which R⁴ is CH₃, and the combination of R¹ and (R³)ₙ for a compound corresponds in each case to one row of Table A

### Table 8

Compounds of formula I.1S* in which R⁴ is CI, and the combination of R¹ and (R³)ₙ for a compound corresponds in each case to one row of Table A

### Table 9

Compounds of formula I.1S* in which R⁴ is C(O)N(CH₃)(C₂H₅), and the combination of R¹ and (R³)ₙ for a compound corresponds in each case to one row of Table A

### Table 10

Compounds of formula I.1S* in which R⁴ is C(O)NHCH₃, and the combination of R¹ and (R³)ₙ for a compound corresponds in each case to one row of Table A

### Table 11

Compounds of formula I.2O* in which R⁴ is H, and the combination of R¹ and (R³)ₙ for a compound corresponds in each case to one row of Table A

### Table 12

Compounds of formula I.2O* in which R⁴ is CH₃, and the combination of R¹ and (R³)ₙ for a compound corresponds in each case to one row of Table A

### Table 13

Compounds of formula I.2O* in which R⁴ is CI, and the combination of R¹ and (R³)ₙ for a compound corresponds in each case to one row of Table A

### Table 14

Compounds of formula I.2O* in which R⁴ is C(O)N(CH₃)(C₂H₅), and the combination of R¹ and (R³)ₙ for a compound corresponds in each case to one row of Table A

### Table 15

Compounds of formula I.2O* in which R⁴ is C(O)NHCH₃, and the combination of R¹ and (R³)ₙ for a compound corresponds in each case to one row of Table A

### Table 16

Compounds of formula I.2S* in which R⁴ is H, and the combination of R¹ and (R³)ₙ for a compound corresponds in each case to one row of Table A

### Table 17

Compounds of formula I.2S* in which R⁴ is CH₃, and the combination of R¹ and (R³)ₙ for a compound corresponds in each case to one row of Table A

### Table 18

Compounds of formula I.2S* in which R⁴ is Cl, and the combination of R¹ and (R³)ₙ for a compound corresponds in each case to one row of Table A

### Table 19

Compounds of formula I.2S* in which R⁴ is C(O)N(CH₃)(C₂H₅), and the combination of R¹ and (R³)ₙ for a compound corresponds in each case to one row of Table A

### Table 20

Compounds of formula I.2S* in which R⁴ is C(O)NHCH₃, and the combination of R¹ and (R³)ₙ for a compound corresponds in each case to one row of Table A

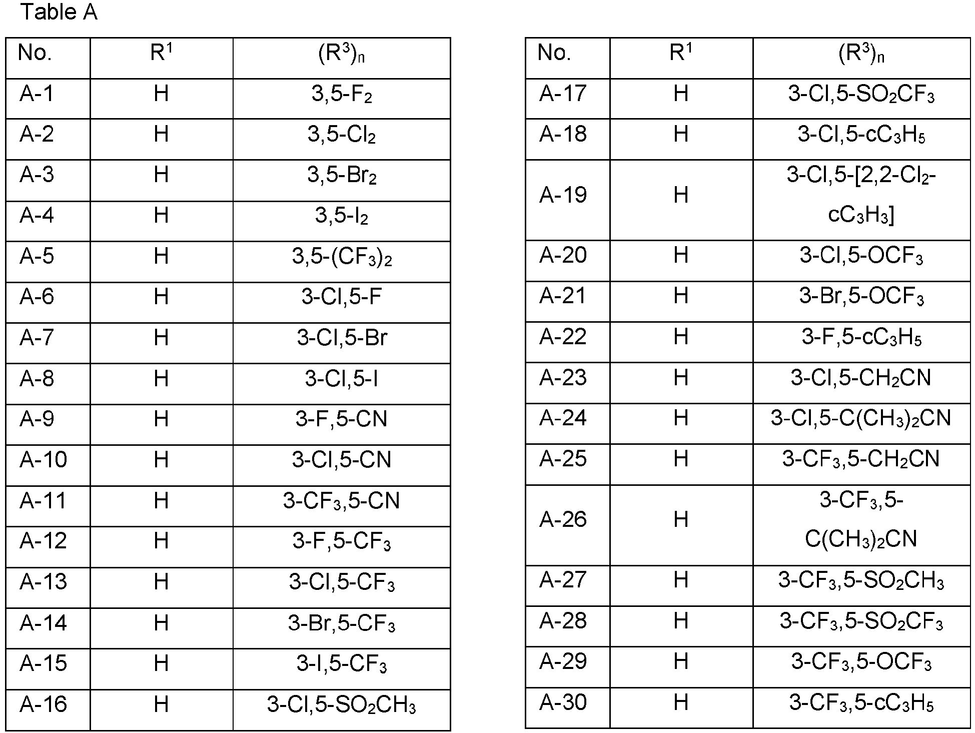

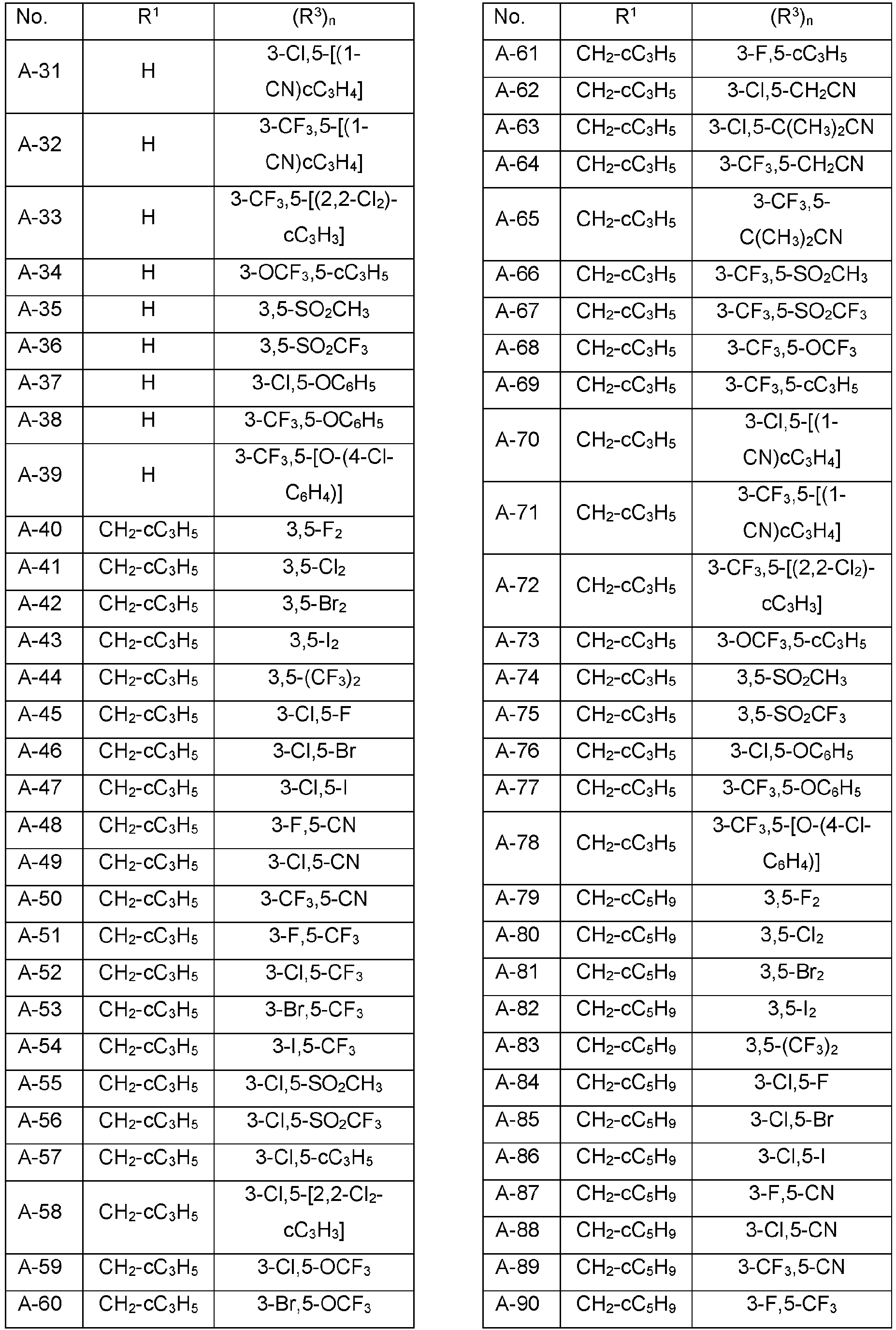

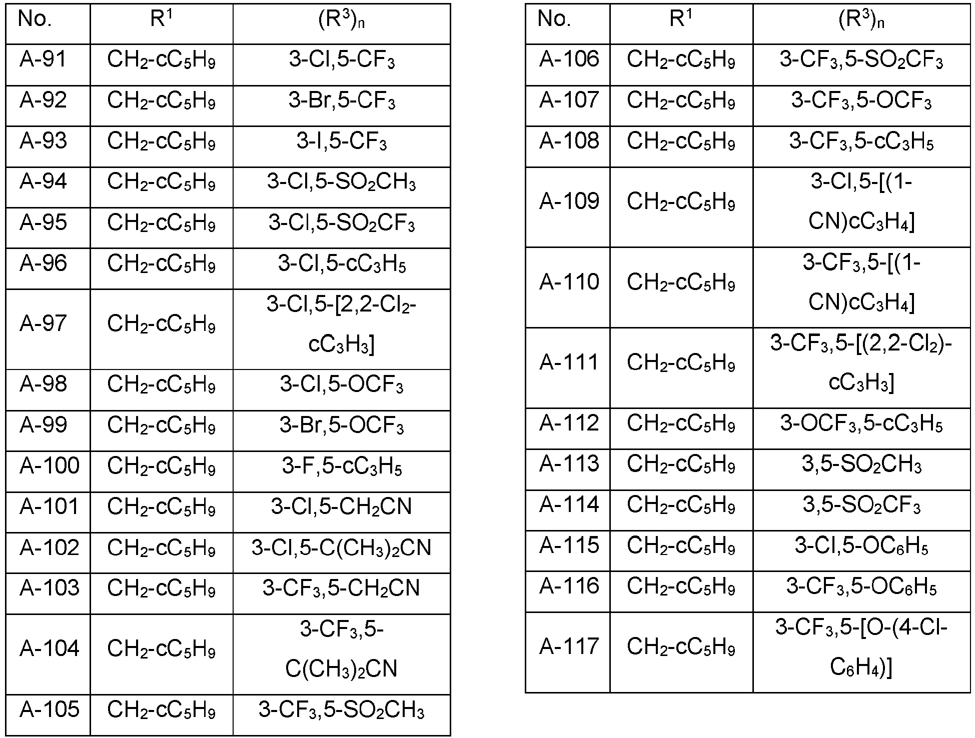

As used herein, the term "compound(s) of the invention" or "compound(s) according to the invention" refers to the compound(s) of formula (I) as defined above, which are also referred to as "compound(s) of formula I" or "compound(s) I" or "formula I compound(s)", and includes their salts, tautomers, stereoisomers, and N-oxides.

The invention also relates to a mixture of at least one compound of the invention with at least one mixing partner. Preferred are binary mixtures of one compound of the invention as component I with one mixing partner herein as component II. Preferred weight ratios for such binary mixtures are from 5000:1 to 1:5000, preferably from 1000:1 to 1:1000, more preferably from 100:1 to 1:100, particularly from 10:1 to 1:10. In such binary mixtures, components I and II may be used in equal amounts, or an excess of component I, or an excess of component II may be used.

Mixing partners can be selected from pesticides, in particular insecticides, nematicides, and acaricides, fungicides, herbicides, plant growth regulators, fertilizers. Preferred mixing partners are insecticides, nematicides, and fungicides.

The following list M of pesticides, grouped according the Mode of Action Classification of the Insecticide Resistance Action Committee (IRAC), together with which the compounds of the invention can be used and with which potential synergistic effects might be produced, illustrates the possible combinations:
M.1 AChE inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, triazamate; acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
M.2. GABA-gated chloride channel antagonists: cyclodiene organochlorine compounds: endo-sulfan, chlordane; phenylpyrazoles: ethiprole, fipronil, flufiprole, pyrafluprole, pyriprole;
M.3 Sodium channel modulators: pyrethroids: acrinathrin, allethrin, d-cis-trans allethrin, d-trans allethrin, bifenthrin, kappa-bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin,metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, transfluthrin; sodium channel modulatorse.g.: DDT, methoxychlor;
M.4 nAChR agonists: neonicotinoids: acetamiprid, clothianidin, cycloxaprid, dinotefuran, imidacloprid, nitenpyram, thiacloprid, thiamethoxam; 4,5-Dihydro-N-nitro-1-(2-oxiranylmethyl)-1H-imidazol-2-amine, (2E-)-1-[(6-Chloropyridin-3-yl)methyl]-N'-nitro-2-pentylidenehydrazinecarboximidamide; 1-[(6-Chloropyridin-3-yl)methyl]-7-methyl-8-nitro-5-propoxy-1,2,3,5,6,7-hexahydroimidazo[1,2-a]pyridine; nicotine; sulfoxaflor; flupyradifurone; triflumezopyrim, (3R)-3-(2-chlorothiazol-5-yl)-8-methyl-5-oxo-6-phenyl-2,3-dihydrothiazolo[3,2-a]pyrimidin-8-ium-7-olate, (3S)-3-(6-chloro-3-pyridyl)-8-methyl-5-oxo-6-phenyl-2,3-dihydrothiazolo[3,2-a]pyrimidin-8-ium-7-olate, (3S)-8-methyl-5-oxo-6-phenyl-3-pyrimidin-5-yl-2,3-dihydrothiazolo[3,2-a]pyrimidin-8-ium-7-olate, fenmezoditiaz; (3R)-3-(2-chlorothiazol-5-yl)-6-(3,5-dichlorophenyl)-8-methyl-5-oxo-2,3-dihydrothiazolo[3,2-a]pyrimidin-8-ium-7-olate, (3R)-3-(2-chlorothiazol-5-yl)-8-ethyl-5-oxo-6-phenyl-2,3-dihydrothiazolo[3,2-a]pyrimidin-8-ium-7-olate;
M.5 Nicotinic acetylcholine receptor allosteric activators: spinosad, spinetoram;
M.6 Chloride channel activators: abamectin, emamectin benzoate, ivermectin, lepimectin, milbemectin;
M.7 Juvenile hormone mimics, e.g.: hydroprene, kinoprene, methoprene; fenoxycarb, pyriproxyfen;
M.8 miscellaneous multi-site inhibitors: CH₃Br, other alkyl halides, chloropicrin, sulfuryl fluoride, borax, tartar emetic;
M.9 Chordotonal organ TRPV channel modulators: pymetrozine; pyrifluquinazon;
M.10 Mite growth inhibitors: clofentezine, hexythiazox, diflovidazin, etoxazole;
M.11 Microbial disruptors of insect midgut membranes: *bacillus thuringiensis*, *bacillus sphaericus,* and insecticdal proteins they producee.g.: *bacillus thuringiensis subsp. israelensis*, *bacillus sphaericus*, *bacillus thuringiensis subsp. aizawai*, *bacillus thuringiensis subsp. kurstaki*, *bacillus thuringiensis subsp. tenebrionis*, Bt crop proteins: Cry1Ab, Cry1Ac, Cry1 Fa, Cry2Ab, mCry3A, Cry3Ab, Cry3Bb, Cry34/35Ab1;
M.12 Inhibitors of mitochondrial ATP synthase: diafenthiuron, organotin miticidese.g.: azocyclotin, cyhexatin, fenbutatin oxide, propargite, tetradifon;
M.13 Uncouplers of oxidative phosphorylation via disruption of the proton gradient: chlorfenapyr, DNOC, sulfluramid;
M.14 nAChR channel blockers: nereistoxin analogues bensultap, cartap hydrochloride, thiocyclam, thiosultap-sodium;
M.15 Inhibitors of the chitin biosynthesis type 0, e.g.: bistrifluron, chlorfluazuron, diflubenzuron, flucycloxuron, flufenoxuron, hexaflumuron, lufenuron, novaluron, noviflumuron, teflubenzuron, triflumuron;
M.16 Inhibitors of the chitin biosynthesis type 1: buprofezin;
M.17 Moulting disruptors: Dipteran, cyromazine;
M.18 Ecdyson receptor agonists, e.g.: methoxyfenozide, tebufenozide, halofenozide, fufenozide, chromafenozide;
M.19 Octopamin receptor agonists: amitraz;
M.20 Mitochondrial complex III electron transport inhibitors: hydramethylnon, acequinocyl, fluacrypyrim; bifenazate;
M.21 METI acaricides and insecticides, e.g.: fenazaquin, fenpyroximate, pyrimidifen, pyridaben, tebufenpyrad, tolfenpyrad, rotenone;
M.22 Voltage-dependent sodium channel blockers: indoxacarb, metaflumizone, 2-[2-(4-cyanophenyl)-1-[3-(trifluoromethyl)phenyl]ethylidene]-N-[4-(difluoromethoxy)phenyl]-hydrazinecarboxamide, N-(3-chloro-2-methylphenyl)-2-[(4-chlorophenyl)[4-[methyl(methylsulfonyl)amino]phenyl]methylene]-hydrazinecarboxamide;
M.23 Inhibitors of the of acetyl CoA carboxylase, e.g.: spirodiclofen, spiromesifen, spirotetramat; spiropidion;
M.24 Mitochondrial complex IV electron transport inhibitors: e.g. aluminium phosphide, calcium phosphide, zinc phosphide, cyanide;
M.25 Mitochondrial complex II electron transport inhibitors, e.g.: cyenopyrafen, cyflumetofen;
M.28 Ryanodine receptor-modulators: flubendiamide, chlorantraniliprole, cyantraniliprole, tetraniliprole, (R)-3-chloro-N1-{2-methyl-4-[1,2,2,2-tetrafluoro-1-(trifluoromethyl)ethyl]phenyl}-N2-(1-methyl-2-methylsulfonylethyl)phthalamid, (S)-3-chloro-N1-{2-methyl-4-[1,2,2,2-tetrafluoro-1-(trifluoromethyl)ethyl]phenyl}-N2-(1-methyl-2-methylsulfonylethyl)phthalamide, cyclaniliprole, methyl-2-[3,5-dibromo-2-({[3-bromo-1-(3-chlorpyridin-2-yl)-1H-pyrazol-5-yl]carbonyl}amino)benzoyl]-1,2-dimethylhydrazinecarboxylate; N-[2-(5-amino-1,3,4-thiadiazol-2-yl)-4-chloro-6-methylphenyl]-3-bromo-1-(3-chloro-2-pyridinyl)-1H-pyrazole-5-carboxamide; 3-chloro-1-(3-chloro-2-pyridinyl)-N-[2,4-dichloro-6-[[(1-cyano-1-methylethyl)amino]carbonyl]phenyl]-1H-pyrazole-5-carboxamide; tetrachlorantraniliprole; N-[4-chloro-2-[[(1,1-dimethylethyl)amino]carbonyl]-6-methylphenyl]-1-(3-chloro-2-pyridinyl)-3-(fluoromethoxy)-1H-pyrazole-5-carboxamide; cyhalodiamide, fluchlordiniliprole;
M.29: Chordotonal organ Modulators: flonicamid;
M.UN. Unknown mode of action: afidopyropen, afoxolaner, azadirachtin, amidoflumet, benzoximate, broflanilide, bromopropylate, chinomethionat, cryolite, dicloromezotiaz, dicofol, dimpropyridaz, flufenerim, flometoquin, fluensulfone, fluhexafon, fluopyram, fluralaner, metaldehyde, metoxadiazone, piperonyl butoxide, pyflubumide, pyridalyl, tioxazafen, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 1-[2-fluoro-4-methyl-5-[(2,2,2-trifluoroethyl)sulfinyl]phenyl]-3-(trifluoromethyl)-1H-1,2,4-triazole-5-amine, actives on basis of *bacillus firmus* (Votivo, I-1582); flupyrimin; fluazaindolizine; 4-[5-(3,5-dichlorophenyl)-5-(trifluoromethyl)-4H-isoxazol-3-yl]-2-methyl-N-(1-oxothietan-3-yl)benzamide; fluxametamide; 5-[3-[2,6-dichloro-4-(3,3-dichloroallyloxy)phenoxy]propoxy]-1H-pyrazole; 4-cyano-N-[2-cyano-5-[[2,6-dibromo-4-[1,2,2,3,3,3-hexafluoro-1-(trifluoromethyl)-propyl]phenyl]carbamoyl]phenyl]-2-methyl-benzamide; 4-cyano-3-[(4-cyano-2-methylbenzoyl)amino]-N-[2,6-dichloro-4-[1,2,2,3,3,3-hexafluoro-1-(trifluoromethyl)propyl]phenyl]-2-fluoro-benzamide; N-[5-[[2-chloro-6-cyano-4-[1,2,2,3,3,3-hexafluoro-1-(trifluoromethyl)propyl]phenyl]carbamoyl]-2-cyano-phenyl]-4-cyano-2-methyl-benzamide; N-[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide; N-[5-[[2-bromo-6-chloro-4-[1,2,2,3,3,3-hexafluoro-1-(trifluoromethyl)propyl]phenyl]carbamoyl]-2-cyano-phenyl]-4-cyano-2-methyl-benzamide; 4-cyano-N-[2-cyano-5-[[2,6-dichloro-4-[1,2,2,3,3,3-hexafluoro-1-(trifluoromethyl)-propyl]phenyl]carbamoyl]phenyl]-2-methyl-benzamide; 4-cyano-N-[2-cyano-5-[[2,6-dichloro-4-[1,2,2,2-tetrafluoro-1-(trifluoromethyl)ethyl]phenyl]carbamoyl]phenyl]-2-methyl-benzamide; N-[5-[[2-bromo-6-chloro-4-[1,2,2,2-tetrafluoro-1-(trifluoromethyl)ethyl]phenyl]carbamoyl]-2-cyanophenyl]-4-cyano-2-methyl-benzamide;
2-(1,3-dioxan-2-yl)-6-[2-(3-pyridinyl)-5-thiazolyl]-pyridine; 2-[6-[2-(5-fluoro-3-pyridinyl)-5-thiazolyl]-2-pyridinyl]-pyrimidine; 2-[6-[2-(3-pyridinyl)-5-thiazolyl]-2-pyridinyl]-pyrimidine; N-methylsulfonyl-6-[2-(3-pyridyl)thiazol-5-yl]pyridine-2-carboxamide; N-methylsulfonyl-6-[2-(3-pyridyl)thiazol-5-yl]pyridine-2-carboxamide; 1-[(6-chloro-3-pyridinyl)methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; N-(1-methylethyl)-2-(3-pyridinyl)-2H-indazole-4-carboxamide; N-cyclopropyl-2-(3-pyridinyl)-2H-indazole-4-carboxamide; N-cyclohexyl-2-(3-pyridinyl)-2H-indazole-4-carboxamide; 2-(3-pyridinyl)-N-(2,2,2-trifluoroethyl)-2H-indazole-4-carboxamide; 2-(3-pyridinyl)-N-[(tetrahydro-2-furanyl)methyl]-2H-indazole-5-carboxamide; methyl 2-[[2-(3-pyridinyl)-2H-indazol-5-yl]carbonyl]hydrazinecarboxylate; N-[(2,2-difluorocyclopropyl)methyl]-2-(3-pyridinyl)-2H-indazole-5-carboxamide; N-(2,2-difluoropropyl)-2-(3-pyridinyl)-2H-indazole-5-carboxamide; 2-(3-pyridinyl)-N-(2-pyrimidinylmethyl)-2H-indazole-5-carboxamide; N-[(5-methyl-2-pyrazinyl)methyl]-2-(3-pyridinyl)-2H-indazole-5-carboxamide, tyclopyrazoflor; sarolaner, lotilaner; N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; 2-(3-ethylsulfonyl-2-pyridyl)-3-methyl-6-(trifluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine; Isocycloseram; N-[4-chloro-3-(cyclopropylcarbamoyl)phenyl]-2-methyl-5-(1,1,2,2,2-pentafluoroethyl)-4-(trifluoromethyl)pyrazole-3-carboxamide, N-[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; acynonapyr; benzpyrimoxan; tigolaner; oxazosulfyl; [(2S,3R,4R,5S,6S)-3,5-dimethoxy-6-methyl-4-propoxy-tetrahydropyran-2-yl] N-[4-[1-[4-(trifluoromethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(trifluoromethoxy)phenyl]-1,2,4-triazol-3-yl]phenyl]carbamate; [(2S,3R,4R,5S,6S)-3,5-dimethoxy-6-methyl-4-propoxy-tetrahydropyran-2-yl] N-[4-[1-[4-(1,1,2,2,2-pentafluoroethoxy)phenyl]-1,2,4-triazol-3-yl]phenyl]carbamate; [(2S,3R,4R,5S,6S)-3,4,5-trimethoxy-6-methyl-tetrahydropyran-2-yl]N-[4-[1-[4-(1,1,2,2,2-pentafluoroethoxy)phenyl]-1,2,4-triazol-3-yl]phenyl]carbamate; (2Z)-3-(2-isopropylphenyl)-2-[(E)-[4-[1-[4-(trifluoromethoxy)phenyl]-1,2,4-triazol-3-yl]phenyl]methylenehydrazono]thiazolidin-4-one, (2Z)-3-(2-isopropylphenyl)-2-[(E)-[4-[1-[4-(1,1,2,2,2-pentafluoroethoxy)phenyl]-1,2,4-triazol-3-yl]phenyl]methylenehydrazono]thiazolidin-4-one; 2-(6-chloro-3-ethylsulfonyl-imidazo[1,2-a]pyridin-2-yl)-3-methyl-6-(trifluoromethyl)imidazo[4,5-b]pyridine, 2-(6-bromo-3-ethylsulfonyl-imidazo[1,2-a]pyridin-2-yl)-3-methyl-6-(trifluoromethyl)imidazo[4,5-b]pyridine, 2-(3-ethylsulfonyl-6-iodo-imidazo[1,2-a]pyridin-2-yl)-3-methyl-6-(trifluoromethyl)imidazo[4,5-b]pyridine, 2-[3-ethylsulfonyl-6-(trifluoromethyl)imidazo[1,2-a]pyridin-2-yl]-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(3-ethylsulfonyl-7-iodo-imidazo[1,2-a]pyridin-2-yl)-3-methyl-6-(trifluoromethyl)imidazo[4,5-b]pyridine, 3-ethylsulfonyl-6-iodo-2-[3-methyl-6-(trifluoromethyl)imidazo[4,5-b]pyridin-2-yl]imidazo[1,2-a]pyridine-8-carbonitrile, 2-[3-ethylsulfonyl-8-fluoro-6-(trifluoromethyl)imidazo[1,2-a]pyridin-2-yl]-3-methyl-6-(trifluoromethyl)imidazo[4,5-b]pyridine, 2-[3-ethylsulfonyl-7 -(trifluoromethyl)imidazo[1,2-a]pyridin-2-yl]-3-methyl-6-(trifluoromethylsulfinyl)imidazo[4,5-b]pyridine, 2-[3-ethylsulfonyl-7-(trifluoromethyl)imidazo[1,2-a]pyridin-2-yl]-3-methyl-6-(trifluoromethyl)imidazo[4,5-c]pyridine, 2-(6-bromo-3-ethylsulfonyl-imidazo[1,2-a]pyridin-2-yl)-6-(trifluoromethyl)pyrazolo[4,3-c]pyridine.

The commercially available compounds M listed above may be found in The Pesticide Manual, 18th Edition, C. MacBean, British Crop Protection Council (2018), or http://bcpcdata.com/pesticide-manual.html, http://www.alanwood.net/pesticides. The active compounds described by IUPAC nomenclature are known from CN103814937; WO2013/003977, WO2007/101369, WO2018/177970, CN10171577, CN102126994, WO2007/101540, WO2007/043677, WO2011/085575, WO2008/134969, WO2012/034403, WO2006/089633, WO2008/067911, WO2006/043635, WO2009/124707, WO2013/050317, WO2010/060379, WO2010/127926, WO2010/006713, WO2012/000896, WO2007/101369, WO2012/143317, WO2015/038503, EP2910126, WO2015/059039, WO2015/190316, WO2012/126766, WO2009/102736, WO2013/116053, WO2018/052136.

The following list of fungicides, in conjunction with which the compounds of the invention can be used, illustrates the possible combinations:
A) Respiration inhibitors
   - Inhibitors of complex III at Qo site: azoxystrobin (A.1.1), coumeth¬oxy¬strobin (A.1.2), coumoxystrobin (A.1.3), dimoxystrobin (A.1.4), enestroburin (A.1.5), fenaminstrobin (A.1.6), fenoxy¬strobin/flufenoxystrobin (A.1.7), fluoxastro¬bin (A.1.8), kresoxim-methyl (A.1.9), mandestrobin (A.1.10), meto¬minostrobin (A.1.11), orysastrobin (A.1.12), picoxy-strobin (A.1.13), pyraclostrobin (A.1.14), pyrametostrobin (A.1.15), pyraoxystrobin (A.1.16), trifloxy¬strobin (A.1.17), 2 (2-(3-(2,6-di¬chlorophenyl)-1-methyl-allylidene¬aminooxy¬methyl)-phenyl)-2 methoxyimino-N methyl-acetamide (A.1.18), pyribencarb (A.1.19), triclopyricarb/chloro¬dincarb (A.1.20), famoxadone (A.1.21), fenamidone (A.1.21), methyl-N-[2-[(1,4-dimethyl-5 phenyl-pyrazol-3-yl)oxylmethyl]phenyl]-N-methoxy-carbamate (A.1.22), metyltetraprole (A.1.25), (Z,2E) 5[1-(2,4-dichloro¬phenyl)pyrazol-3-yl]-oxy-2-methoxyimino-N,3-dimethyl-pent-3-en¬amide (A.1.34), (Z,2E) 5 [1 (4-chlorophenyl)pyrazol-3-yl]oxy-2-methoxyimino-N,3-dimethyl-pent-3-enamide (A.1.35), pyriminostrobin (A.1.36), bifujunzhi (A. 1.37), 2-(ortho-((2,5-dimeth¬ylphenyl-oxy-methylen)phenyl)-3-methoxy-acrylic acid methylester (A.1.38) ;
   - inhibitors of complex III at Qi site: cyazofamid (A.2.1), amisulbrom (A.2.2), [(6S,7R,8R) 8 benzyl-3-[(3-hydroxy-4-methoxy-pyridine-2-carbonyl)amino]-6-methyl-4,9-di¬oxo-1,5-dioxonan-7-yl] 2-methylpropanoate (A.2.3), fenpicoxamid (A.2.4), florylpicoxamid (A.2.5), [(1S,2S)-2-(4-fluoro-2-methyl-phenyl)-1,3-dimethyl-butyl] (2S)-2-[(3-hydroxy-4-methoxy-pyridine-2-carbonyl)amino]propanoate, [(1S,2S)-2-(2,4-dimethylphenyl)-1,3-di¬methyl-butyl] (2S)-2-[(3-hydroxy-4-methoxy-pyridine-2-carbonyl)amino]propanoate, [(1S,2S)-2-(2,4-di¬fluorophenyl)-1,3-dimethyl-butyl](2S)-2-[(3-hydroxy-4-methoxy-pyridine-2 carbonyl)amino]¬propanoate, [(1S,2S)-2-(2-fluoro-4-methyl-phenyl)-1,3-dimethyl-butyl] (2S)-2-[(3-hydroxy-4-methoxy-pyridine-2-carbonyl)amino]propanoate, [(1S,2S)-2-(4-fluoro-2 methyl-phenyl)-1,3-dimethyl-butyl] (2S)-2-[(3-acetoxy-4-methoxy-pyridine-2-carbonyl)¬amino]¬propanoate, [(1S,2S)-2-(2,4-dimethylphenyl)-1,3-dimethyl-butyl] (2S)-2-[(3-acetoxy-4 methoxy-pyridine-2-carbonyl)amino]propanoate, [(1S,2S)-2-(2,4-difluorophenyl)-1,3-di¬methyl-butyl] (2S)-2-[(3-acetoxy-4-methoxy-pyridine-2-carbonyl)amino]propanoate, [(1S,2S)-2-(2-fluoro-4-methyl-phenyl)-1,3-dimethyl-butyl] (2S)-2-[(3-acetoxy-4-methoxy-pyridine-2-car¬bonyl)amino]propanoate, [2-[[(1S)-2-[(1S,2S)-2-(4-fluoro-2-methyl-phenyl)-1,3-dimethyl-but¬oxy]-1-methyl-2-oxo-ethyl]carbamoyl]-4-methoxy-3-pyhdyl]oxymethyl 2-methylpropanoate, [2-[[(1S)-2-[(1S,2S)-2-(2,4-dimethylphenyl)-1,3-dimethyl-butoxy]-1-methyl-2-oxoethyl]carba¬moyl]-4-methoxy-3-pyridyl]oxymethyl 2-methylpropanoate, [2-[[(1S)-2-[(1S,2S)-2-(2,4-difluo-rophenyl)-1,3-dimethyl-butoxy]-1-methyl-2-oxo-ethyl]carbamoyl]-4-methoxy-3-pyridyl]oxy¬methyl 2-methylpropanoate, [2-[[(1S)-2-[(1S,2S)-2-(2-fluoro-4-methyl-phenyl)-1,3-dimethyl-but¬oxy]-1-methyl-2-oxo-ethyl]carbamoyl]-4-methoxy-3-pyhdyl]oxymethyl 2-methylpropa¬no¬ate, [(1S,2S)-1-methyl-2-(o-tolyl)propyl] (2S)-2-[(3-acetoxy-4-methoxy-pyridine-2-carbonyl)¬amino]¬propanoate, [(1S,2S)-1-methyl-2-(o-tolyl)propyl] (2S)-2-[(4-methoxy-3-propanoyloxy-pyridine-2-carbonyl)amino]propanoate, [(1S,2S)-1-methyl-2-(o-tolyl)propyl] (2S)-2-[(3-hydro¬xy-4-methoxy-pyridine-2-carbonyl)amino]propanoate, [4-methoxy-2-[[(1S)-1-methyl-2-[(1S,2S)-1-methyl-2-(o-tolyl)propoxy]-2-oxo-ethyl]carbamoyl]-3-pyridyl] 2-methylpropanoate, [(1S,2S)-2-(2,4-dimethylphenyl)-1-methyl-propyl] (2S)-2-[(3-acetoxy-4-methoxy-pyridine-2-carbonyl)amino]propanoate, [2-[[(1S)-2-[(1S,2S)-2-(2,4-dimethylphenyl)-1-methyl-propoxy]-1-methyl-2-oxo-ethyl]carbamoyl]-4-methoxy-3-pyridyl] 2-methylpropanoate, [(1S,2S)-2-(2,4-dimethylphenyl)-1-methyl-propyl] (2S)-2-[(3-hydroxy-4-methoxy-pyridine-2 carbonyl)amino]propanoate, [(1S,2S)-2-(2,6-dimethylphenyl)-1-methyl-propyl] (2S)-2-[(3-acetoxy-4-methoxy-pyridine-2-carbonyl)amino]propanoate, [2-[[(1S)-2-[(1S,2S)-2-(2,6-di¬methylphenyl)-1-methyl-propoxy]-1-methyl-2-oxo-ethyl]carbamoyl]-4-methoxy-3- pyridyl]-2-methylpropanoate, [(1S,2S)-2-(2,6-dimethylphenyl)-1-methyl-propyl] (2S)-2-[(3-hydroxy-4-methoxy-pyridine-2-carbonyl)amino]propanoate, [(1S,2S)-2-[4-fluoro-2-(trifluoromethyl)¬phenyl]-1-methyl-propyl] (2S)-2-[(3-acetoxy-4-methoxy-pyridine-2-carbonyl)amino]propano¬ate, [2-[[(1S)-2-[(1S,2S)-2-[4-fluoro-2-(trifluoromethyl)phenyl]-1-methyl-propoxy]-1-methyl-2 oxoethyl]carbamoyl]-4-methoxy-3-pyridyl] 2-methylpropanoate, [(1S,2S)-2-[4-fluoro-2-(tri¬fluoromethyl)phenyl]-1-methyl-propyl] (2S)-2-[(3-hydroxy-4-methoxy-pyridine-2-carbo¬nyl)¬amino]propanoate, [(1S,2S)-2-(4-fluoro-2-methyl-phenyl)-1-methyl-propyl] (2S)-2-[(3-acet¬oxy-4-methoxy-pyridine-2-carbonyl)amino]propanoate, [2-[[(1S)-2-[(1S,2S)-2-(4-fluoro-2-me¬thyl-phenyl)-1-methyl-propoxy]-1-methyl-2-oxo-ethyl]carbamoyl]-4-methoxy-3-pyridyl]2-me¬thylpropanoate, [(1S,2S)-2-(4-fluoro-2-methyl-phenyl)-1-methyl-propyl] (2S)-2-[(3-hydroxy-4 methoxy-pyridine-2-carbonyl)amino]propanoate, [(1S,2S)-1-methyl-2-[2-(trifluoromethyl)-phenyl]propyl] (2S)-2-[(3-acetoxy-4-methoxy-pyridine-2-carbonyl)amino]propanoate, [4-me¬thoxy-2-[[(1S)-1-methyl-2-[(1S,2S)-1-methyl-2-[2-(trifluoromethyl)phenyl]propoxy]-2-oxo-ethyl]carbamoyl]-3-pyridyl] 2-methylpropanoate, [(1S,2S)-1-methyl-2-[2-(trifluoromethyl)¬phenyl]¬propyl] (2S)-2-[(3-hydroxy-4-methoxy-pyridine-2-carbonyl)amino]propanoate, [(1S,2S)-2-(4-fluoro-2,6-dimethyl-phenyl)-1-methyl-propyl] (2S)-2-[(3-acetoxy-4-methoxy-pyridine-2-carbonyl)amino]propanoate, [2-[[(1S)-2-[(1S,2S)-2-(4-fluoro-2,6-dimethyl-phenyl)-1-methyl-propoxy]-1-methyl-2-oxo-ethyl]carbamoyl]-4-methoxy-3-pyridyl] 2-methylpropano¬ate, [(1S,2S)-2-(4-fluoro-2,6-dimethyl-phenyl)-1-methyl-propyl] (2S)-2-[(3-hydroxy-4-meth¬oxy-pyridine-2-carbonyl)amino]propanoate;
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28) , N-[2-[2-chloro-4-(trifluoro¬methyl)phenoxy]phenyl]-3-(difluoromethyl)-5-fluoro-1-methyl-pyrazole-4-carboxamide (A.3.29) , methyl (E)-2-[2-[(5-cyano-2-methyl-phenoxy)methyl]phenyl]-3-methoxy-prop-2 enoate (A.3.30), isoflucypram (A.3.31) , 2-(difluoromethyl)-N-(1,1,3-trimethyl-indan-4 yl)¬pyridine-3-carboxamide (A.3.32), 2-(difluoromethyl)-N-[(3R)-1,1,3-trimethylindan-4-yl]¬pyridine-3-carboxamide (A.3.33), 2-(difluoromethyl)-N-(3-ethyl-1,1-dimethyl-indan-4-yl)¬pyridine-3-carboxamide (A.3.34), 2-(difluoromethyl)-N-[(3R)-3-ethyl-1,1-dimethyl-indan-4-yl]-pyridine-3-carboxamide (A.3.35), 2-(difluoromethyl)-N-(1,1-dimethyl-3-propyl-indan-4-yl)¬py¬ridine-3-carboxamide (A.3.36), 2-(difluoromethyl)-N-[(3R)-1,1-dimethyl-3-propyl-indan-4-yl]¬pyridine-3-carboxamide (A.3.37), 2-(difluoromethyl)-N-(3-isobutyl-1,1-dimethyl-indan-4-yl)¬pyridine-3-carboxamide (A.3.38), 2-(difluoromethyl)-N-[(3R)-3-isobutyl-1,1-dimethyl-indan-4 yl]pyridine-3-carboxamide (A.3.39) ;
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromu-conazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothio-conazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2 (2,4-difluorophenyl)-1,1-difluoro-3-(tetrazol-1-yl)-1-[5-[4-(2,2,2-trifluoroethoxy)phenyl]-2 pyridyl]propan-2-ol (B.1.31), 2-(2,4-difluorophenyl)-1,1-difluoro-3-(tetrazol-1-yl)-1-[5-[4-(trifluoromethoxy)phenyl]-2-pyridyl]propan-2-ol (B.1.32), fluooxytioconazole (B.1.33), ipfentrifluconazole (B.1.37), mefentrifluconazole (B.1.38), 2-(chloromethyl)-2-methyl-5-(p-tolylmethyl)-1 (1,2,4-triazol-1 ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fe¬narimol (B.1.49), pyrifenox (B.1.50), triforine (B.1.51), [3-(4-chloro-2-fluorophenyl)-5-(2,4-di¬fluoro¬phenyl)isoxazol-4-yl]-(3-pyridyl)methanol (B.1.52), 4-[[6-[2-(2,4-difluorophenyl)-1,1-difluoro-2-hydroxy-3-(1,2,4-triazol-1-yl)propyl]-3-pyridyl]oxy]¬benzo¬nitrile (B.1.53), 2-[6-(4-bromophenoxy)-2-(trifluoromethyl)-3-pyridyl]-1-(1,2,4-triazol-1 yl)propan-2-ol (B.1.54), 2-[6-(4-chlorophenoxy)-2-(trifluoromethyl)-3-pyridyl]-1-(1,2,4-tri¬azol-1-yl)propan-2-ol (B.1.55);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4 amine (C.2.6), 5-fluoro-2-(4-fluorophenylmethoxy)pyrimidin-4 amine (C.2.7), 5-fluoro-2 (4 chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), N eth¬yl-2-[(3-ethynyl-8-methyl-6-quinolyl)oxy]butanamide (D.1.8), N-ethyl-2-[(3-ethynyl-8 methyl-6 quinolyl)oxy]-2-methylsulfanyl-acetamide (D.1.9), 2-[(3-ethynyl-8-methyl-6-quinol-yl)oxy]-N (2-fluoroethyl)butanamide (D.1.10), 2-[(3-ethynyl-8-methyl-6-quinolyl)oxy]-N-(2-flu¬oroeth¬yl)-2-methoxy-acetamide (D.1.11), 2-[(3-ethynyl-8-methyl-6-quinolyl)oxy]-N-propyl-butanam¬ide (D.1.12), 2-[(3-ethynyl-8-methyl-6-quinolyl)oxy]-2-methoxy-N-propyl-acetamide (D.1.13), 2-[(3-ethynyl-8-methyl-6-quinolyl)oxy]-2-methylsulfanyl-N-propyl-acetamide (D.1.14), 2 [(3 ethynyl-8-methyl-6-quinolyl)oxy]-N-(2-fluoroethyl)-2-methylsulfanyl-acetamide (D.1.15), 4-(2-bromo-4-fluoro-phenyl)-N-(2-chloro-6-fluoro-phenyl)-2,5-dimethyl-pyrazol-3 amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7), phenamacril (D.2.8);
E) Inhibitors of amino acid and protein synthesis
   - methionine synthesis inhibitors: cyprodinil (E.1.1), mepani¬pyrim (E.1.2), pyrimethanil (E.1.3);
   - protein synthesis inhibitors: blasticidin-S (E.2.1), kasugamycin (E.2.2), kasugamycin hydro-chloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7), zinc thiazole (G.2.8);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1); - inhibitors of oxysterol binding protein: oxathiapiprolin (G.5.1), fluoxapiprolin (G.5.3), 4-[1-[2-[3-(difluoromethyl)-5-methyl-pyrazol-1-yl]acetyl]-4-piperidyl]-N-te¬tralin-1-yl-pyridine-2-carboxamide (G.5.4), 4-[1-[2-[3,5-bis(difluoromethyl)pyrazol-1-yl]acetyl]-4-piperidyl]-N-tetralin-1-yl-pyridine-2-carboxamide (G.5.5), 4-[1-[2-[3-(difluoromethyl)-5-(tri¬fluoromethyl)pyrazol-1-yl]acetyl]-4-piperidyl]-N-tetralin-1-yl-pyridine-2-carboxamide (G.5.6), 4-[1-[2-[5-cyclopropyl-3-(difluoromethyl)pyrazol-1-yl]acetyl]-4-piperidyl]-N-tetralin-1-yl-pyri¬dine-2-carboxamide (G.5.7), 4-[1-[2-[5-methyl-3-(trifluoromethyl)pyrazol-1-yl]acetyl]-4-pi¬peri¬dyl]-N-tetralin-1-yl-pyridine-2-carboxamide (G.5.8), 4-[1-[2-[5-(difluoromethyl)-3-(trifluoro¬methyl)pyrazol-1-yl]acetyl]-4-piperidyl]-N-tetralin-1-yl-pyridine-2-carboxamide (G.5.9), 4 [1 [2-[3,5-bis(trifluoromethyl)pyrazol-1-yl]acetyl]-4-piperidyl]-N-tetralin-1-yl-pyridine-2-car¬boxamide (G.5.10), (4-[1-[2-[5-cyclopropyl-3-(trifluoromethyl)pyrazol-1-yl]acetyl]-4-piperidyl]-N-tetralin-1-yl-pyridine-2-carboxamide (G.5.11) ;
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachloro-benzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-di¬methyl-1H,5H-[1,4]di¬thiino[2,3-c:5,6-c']dipyrrole-1,3,5,7(2H,6H)-tetraone (H.4.10);
I) Cell wall synthesis inhibitors
   - inhibitors of glucan synthesis: validamycin (1.1.1), polyoxin B (1.1.2);
   - melanin synthesis inhibitors: pyroquilon (1.2.1), tricyclazole (1.2.2), carpropamid (1.2.3), dicyclomet (1.2.4), fenoxanil (1.2.5);
J) Plant defence inducers
   - acibenzolar-S-methyl (J.1.1), probenazole (J.1.2), isotianil (J.1.3), tiadinil (J.1.4), prohexa-dione-calcium (J.1.5); phosphonates: fosetyl (J.1.6), fosetyl-aluminum (J.1.7), phosphorous acid and its salts (J.1.8), calcium phosphonate (J.1.11), potassium phospho¬nate (J.1.12), potassium or sodium bicarbonate (J.1.9), 4 cyclopropyl-N-(2,4-di¬methoxy-phenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclo¬mezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flusulfamide (K.1.15), flutianil (K.1.16), harpin (K.1.17), metha¬sulfocarb (K.1.18), nitrapyrin (K.1.19), nitrothal-isopropyl (K.1.20), tolprocarb (K.1.21), oxin-copper (K.1.22), proquinazid (K.1.23), tebufloquin (K.1.24), tecloftalam (K.1.25), triazoxide (K.1.26), N'-(4-(4-chloro-3-trifluoro¬methyl-phen¬oxy)-2,5-dimethyl-phenyl)-N-ethyl-N methyl formamidine (K.1.27), N' (4-(4-fluoro-3-trifluoro-methyl-phenoxy)-2,5-dimethyl-phenyl)-N-eth¬yl-N-methyl formamidine (K.1.28), N'-[4-[[3-[(4-chlorophenyl)methyl]-1,2,4-thiadiazol-5-yl]¬oxy]-2,5-dimethyl-phenyl]-N-ethyl-N-methyl-formamidine (K.1.29), N'-(5-bromo-6-indan-2-yl¬oxy-2-methyl-3-pyridyl)-N-ethyl-N-methyl-formamidine (K.1.30), N'-[5-bromo-6-[1-(3,5-diflu¬orophenyl)ethoxy]-2-methyl-3-pyridyl]-N-ethyl-N-methyl-formamidine (K.1.31), N'-[5-bromo-6-(4-isopropylcyclohexoxy)-2-methyl-3-pyridyl]-N-ethyl-N-methyl-formamidine (K.1.32), N' [5 bromo-2-methyl-6-(1-phenylethoxy)-3-pyridyl]-N-ethyl-N-methyl-formamidine (K.1.33), N'-(2-methyl-5-trifluoromethyl-4-(3-trimethyl¬silanyl-prop¬oxy)-phenyl)-N-ethyl-N-methyl forma¬midine (K.1.34), N'-(5-difluoromethyl-2 methyl-4-(3-tri-methylsilanyl-propoxy)-phenyl)-N-ethyl-N-methyl formamidine (K.1.35), 2-(4-chloro-phenyl)-N-[4-(3,4-dimeth¬oxy-phenyl)-isoxazol-5 yl]-2-prop-2-ynyloxy-acetamide (K.1.36), 3 [5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3 yl]-pyridine (K.1.38), 5-chloro-1 (4,6-di-methoxy-pyrimidin-2-yl)-2-methyl-1H-benzoimidazole (K.1.39), ethyl (Z) 3 amino-2-cyano-3-phenyl-prop-2-enoate (K.1.40), picarbutrazox (K.1.41), pentyl N-[6-[[(Z)-[(1-methyltetrazol-5-yl)-phenylmethylene]amino]oxy¬methyl]-2-pyridyl]carba¬mate (K.1.42), but-3-ynyl N-[6-[[(Z)-[(1-methyltetrazol-5-yl)-phenyl-methylene]amino]oxy¬methyl]-2-pyridyl]carbamate (K.1.43), ipflufenoquin (K.1.44), quinofumelin (K.1.47), benziothiazolinone (K.1.48), bromothalonil (K.1.49), 2-(6-benzyl-2-pyridyl)quinazoline (K.1.50), 2-[6-(3-fluoro-4 methoxy-phenyl)-5-methyl-2-pyridyl]¬quinazoline (K.1.51), dichlobentiazox (K.1.52), N'-(2,5-dimethyl-4-phen¬oxy-phenyl)-N-ethyl-N-methyl-formamidine (K.1.53), aminopyrifen (K.1.54), fluopimomide (K.1.55), N'-[5-bromo-2-methyl-6-(1-methyl-2 propoxy-ethoxy)-3-pyridyl]-N-ethyl-N-methyl-formamidine (K.1.56), N'-[4-(4,5-dichlorothiazol-2-yl)oxy-2,5-dimethyl-phenyl]-N-ethyl-N-methyl-formamidine (K.1.57), flufenoxadiazam (K.1.58), N-methyl-4-[5-(trifluoromethyl)-1,2,4-oxadiazol-3-yl]benzenecarbothioamide (K.1.59), flumetylsulforim (K.1.60), seboctylamine (K.1.61);
L) Biopesticides
   L1) Microbial pesticides with fungicidal, bactericidal, viricidal and/or plant defense activator activity: Ampelomyces quisqualis, Aspergillus flavus, Aureobasidium pullulans, Bacillus altitudinis, B. amyloliquefaciens, B. amyloliquefaciens ssp. plantarum (also referred to as B. velezensis), B. megaterium, B. mojavensis, B. mycoides, B. pumi¬lus, B. simplex, B. solisalsi, B. subtilis, B. subtilis var. amyloliquefaciens, B. velezensis, Candida oleo¬phila, C. saitoana, Clavibacter michiga-nensis (bacteriophages), Coniothyrium minitans, Cryphonectria parasitica, Cryptococcus albidus, Dilophosphora alopecuri, Fusarium oxysporum, Clonostachys rosea f. catenulate (also named Gliocladium catenulatum), Gliocladium roseum, Lysobacter antibioticus, L. enzymogenes, Metschnikowia fructi¬cola, Microdochium dimerum, Microsphaeropsis ochracea, Muscodor albus, Paeni¬bacillus alvei, Paenibacillus epiphyticus, P. polymyxa, Pantoea vagans, Penicillium bilaiae, Phlebiopsis gigantea, Pseudomonas sp., Pseudomonas chloraphis, Pseudo¬zyma flocculosa, Pichia anomala, Pythium oligandrum, Sphaerodes myco¬parasitica, Streptomyces griseoviridis, S. lydicus, S. violaceusniger, Talaromyces flavus, Tricho-derma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia, zucchini yellow mosaic virus (avirulent strain);
   L2) Biochemical pesticides with fungicidal, bactericidal, viricidal and/or plant defense activator activity: harpin protein, Reynoutria sachalinensis extract;
   L3) Microbial pesticides with insecticidal, acaricidal, molluscidal and/or nematicidal activity: Agrobacterium radiobacter, Bacillus cereus, B. firmus, B. thuringiensis, B. thuringiensis ssp. aizawai, B. t. ssp. israelensis, B. t. ssp. galleriae, B. t. ssp. kurstaki, B. t. ssp. tene¬brionis, Beauveria bassiana, B. brongniartii, Burkholderia spp., Chromobacterium sub¬tsugae, Cydia pomonella granulovirus (CpGV), Cryptophlebia leucotreta granulovirus (CrleGV), Flavobacterium spp., Helicoverpa armigera nucleopolyhedrovirus (HearNPV), Helicoverpa zea nucleopolyhedrovirus (HzNPV), Helicoverpa zea single capsid nucleopolyhedrovirus (HzSNPV), Heterorhabditis bacteriophora, Isaria fumoso¬rosea, Lecanicillium longispo¬rum, L. muscarium, Metarhizium anisopliae, M. anisopliae var. anisopliae, M. anisopliae var. acridum, Nomuraea rileyi, Paecilomyces fumoso¬roseus, P. lilacinus, Paenibacillus popilliae, Pasteuria spp., P. nishizawae, P. pene¬trans, P. ramosa, P. thornea, P. usgae, Pseudomonas fluorescens, Spodoptera litto¬ralis nucleopoly¬hedrovirus (SpliNPV), Steinernema carpocapsae, S. feltiae, S. kraussei, Streptomyces galbus, S. microflavus;
   L4) Biochemical pesticides with insecticidal, acaricidal, molluscidal, pheromone and/or nematicidal activity: L-carvone, citral, (E,Z)-7,9-dodecadien-1-yl acetate, ethyl formate, (E,Z)-2,4-ethyl decadienoate (pear ester), (Z,Z,E)-7,11,13-hexadecatrienal, heptyl buty¬rate, isopropyl myristate, lavanulyl senecioate, cis-jasmone, 2-methyl 1-butanol, methyl eugenol, methyl jasmonate, (E,Z)-2,13-octadecadien-1-ol, (E,Z)-2,13-octadeca¬dien-1-ol acetate, (E,Z)-3,13-octadecadien-1-ol, (R)-1-octen-3-ol, pentatermanone, (E,Z,Z) 3,8,11-tetradecatrienyl acetate, (Z,E) 9,12-tetradecadien-1-yl acetate, (Z) 7 tetradecen-2-one, (Z)-9-tetradecen-1-yl acetate, (Z)-11-tetradecenal, (Z)-11-tetra¬decen-1-ol, extract of Chenopodium ambrosiodes, Neem oil, Quillay extract;
   L5) Microbial pesticides with plant stress reducing, plant growth regulator, plant growth promoting and/or yield enhancing activity: Azospirillum amazonense, A. brasilense, A. lipoferum, A. irakense, A. halopraeferens, Bradyrhizobium spp., B. elkanii, B. japo¬ni-cum, B. liaoningense, B. lupini, Delftia acidovorans, Glomus intraradices, Mesorhizo-bium spp., Rhizobium leguminosarum bv. pha¬seoli, R. I. bv. trifolii, R. I. bv. viciae, R. tropici, Sinorhizobium meliloti;The fungicides described by common names, their preparation and their activity e.g. against harmful fungi is known (cf.: http://www.alanwood.net/pesticides/); these substances are commercially available.

The active substances referred to as component 2, their preparation and their activity e.g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP 141 317; EP 152 031; EP 226 917; EP 243 970; EP 256 503; EP 428 941; EP 532 022; EP 1 028 125; EP 1 035 122; EP 1 201 648; EP 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441). Some compounds are identified by their CAS Registry Number.

Suitable mixing partners for the compounds of the invention also include biopesticides. Biopesticides have been defined as a form of pesticides based on micro-organisms (bacteria, fungi, viruses, nematodes, etc.) or natural products (compounds, e.g. metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances or or structurally-similar and functionally identical to a naturally-occurring substance and extracts from biological sources that control pests or provide other crop protection uses as defined below, but have non-toxic mode of actions (e.g. growth or developmental regulation, attractants, repellents or defence activators (e.g. induced resistance) and are relatively non-toxic to mammals.

The following list of biopesticides, in conjunction with which the compounds of the invention can be used, illustrates the possible combinations:
L) Biopesticides
L1) Microbial pesticides with fungicidal, bactericidal, viricidal and/or plant defense activator activity: *Ampelomyces quisqualis*, *Aspergillus flavus*, *Aureobasidium pullulans*, *Bacillus altitudinis, B. amyloliquefaciens, B. amyloliquefaciens* ssp. *plantarum (B. velezensis), B. megaterium, B. mojavensis, B. mycoides, B. pumilus, B. simplex, B. solisalsi, B. subtilis, B. subtilis* var. *amyloliquefaciens*, *B. velezensis*, *Candida oleophila*, C. *saitoana*, *Clavibacter michiganensis* (bacteriophages), *Coniothyrium minitans*, *Cryphonectria parasitica*, *Cryptococcus albidus*, *Dilophosphora alopecuri*, *Fusarium oxysporum*, *Clonostachys rosea* f. *catenulate (Gliocladium catenulatum), Gliocladium roseum, Lysobacter antibioticus, L. enzymogenes, Metschnikowia fructicola, Microdochium dimerum, Microsphaeropsis ochracea, Muscodor albus, Paenibacillus alvei, Paenibacillus epiphyticus*, *P. polymyxa, Pantoea vagans, Penicillium bilaiae*, *Phlebiopsis gigantea*, *Pseudomonas* sp., *Pseudomonas chloraphis*, *Pseudo-zyma flocculosa, Pichia anomala, Pythium oligandrum*, *Sphaerodes mycoparasitica, Streptomyces griseoviridis, S. lydicus, S. violaceusniger, Talaromyces flavus, Trichoderma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia*, zucchini yellow mosaic virus (avirulent strain);
L2) Biochemical pesticides with fungicidal, bactericidal, viricidal and/or plant defense activator activity: harpin protein, *Reynoutria sachalinensis* extract;
L3) Microbial pesticides with insecticidal, acaricidal, molluscidal and/or nematicidal activity: *Agrobacterium radiobacter*, *Bacillus cereus*, *B. firmus*, *B. thuringiensis*, *B. thuringiensis* ssp. *aizawai*, *B. t.* ssp. *israelensis*, *B. t.* ssp. *galleriae*, *B. t.* ssp. *kurstaki*, *B. t.* ssp. *tenebrionis*, *Beauveria bassiana*, *B. brongniartii*, *Burkholderia* spp., *Chromobacterium subtsugae*, *Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpa zea* nucleopolyhedrovirus (HzNPV), *Helicoverpa zea* single capsid nucleopolyhedrovirus (HzSNPV), *Heterorhabditis bacteriophora, Isaria fumosorosea, Lecanicillium longisporum, L. muscarium, Metarhizium anisopliae*, *M. anisopliae* var. *anisopliae*, *M. anisopliae* var. *acridum*, *Nomuraea rileyi*, *Paecilomyces fumosoroseus, P. lilacinus, Paenibacillus popilliae, Pasteuria* spp., *P*. *nishizawae*, *P. penetrans*, P. *ramosa*, *P. thornea*, *P. usgae*, *Pseudomonas fluorescens*, *Spodoptera littoralis* nucleopolyhedrovirus (SpliNPV), *Steinernema carpocapsae*, *S. feltiae*, *S. kraussei, Streptomyces galbus, S. microflavus;*
L4) Biochemical pesticides with insecticidal, acaricidal, molluscidal, pheromone and/or nematicidal activity: L-carvone, citral, (*E*,*Z*)-7,9-dodecadien-1-yl acetate, ethyl formate, (*E*,*Z*)-2,4-ethyl decadienoate (pear ester), (*Z*,*Z*,*E*)-7,11,13-hexadecatrienal, heptyl butyrate, isopropyl myristate, lavanulyl senecioate, cis-jasmone, 2-methyl 1-butanol, methyl eugenol, methyl jasmonate, (*E*,*Z*)-2,13-octadecadien-1-ol, (*E*,*Z*)-2,13-octadecadien-1-ol acetate, (*E*,*Z*)-3,13-octadecadien-1-ol, (*R*)-1-octen-3-ol, pentatermanone, (*E*,*Z*,*Z*)-3,8,11-tetradecatrienyl acetate, (*Z*,*E*)-9,12-tetra-decadien-1-yl acetate, (Z)-7-tetradecen-2-one, (Z)-9-tetradecen-1-yl acetate, (*Z*)-11-tetradece-nal, (*Z*)-11-tetradecen-1-ol, extract of *Chenopodium ambrosiodes*, Neem oil, Quillay extract;
L5) Microbial pesticides with plant stress reducing, plant growth regulator, plant growth promoting and/or yield enhancing activity: *Azospirillum amazonense*, *A. brasilense*, *A. lipoferum*, *A. irakense*, *A. halopraeferens*, *Bradyrhizobium* spp., *B. elkanii*, *B. japonicum*, *B. liaoningense*, *B. lupini*, *Delftia acidovorans*, *Glomus intraradices*, *Mesorhizobium* spp., *Rhizobium leguminosarum* bv. *phaseoli*, *R. I.* bv. *trifolii*, *R. I.* bv. *viciae*, *R. tropici*, *Sinorhizobium meliloti*;

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.

Many of these biopesticides have been deposited under deposition numbers mentioned herein (the prefixes e.g. ATCC or DSM refer to the acronym of the respective culture collection, for details see e.g. here: http://www. wfcc.info/ccinfo/collection/by acronym/), are referred to in literature, registered and/or are commercially available: mixtures of *Aureobasidium pullulans* DSM 14940 and DSM 14941 isolated in 1989 in Konstanz, Germany (e.g. blastospores in BlossomProtect^{®} from bio-ferm GmbH, Austria), *Azospirillum brasilense* Sp245 originally isolated in wheat reagion of South Brazil (Passo Fundo) at least prior to 1980 (BR 11005; e.g. GELFIX^{®} Gramíneas from BASF Agricultural Specialties Ltd., Brazil), *A. brasilense* strains Ab-V5 and Ab-V6 (e.g. in AzoMax from Novozymes BioAg Produtos papra Agricultura Ltda., Quattro Barras, Brazil or Simbiose-Maíz^{®} from Simbiose-Agro, Brazil; Plant Soil 331, 413-425, 2010), *Bacillus amyloliquefaciens* strain AP-188 (NRRL B-50615 and B-50331; US8,445,255); *B. amyloliquefaciens* ssp. *plantarum* strains formerly also sometimes referred to as B. *subtilis*, recently together with B. *methylotrophicus*, and B. *velezensis* classified as B. *velezensis* (Int. J. Syst. Evol. Microbiol. 66, 1212-1217, 2016): B. a. ssp. *plantarum* or B. *velezensis* D747 isolated from air in Kikugawa-shi, Japan (US 20130236522 A1; FERM BP-8234; e.g. Double Nickel^{™} 55 WDG from Certis LLC, USA), B. a. ssp. *plantarum* or B. *velezensis* FZB24 isolated from soil in Brandenburg, Germany (also called SB3615; DSM 96-2; J. Plant Dis. Prot. 105, 181-197, 1998; e.g. Taegro^{®} from Novozyme Biologicals, Inc., USA), B. a. ssp. *plantarum* or B. *velezensis* FZB42 isolated from soil in Brandenburg, Germany (DSM 23117; J. Plant Dis. Prot. 105, 181-197, 1998; e.g. RhizoVital^{®} 42 from AbiTEP GmbH, Germany), B. a. ssp. *plantarum* or B. *velezensis* MBI600 isolated from faba bean in Sutton Bonington, Nottinghamshire, U.K. at least before 1988 (also called 1430; NRRL B-50595; US 2012/0149571 A1; e.g. Integral^{®} from BASF Corp., USA), B. a. ssp. *plantarum* or B. *velezensis* QST-713 isolated from peach orchard in 1995 in California, U.S.A. (NRRL B-21661; e.g. Serenade^{®} MAX from Bayer Crop Science LP, USA), B. a. ssp. *plantarum* or B. *velezensis* TJ1000 isolated in 1992 in South Dakoda, U.S.A. (also called 1BE; ATCC BAA-390; CA 2471555 A1; e.g. QuickRoots^{™} from TJ Technologies, Watertown, SD, USA); *B. firmus* CNCM I-1582, a variant of parental strain EIP-N1 (CNCM I-1556) isolated from soil of central plain area of Israel (WO 2009/126473, US6,406,690; e.g. Votivo^{®} from Bayer CropScience LP, USA), *B. pumilus* GHA 180 isolated from apple tree rhizosphere in Mexico (IDAC 260707-01; e.g. PRO-MIX^{®} BX from Premier Horticulture, Quebec, Canada), *B. pumilus* INR-7 otherwise referred to as BU-F22 and BU-F33 isolated at least before 1993 from cucumber infested by *Erwinia tracheiphila* (NRRL B-50185, NRRL B-50153; US 8,445,255), *B. pumilus* KFP9F isolated from the rhizosphere of grasses in South Africa at least before 2008 (NRRL B-50754; WO 2014/029697; e.g. BAC-UP or FUSION-P from BASF Agricultural Specialities (Pty) Ltd., South Africa), *B. pumilus* QST 2808 was isolated from soil collected in Pohnpei, Federated States of Micronesia, in 1998 (NRRL B-30087; e.g. Sonata^{®} or Ballad^{®} Plus from Bayer Crop Science LP, USA), *B. simplex* ABU 288 (NRRL B-50304; US8,445,255), *B. subtilis* FB17 also called UD 1022 or UD10-22 isolated from red beet roots in North America (ATCC PTA-11857; System. Appl. Microbiol. 27, 372-379, 2004; US2010/0260735; WO 2011/109395); *B. thuringiensis* ssp. *aizawai* ABTS-1857 isolated from soil taken from a lawn in Ephraim, Wisconsin, U.S.A., in 1987 (also called ABG-6346; ATCC SD-1372; e.g. XenTari^{®} from BioFa AG, Münsingen, Germany), *B. t.* ssp. *kurstaki* ABTS-351 identical to HD-1 isolated in 1967 from diseased Pink Bollworm black larvae in Brownsville, Texas, U.S.A. (ATCC SD-1275; e.g. Dipel^{®} DF from Valent BioSciences, IL, USA), *B. t.* ssp. *kurstaki* SB4 isolated from *E. saccharina* larval cadavers (NRRL B-50753; e.g. Beta Pro^{®} from BASF Agricultural Specialities (Pty) Ltd., South Africa), *B. t.* ssp. *tenebrionis* NB-176-1, a mutant of strain NB-125, a wild type strain isolated in 1982 from a dead pupa of the beetle Tenebrio molitor (DSM 5480; EP 585 215 B1; e.g. Novodor^{®} from Valent BioSciences, Switzerland), *Beauveria bassiana* GHA (ATCC 74250; e.g. BotaniGard^{®} 22WGP from Laverlam Int. Corp., USA), B. *bassiana* JW-1 (ATCC 74040; e.g. Naturalis^{®} from CBC (Europe) S.r.l., Italy), B. *bassiana* PPRI 5339 isolated from the larva of the tortoise beetle *Conchyloctenia punctata* (NRRL 50757; e.g. Broad Band^{®} from BASF Agricultural Specialities (Pty) Ltd., South Africa), *Bradyrhizobium elkanii* strains SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e.g. GELFIX 5 from BASF Agricultural Specialties Ltd., Brazil), *B. japonicum* 532c isolated from Wisconsin field in U.S.A. (Nitragin 61A152; Can. J. Plant. Sci. 70, 661-666, 1990; e.g. in Rhizoflo^{®}, Histick^{®}, Hicoat^{®} Super from BASF Agricultural Specialties Ltd., Canada), *B. japonicum* E-109 variant of strain USDA 138 (INTA E109, SEMIA 5085; Eur. J. Soil Biol. 45, 28-35, 2009; Biol. Fertil. Soils 47, 81-89, 2011); B. *japonicum* strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e.g. GELFIX 5 or ADHERE 60 from BASF Agricultural Specialties Ltd., Brazil), *B. japonicum* SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e.g. GELFIX 5 or ADHERE 60 from BASF Agricultural Specialties Ltd., Brazil); *Burkholderia sp.* A396 isolated from soil in Nikko, Japan, in 2008 (NRRL B-50319; WO 2013/032693; Marrone Bio Innovations, Inc., USA), *Coniothyrium minitans* CON/M/91-08 isolated from oilseed rape (WO 1996/021358; DSM 9660; e.g. Contans^{®} WG, Intercept^{®} WG from Bayer CropScience AG, Germany), harpin (alpha-beta) protein (Science 257, 85-88, 1992; e.g. Messenger^{™} or HARP-N-Tek from Plant Health Care plc, U.K.), *Helicoverpa armigera* nucleopolyhedrovirus (HearNPV) (J. Invertebrate Pathol. 107, 112-126, 2011; e.g. Helicovex^{®} from Adermatt Biocontrol, Switzerland; Diplomata^{®} from Koppert, Brazil; Vivus^{®} Max from AgBiTech Pty Ltd., Queensland, Australia), *Helicoverpa zea* single capsid nucleopolyhedrovirus (HzSNPV) (e.g. Gemstar^{®} from Certis LLC, USA), *Helicoverpa zea* nucleopolyhedrovirus ABA-NPV-U (e.g. Heligen^{®} from AgBiTech Pty Ltd., Queensland, Australia), *Heterorhabditis bacteriophora* (e.g. Nemasys^{®} G from BASF Agricultural Specialities Limited, UK), *Isaria fumosorosea* Apopka-97 isolated from mealy bug on gynura in Apopka, Florida, U.S.A. (ATCC 20874; Biocontrol Science Technol. 22(7), 747-761, 2012; e.g. PFR-97^{™} or PreFeRal^{®} from Certis LLC, USA), *Metarhizium anisopliae* var. *anisopliae* F52 also called 275 or V275 isolated from codling moth in Austria (DSM 3884, ATCC 90448; e.g. Met52^{®} Novozymes Biologicals BioAg Group, Canada), *Metschnikowia fructicola* 277 isolated from grapes in the central part of Israel (US 6,994,849; NRRL Y-30752; e.g. formerly Shemer^{®} from Agrogreen, Israel), *Paecilomyces ilacinus* 251 isolated from infected nematode eggs in the Philippines (AGAL 89/030550; WO1991/02051; Crop Protection 27, 352-361, 2008; e.g. BioAct^{®}from Bayer CropScience AG, Germany and MeloCon^{®} from Certis, USA), *Paenibacillus alvei* NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), *Paenibacillus* strains isolated from soil samples from a variety of European locations including Germany: *P. epiphyticus* Lu17015 (WO 2016/020371; DSM 26971), *P. polymyxa* ssp. *plantarum* Lu16774 (WO 2016/020371; DSM 26969), *P. p.* ssp. *plantarum* strain Lu17007 (WO 2016/020371; DSM 26970); *Pasteuria nishizawae* Pn1 isolated from a soybean field in the mid-2000s in Illinois, U.S.A. (ATCC SD-5833; Federal Register 76(22), 5808, February 2, 2011; e.g. Clariva^{™} PN from Syngenta Crop Protection, LLC, USA), *Penicillium bilaiae* (also called P. *bilaii)* strains ATCC 18309 (= ATCC 74319), ATCC 20851 and/or ATCC 22348 (=ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e.g. Jump Start^{®}, Provide^{®} from Novozymes Biologicals BioAg Group, Canada), *Reynoutria sachalinensis* extract (EP 0307510 B1; e.g. Regalia^{®} SC from Marrone Biolnnovations, Davis, CA, USA or Milsana^{®} from BioFa AG, Germany), *Steinernema carpocapsae* (e.g. Millenium^{®} from BASF Agricultural Specialities Limited, UK), *S*. *feltiae* (e.g. Nemashield^{®} from BioWorks, Inc., USA; Nemasys^{®} from BASF Agricultural Specialities Limited, UK), *Streptomyces microflavus* NRRL B-50550 (WO 2014/124369; Bayer CropScience, Germany), *Trichoderma asperelloides* JM41R isolated in South Africa (NRRL 50759; also referred to as *T. fertile*; e.g. Trichoplus^{®} from BASF Agricultural Specialities (Pty) Ltd., South Africa), *T. harzianum* T-22 also called KRL-AG2 (ATCC 20847; BioControl 57, 687-696, 2012; e.g. Plantshield^{®} from BioWorks Inc., USA or SabrEx^{™} from Advanced Biological Marketing Inc., Van Wert, OH, USA).

According to the invention, the solid material (dry matter) of the biopesticides (with the exception of oils e.g. Neem oil) are considered as active components (e.g. to be obtained after drying or evaporation of the extraction or suspension medium in case of liquid formulations of the microbial pesticides).

In accordance with the invention, the weight ratios and percentages used herein for a biological extract e.g. Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1×10¹⁰ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, e.g. *Steinernema feltiae.*

When mixtures comprising microbial pesticides are employed in crop protection, the application rates range from 1×10⁶ to 5×10¹⁶ (or more) CFU/ha, preferably from 1×10⁸ to 1×10¹³ CFU/ha, and even more preferably from 1×10⁹ to 5×10¹⁵ CFU/ha and in particular from 1x10¹² to 5x10¹⁴ CFU/ha. In the case of nematodes as microbial pesticides (e.g. *Steinernema feltiae*), the application rates regularly range from 1×10⁵ to 1x10¹² (or more), preferably from 1×10⁸ to 1x10¹¹, more preferably from 5×10⁸ to 1×10¹⁰ individuals (e.g. in the form of eggs, juvenile or any other live stages, preferably in an infetive juvenile stage) per ha.

When mixtures comprising microbial pesticides are employed in seed treatment, the application rates generally range from 1×10⁶ to 1x10¹² (or more) CFU/seed, preferably from 1×10⁶ to 1×10⁹ CFU/seed. Furthermore, the application rates with respect to seed treatment generally range from 1×10⁷ to 1×10¹⁴ (or more) CFU per 100 kg of seed, preferably from 1×10⁹ to 1x10¹² CFU per 100 kg of seed.

The invention also relates to agrochemical compositions comprising an auxiliary and at least one compound of the invention or a mixture thereof.

An agrochemical composition comprises a pesticidally effective amount of a compound of the invention or a mixture thereof.

The compounds of the invention or the mixtures thereof can be converted into customary types of agrochemical compositions, e.g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials e.g. seeds (e.g. GF). These and further compositions types are defined in the "Catalogue of pesticide formulation types and international coding system", Technical Monograph No. 2, 6th Ed. May 2008, CropLife International.

The compositions are prepared in a known manner, e.g. described by Mollet and Grubenmann, Formulation technology, Wiley VCH, Weinheim, 2001; or Knowles, New developments in crop protection product formulation, Agrow Reports DS243, T&F Informa, London, 2005.

Examples for suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, e.g. mineral oil fractions of medium to high boiling point, e.g. kerosene, diesel oil; oils of vegetable or animal origin; hydrocarbons, e.g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, CaSO₄, MgSO₄, MgO; polysaccharide powders, e.g. cellulose, starch; fertilizers, e.g. (NH₄)₂SO₄, (NH₄)₃PO₄, NH₄NO₃, ureas; products of vegetable origin, e.g. cereal meal, tree bark meal, wood meal, nutshell meal, and mixtures thereof.

Suitable surfactants are surface-active compounds, e.g. anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds e.g. alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are homo- or copolymers of vinylpyrrolidone, vinylalcohols, or vinylacetate.

Suitable cationic surfactants are quaternary surfactants, e.g. quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compounds of the invention on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives e.g. alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo-, and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.
ii) Dispersible concentrates (DC)
   5-25 wt% of a compound I according to the invention and 1-10 wt% dispersant (e.g. polyvinylpyrrolidone) are dissolved in up to 100 wt% organic solvent (e.g. cyclohexanone). Dilution with water gives a dispersion.
iii) Emulsifiable concentrates (EC)
   15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.
iv) Emulsions (EW, EO, ES)
   5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.
v) Suspensions (SC, OD, FS)
   In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active sub-stance. For FS type composition up to 40 wt% binder (e.g. polyvinylalcohol) is added.
vi) Water-dispersible granules and water-soluble granules (WG, SG)
   50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e.g. extrusion, spray tower, fluidized bed). Dilution with water gives a stable dispersion or solution of the active substance.
vii) Water-dispersible powders and water-soluble powders (WP, SP, WS)
   50-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dispersion or solution of the active substance.
viii) Gel (GW, GF)
   In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active substance. Dilution with water gives a stable suspension of the active substance.
ix) Microemulsion (ME)
   5-20 wt% of a compound I according to the invention are added to 5-30 wt% organic solvent blend (e.g. fatty acid dimethylamide and cyclohexanone), 10-25 wt% surfactant blend (e.g. alkohol ethoxylate and arylphenol ethoxylate), and water up to 100 %. This mixture is stirred for 1 h to produce spontaneously a thermodynamically stable microemulsion.
x) Microcapsules (CS)
   An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol). The addition of a polyamine (e.g. hexamethylenediamine) results in the formation of a polyurea microcapsule. The monomers amount to 1-10 wt%. The wt% relate to the total CS composition.
xi) Dustable powders (DP, DS)
   1-10 wt% of a compound I according to the invention are ground finely and mixed intimately with up to 100 wt% solid carrier, e.g. finely divided kaolin.
xii) Granules (GR, FG)
   0.5-30 wt% of a compound I according to the invention is ground finely and associated with up to 100 wt% solid carrier (e.g. silicate). Granulation is achieved by extrusion, spray-drying or the fluidized bed.
xiii) Ultra-low volume liquids (UL)
   1-50 wt% of a compound I according to the invention are dissolved in up to 100 wt% organic solvent, e.g. aromatic hydrocarbon.

The compositions types i) to xi) may optionally comprise further auxiliaries, e.g. 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and most preferably between 0.5 and 75%, by weight of active substance. The active substances are employed in a purity of from 90% to 100%, preferably from 95% to 100% (according to NMR spectrum).

Various types of oils, wetters, adjuvants, fertilizer, or micronutrients, and other pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition of the invention e.g. parts of a kit or parts of a binary or ternary mixture may be mixed by the user himself in a spray tank and further auxiliaries may be added, if appropriate.

In a further embodiment, either individual components of the composition according to the invention or partially premixed components, e.g. components comprising compounds of the invention and/or mixing partners as defined above, may be mixed by the user in a spray tank and further auxiliaries and additives may be added.

In a further embodiment, either individual components of the composition according to the invention or partially premixed components, e.g. components comprising compounds of the invention and/or mixing partners as defined above, can be applied jointly (e.g. after tank mix) or consecutively.

The compounds of the invention are suitable for use in protecting crops, plants, plant propagation materials, e.g. seeds, or soil or water, in which the plants are growing, from attack or infestation by animal pests. Therefore, the invention also relates to a plant protection method, which comprises contacting crops, plants, plant propagation materials, e.g. seeds, or soil or water, in which the plants are growing, to be protected from attack or infestation by animal pests, with a pesticidally effective amount of a compound of the invention.

The compounds of the invention are also suitable for use in combating or controlling animal pests. Therefore, the invention also relates to a method of combating or controlling animal pests, which comprises contacting the animal pests, their habitat, breeding ground, or food supply, or the crops, plants, plant propagation materials, e.g. seeds, or soil, or the area, material or environment in which the animal pests are growing or may grow, with a pesticidally effective amount of a compound of the invention.

The compounds of the invention are effective through both contact and ingestion. Furthermore, the compounds of the invention can be applied to any and all developmental stages, e.g. egg, larva, pupa, and adult.

The compounds of the invention can be applied as such or in form of compositions comprising them as defined above. Furthermore, the compounds of the invention can be applied together with a mixing partner or in form of compositions comprising said mixtures. The components of said mixture can be applied simultaneously, jointly or separately, or in succession, that is immediately one after another and thereby creating the mixture "in situ" on the desired location, e.g. the plant, the sequence, in the case of separate application, generally not having any effect on the result of the control measures.

The application can be carried out both before and after the infestation of the crops, plants, plant propagation materials, e.g. seeds, soil, or the area, material or environment by the pests.

Suitable application methods include i.a. soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the pesticidally active compound to the furrow, and closing the furrow. Foliar application refers to the application of the pesticidally active compound to plant foliage, e.g. through spray equipment. For foliar applications, it can be advantageous to modify the behavior of the pests by use of pheromones in combination with the compounds of the invention. Suitable pheromones for specific crops and pests are known and publicly available from databases of pheromones and semiochemicals, e.g. http://www.pherobase.com.

The compounds of the invention and the compositions containing them may be applied in combination with, or by utilizing smart agricultural technologies, such as precision agriculture, remote and proximate imaging and image recognition, or smart agricultural site management programs. These smart agricultural technologies typically include models, e.g. computer programs, that support the user by considering information from a wide variety of sources to increase the quality and yield of harvested material, reduce damage by pests including the prediction of pest pressure and smart application of crop protection products, secure environmental protection, support quick and reliable agronomic decision making, reduce usage of fertilizers and crop protection products, reduce product residues in consumables increase spatial and temporal precision of agronomical measures, automate processes, and enable traceability of measures.

Commercially available systems which include agronomic models are e.g. FieldScripts^{™} from The Climate Corporation, Xarvio^{™} from BASF, AGLogic^{™} from John Deere, etc.

Information input for these models include but is not limited to soil data, information on the plants that are currently growing or that may grow at the area of interest including crop plants and/or unwanted vegetation, weather information, information on the location of the area and directly derivable information thereof, information on pest pressure, information on beneficial organisms, and / or historic information of any of the aforementioned.

The information usable for precision agriculture may be based on input by at least one user, be accessible from external data sources and databases, or be based on sensor data. Data sources typically includes proximate-detection systems like soil-borne sensors and remote sensing as may be achieved by imaging with unmanned airborne vehicles like drones, or satellites. Sensors may be included in an Internet-of-Things system and may be directly or indirectly connected to the processing unit, e.g. via a wireless network and/or cloud applications. The information is typically taken into account by at least one processing unit and used to provide recommendations and generate control signals.

Typical technologies that are used in smart agricultural technologies include self-steering robots (such as tractors, harvesters, drones), artificial intelligence (e.g. machine learning), imaging technologies (e.g. image segmentation technologies), big data analysis, and model generation, cloud computing, and machine-to-machine communication.

Precision agriculture such as precision farming is characterized by spatially and/or temporally resolved, targeted application of active ingredients like pesticides, plant-growth-regulators, fertilizers, and/or water including the variation of application rates over the agronomic site, zone or spot application, and of the spatially and/or temporally resolved, targeted planting or seeding of desired plant propagation material to a agronomic site. Precision farming typically includes the use of geo-positioning technologies like GPS for gaining information on the location and boundaries of the area of interest, the utilized application equipment, sensing equipment and recorded data, and to control the actions of farm vehicles such as spraying. By combining geo-positioning data with (digital) maps, it is possible to (semi)-automate agricultural measures at the site of interest, e.g. by using (semi)-autonomous spraying or seeding equipment.

Precision farming may typically include the application of smart spraying equipment, e.g. spot spraying, and precision spraying at a farm, e.g. by irrigation systems, tractors, robots, helicopters, airplanes, unmanned aerial vehicles, such as drones. Such equipment usually includes input sensors (such as e.g. a camera) and a processing unit configured to analyze the input data and configured to provide a recommendation or decision based on the analysis of the input data to apply the compounds of the invention or compositions comprising them to the agronomic site, e.g. the soil, the crop plants, or to control pests in a specific and precise manner. For example, pests may be detected, identified, and/or classified from imagery acquired by a camera. Such identification and/ classification can make use of image processing algorithms, which may utilize artificial intelligence (e.g. machine learning algorithms), or decision trees. In this manner, the compounds or compositions described herein can be applied only at the required location, point in time and dose rate.

As used herein, the term "contacting" includes both direct contact (applying the compounds/compositions directly on the animal pest or plant - typically to the foliage, stem or roots of the plant) and indirect contact (applying the compounds/compositions to the locus, i.e. habitat, breeding ground, plant, seed, soil, area, material, or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests.

The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet, or fodder beet; fruits, e.g. pomes, stone fruits, or soft fruits, e.g. apples, pears, plums, peaches, nectarines, almonds, cherries, papayas, strawberries, raspberries, blackberries or gooseberries; leguminous plants, e.g. beans, lentils, peas, alfalfa, or soybeans; oil plants, e.g. rapeseed (oilseed rape), turnip rape, mustard, olives, sunflowers, coconut, cocoa beans, castor oil plants, oil palms, ground nuts, or soybeans; cucurbits, e.g. squashes, pumpkins, cucumber or melons; fiber plants, e.g. cotton, flax, hemp, or jute; citrus fruit, e.g. oranges, lemons, grapefruits or mandarins; vegetables, e.g. eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, e.g. avocados, cinnamon, or camphor; energy and raw material plants, e.g. corn, soybean, rapeseed, sugar cane or oil palm; tobacco; nuts, e.g. walnuts; pistachios; coffee; tea; bananas; vines; hop; sweet leaf (Stevia); natural rubber plants or ornamental and forestry plants, shrubs, broad-leaved trees or evergreens, eucalyptus; turf; lawn; grass. Preferred plants include potatoes, sugar beets, tobacco, wheat, rye, barley, oats, rice, corn, cotton, soybeans, rapeseed, legumes, sunflowers, coffee, or sugar cane; fruits; vines; ornamentals; or vegetables, e.g. cucumbers, tomatoes, beans or squashes.

The term "cultivated plants" is to be understood as including plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or meganucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant in order to add a trait or improve a trait. These integrated genes are also referred to as transgenes in the art, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to ALS inhibitor herbicides by conventional methods of mutagenesis and breeding comprise plant varieties commercially available under the name Clearfield^{®}.

Herbicide tolerance has been created to glyphosate, glufosinate, 2,4-D, dicamba, oxynil herbicides, like bromoxynil and ioxynil, sulfonylurea herbicides, ALS inhibitor herbicides and HPPD inhibitors, like isoxaflutole and mesotrione.

Transgenes wich have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are e.g., but not excluding others, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are e.g., but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127.

Transgenic cotton events comprising herbicide tolerance genes are e.g., but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are e.g., but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants. Transgenes which have most frequently been used are toxin genes of *Bacillus spec.* and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinll. A further approach uses transgenes in order to produce double stranded RNA in plants to target and downregulate insect genes. An example for such a transgene is dvsnf7.

Transgenic corn events comprising genes for insecticidal proteins or double stranded RNA are e.g., but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are e.g., but not excluding others, MON87701, MON87751 and DAS-81419.

Transgenic cotton events comprising genes for insecticidal proteins are e.g., but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, being present in corn event MON87403, or by enhancing photosynthesis using the transgene bbx32, being present in the soybean event MON87712.

Cultivated plants comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are known (http://www.isaaa.org/gmapprovaldatabase) and (http://cera-gmc.org/GMCropDatabase).

Further information on specific events and methods to detect them can be found for canola events MS1, MS8, RF3, GT73, MON88302, KK179 in WO01/031042, WO01/041558, WO01/041558, WO02/036831, WO11/153186, WO13/003558, for cotton events MON1445, MON15985, MON531 (MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527, for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571, for potato events E12, F10, J3, J55, V11, X17, Y9 in WO14/178910, WO14/178913, WO14/178941, WO14/179276, WO16/183445, WO17/062831, WO17/062825, for rice events LLRICE06, LLRICE601, LLRICE62 in WO00/026345, WO00/026356, WO00/026345 for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compositions according to the invention on cultivated plants may result in effects which are specific to a cultivated plant comprising a certain gene or event. These effects may comprise enhanced yield, enhanced resistance or tolerance to insects, nematodes, fungal, bacterial, mycoplasma, viral or viroid pathogens as well as early vigour, early or delayed ripening, cold or heat tolerance as well as changed amino acid or fatty acid spectrum or content.

It has been found that the pesticidal activity of the compounds of the invention may be enhanced by the insecticidal trait of a modified plant. Furthermore, it has been found that the compounds of the invention are suitable for preventing insects to become resistant to the insecticidal trait or for combating pests, which already have become resistant to the insecticidal trait of a modified plant. Moreover, the compounds of the invention are suitable for combating pests, against which the insecticidal trait is not effective, so that a complementary insecticidal activity can advantageously be used.

The term "plant propagation material" refers to all the generative parts of the plant e.g. seeds and vegetative plant material e.g. cuttings and tubers (e.g. potatoes), which can be used for the multiplication of the plant. This includes seeds, roots, fruits, tubers, bulbs, rhizomes, shoots, sprouts and other parts of plants. Seedlings and young plants, which are to be transplanted after germination or after emergence from soil, may also be included. These plant propagation materials may be treated prophylactically with a plant protection compound either at or before planting or transplanting.

The term "seed" embraces seeds and plant propagules of all kinds including but not limited to true seeds, seed pieces, suckers, corms, bulbs, fruit, tubers, grains, cuttings, cut shoots and the like, and means in a preferred embodiment true seeds.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions e.g. desired pesticidal effect and duration, weather, target species, locus, mode of application.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hectare, 30 to 40 g per hectare, or 40 to 50 g per hectare.

The compounds of the invention are particularly suitable for use in the treatment of seeds in order to protect the seeds from insect pests, in particular from soil-living insect pests, and the resulting seedling's roots and shoots against soil pests and foliar insects. The invention therefore also relates to a method for the protection of seeds from insects, in particular from soil insects, and of the seedling's roots and shoots from insects, in particular from soil and foliar insects, said method comprising treating the seeds before sowing and/or after pregermination with a compound of the invention. The protection of the seedling's roots and shoots is preferred. More preferred is the protection of seedling's shoots from piercing and sucking insects, chewing insects and nematodes.

The term "seed treatment" comprises e.g. seed dressing, seed coating, seed dusting, seed soaking, seed pelleting, and in-furrow application methods. Preferably, the seed treatment application of the active compound is carried out by spraying or by dusting the seeds before sowing of the plants and before emergence of the plants.

The invention also comprises seeds coated with or containing the active compound. The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is e.g. seed of cereals, root crops, oil crops, vegetables, spices, ornamentals, e.g. seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugar beet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants e.g. potatoes, sugar cane, tobacco, grapes, petunias, geranium/pelargoniums, pansies and impatiens.

In addition, the active compound may also be used for the treatment of seeds from plants, which have been modified by mutagenesis or genetic engineering, and which e.g. tolerate the action of herbicides or fungicides or insecticides.

Conventional seed treatment formulations include e.g. flowable concentrates FS, solutions LS, suspoemulsions (SE), powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter. Preferably, the formulations are applied such that germination is not included.

The active substance concentrations in ready-to-use formulations, which may be obtained after two-to-tenfold dilution, are preferably from 0.01 to 60% by weight, more preferably from 0.1 to 40% by weight.

In a preferred embodiment a FS formulation is used for seed treatment. Typically, a FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.

Especially preferred FS formulations of the compounds of the invention for seed treatment usually comprise from 0.1 to 80% by weight (1 to 800 g/l) of the active ingredient, from 0.1 to 20% by weight (1 to 200 g/l) of at least one surfactant, e.g. 0.05 to 5% by weight of a wetter and from 0.5 to 15% by weight of a dispersing agent, up to 20% by weight, e.g. from 5 to 20% of an anti-freeze agent, from 0 to 15% by weight, e.g. 1 to 15% by weight of a pigment and/or a dye, from 0 to 40% by weight, e.g. 1 to 40% by weight of a binder (sticker/adhesion agent), optionally up to 5% by weight, e.g. from 0.1 to 5% by weight of a thickener, optionally from 0.1 to 2% of an anti-foam agent, and optionally a preservative e.g. a biocide, antioxidant or the like, e.g. in an amount from 0.01 to 1% by weight and a filler/vehicle up to 100% by weight.

In the treatment of seed, the application rates of the compounds of the invention are generally from 0.1 g to 10 kg per 100 kg of seed, preferably from 1 g to 5 kg per 100 kg of seed, more preferably from 1 g to 1000 g per 100 kg of seed and in particular from 1 g to 200 g per 100 kg of seed, e.g. from 1 g to 100 g or from 5 g to 100 g per 100 kg of seed.

The invention therefore also relates to seed comprising a compound of the invention, or an agriculturally useful salt thereof, as defined herein. The amount of the compound of the invention or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops e.g. lettuce the rate can be higher.

The compounds of the invention may also be used for improving the health of a plant. Therefore, the invention also relates to a method for improving plant health by treating a plant, plant propagation material and/or the locus where the plant is growing or is to grow with an effective and non-phytotoxic amount of a compound of the invention.

As used herein "an effective and non-phytotoxic amount" means that the compound is used in a quantity which allows to obtain the desired effect but which does not give rise to any phytotoxic symptom on the treated plant or on the plant grown from the treated propagule or treated soil.

"Plant health" is defined as a condition of the plant and/or its products which is determined by several aspects alone or in combination with each other e.g. yield (e.g. increased biomass and/or increased content of valuable ingredients), quality (e.g. improved content or composition of certain ingredients or shelf life), plant vigour (e.g. improved plant growth and/or greener leaves ("greening effect"), tolerance to abiotic (e.g. drought) and/or biotic stress (e.g. disease) and production efficiency (e.g., harvesting efficiency, processability).

The above identified indicators for the health condition of a plant may be interdependent and may result from each other. Each indicator is defined in the art and can be determined by methods known to a skilled person.

The compounds of the invention are also suitable for use against non-crop insect pests. For use against said non-crop pests, compounds of the invention can be used as bait composition, gel, general insect spray, aerosol, as ultra-low volume application and bed net (impregnated or surface applied). Furthermore, drenching and rodding methods can be used.

As used herein, the term "non-crop insect pest" refers to pests, which are particularly relevant for non-crop targets, e.g. ants, termites, wasps, flies, ticks, mosquitoes, bed bugs, crickets, or cockroaches.

The bait can be a liquid, a solid or a semisolid preparation (e.g. a gel). The bait employed in the composition is a product, which is sufficiently attractive to incite insects e.g. ants, termites, wasps, flies, mosquitoes, crickets etc. or cockroaches to eat it. The attractiveness can be manipulated by using feeding stimulants or sex pheromones. Food stimulants are preferably chosen from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are known (http://www.pherobase.com).

For use in bait compositions, the typical content of active ingredient is from 0.001 wt% to 15 wt%, desirably from 0.001 wt% to 5 wt% of active compound.

Formulations of the compounds of the invention as aerosols (e.g in spray cans), oil sprays or pump sprays are highly suitable for professional or non-professional users for controlling pests e.g. flies, fleas, ticks, bed bugs, mosquitoes or cockroaches. Aerosol recipes are preferably composed of the active compound, solvents, furthermore auxiliaries e.g. emulsifiers, perfume oils, if appropriate stabilizers, and, if required, propellants.

The oil spray formulations differ from the aerosol recipes in that no propellants are used.

For use in spray compositions, the content of active ingredient is from 0.001 to 80 wt%, preferably from 0.01 to 50 wt% and most preferably from 0.01 to 15 wt%.

The compounds of the invention and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of the invention and its respective compositions also comprise treating surfaces of huts and houses, air spraying and impregnation of curtains, tents, clothing items, bed nets, tsetse-fly trap. Insecticidal compositions for application to fibers, fabric, knitgoods, nonwovens, netting material or foils and tarpaulins preferably comprise a mixture including the insecticide, optionally a repellent and at least one binder.

The compounds of the invention and its compositions can be used for protecting wooden materials e.g. trees, board fences, sleepers, frames, artistic artifacts, etc. and buildings, but also construction materials, furniture, leathers, fibers, vinyl articles, electric wires and cables etc. from ants, termites and/or wood or textile destroying beetles, and for controlling ants and termites from doing harm to crops or human beings (e.g. when the pests invade into houses and public facilities or nest in yards, orchards or parks).

Customary application rates in the protection of materials are, e.g., from 0.001 g to 2000 g or from 0.01 g to 1000 g of active compound per m² treated material, desirably from 0.1 g to 50 g per m².

Insecticidal compositions for use in the impregnation of materials typically contain from 0.001 to 95 wt%, preferably from 0.1 to 45 wt%, and more preferably from 1 to 25 wt% of at least one repellent and/or insecticide.

The compounds of the invention are especially suitable for efficiently combating animal pests e.g. arthropods, gastropods and nematodes including:
insects from the order of Lepidoptera, e.g. *Achroia grisella*, *Acleris* spp. e.g. *A. fimbriana, A. gloverana, A. variana; Acrolepiopsis assectella, Acronicta major, Adoxophyes* spp. e.g. *A. cyrtosema, A. orana; Aedia leucomelas, Agrotis* spp. e.g. *A. exclamationis, A. fucosa, A. ipsilon, A. orthogoma, A. segetum, A. subterranea; Alabama argillacea, Aleurodicus dispersus, Alsophila pometaria, Ampelophaga rubiginosa, Amyelois transitella, Anacampsis sarcitella, Anagasta kuehniella, Anarsia lineatella, Anisota senatoria, Antheraea pernyi, Anticarsia (=Thermesia)* spp. e.g. *A. gemmatalis; Apamea* spp., *Aproaerema modicella, Archips* spp. e.g. *A. argyrospila, A. fuscocupreanus, A. rosana, A. xyloseanus; Argyresthia conjugella, Argyroploce* spp., *Argyrotaenia* spp. e.g. *A. velutinana; Athetis mindara, Austroasca viridigrisea, Autographa gamma, Autographa nigrisigna, Barathra brassicae, Bedellia* spp., *Bonagota salubricola, Borbo cinnara, Bucculatrix thurberiella, Bupalus piniarius, Busseola* spp., *Cacoecia* spp. e.g. C. *murinana, C. podana; Cactoblastis cactorum, Cadra cautella, Calingo braziliensis, Caloptilis theivora, Capua reticulana, Carposina* spp. e.g. C. *niponensis,* C. *sasakii; Cephus* spp., *Chaetocnema aridula, Cheimatobia brumata, Chilo* spp. e.g. C. *Indicus, C. suppressalis, C. partellus; Choreutis pariana, Choristoneura* spp. e.g. C. *conflictana, C. fumiferana, C. longicellana, C. murinana, C. occidentalis,* C. *rosaceana; Chrysodeixis (=Pseudoplusia)* spp. *e.g.* C. *eriosoma, C. includens; Cirphis unipuncta, Clysia ambiguella, Cnaphalocerus* spp., *Cnaphalocrocis medinalis, Cnephasia* spp., *Cochylis hospes, Coleophora* spp., *Colias eurytheme, Conopomorpha* spp., *Conotrachelus* spp., *Copitarsia* spp., *Corcyra cephalonica, Crambus caliginosellus, Crambus teterrellus, Crocidosema (=Epinotia) aporema, Cydalima (=Diaphania) perspectalis, Cydia (=Carpocapsa)* spp. e.g. C. *pomonella, C. latiferreana; Dalaca noctuides, Datana integerrima, Dasychira pinicola, Dendrolimus* spp. e.g. *D. pini, D. spectabilis, D. sibiricus; Desmia funeralis, Diaphania* spp. e.g. *D. nitidalis, D. hyalinata; Diatraea grandiosella, Diatraea saccharalis, Diphthera festiva, Earias* spp. e.g. *E. insulana, E. vittella; Ecdytolopha aurantianu, Egira (=Xylomyges) curialis, Elasmopalpus lignosellus, Eldana saccharina, Endopiza viteana, Ennomos subsignaria, Eoreuma loftini, Ephestia* spp. e.g. *E. cautella, E. elutella, E. kuehniella; Epinotia aporema, Epiphyas postvittana, Erannis tiliaria, Erionota thrax, Etiella* spp., *Eulia* spp., *Eupoecilia ambiguella, Euproctis chrysorrhoea, Euxoa* spp., *Evetria bouliana, Faronta albilinea, Feltia* spp. e.g. *F. subterranean; Galleria mellonella, Gracillaria* spp., *Grapholita* spp. e.g. *G. funebrana, G. molesta, G. inopinata; Halysidota* spp., *Harrisina americana*, *Hedylepta* spp., *Helicoverpa* spp. e.g. *H. armigera (=Heliothis armigera), H. zea (=Heliothis zea); Heliothis* spp. e.g. *H. assulta, H. subflexa, H. virescens; Hellula* spp. e.g. *H. undalis, H. rogatalis; Helocoverpa gelotopoeon, Hemileuca oliviae, Herpetogramma licarsisalis, Hibernia defoliaria, Hofmannophila pseudospretella, Homo*eosoma *electellum, Homona magnanima, Hypena scabra, Hyphantria cunea, Hyponomeuta padella, Hyponomeuta malinellus, Kakivoria flavofasciata, Keiferia lycopersicella, Lambdina fiscellaria fiscellaria, Lambdina fiscellaria lugubrosa, Lamprosema indicata, Laspeyresia molesta, Leguminivora glycinivorella, Lerodea eufala, Leucinodes orbonalis, Leucoma salicis, Leucoptera* spp. e.g. *L. coffeella, L. scitella; Leuminivora lycinivorella, Lithocolletis blancardella, Lithophane antennata, Llattia octo (=Amyna axis), Lobesia botrana, Lophocampa* spp., *Loxagrotis albicosta, Loxostege* spp. e.g. *L. sticticalis, L. cereralis; Lymantria* spp. e.g. *L. dispar, L. monacha; Lyonetia clerkella, Lyonetia prunifoliella, Malacosoma* spp. e.g. *M. americanum, M. californicum, M. constrictum, M. neustria; Mamestra* spp. e.g. *M. brassicae, M. configurata; Mamstra brassicae, Manduca* spp. e.g. *M. quinquemaculata, M. sexta; Marasmia spp, Marmara* spp., *Maruca testu-lalis, Megalopyge lanata, Melanchra picta, Melanitis leda, Mocis* spp. e.g. *M. lapites, M. repanda; Mocis latipes, Monochroa fragariae, Mythimna separata, Nemapogon cloacella, Neoleucino-des elegantalis, Nepytia* spp., *Nymphula* spp., *Oiketicus* spp., *Omiodes indicata, Omphisa anas-tomosalis, Operophtera brumata, Orgyia pseudotsugata, Oria* spp., *Orthaga thyrisalis, Ostrinia* spp. e.g. O. *nubilalis; Oulema oryzae, Paleacrita vernata, Panolis flammea, Parnara* spp., *Papaipema nebris, Papilio cresphontes, Paramyelois transitella, Paranthrene regalis, Paysandisia archon*, *Pectinophora* spp. e.g. *P. gossypiella; Peridroma saucia, Perileucoptera* spp., e.g. *P. coffeella; Phalera bucephala, Phryganidia californica, Phthorimaea* spp. e.g. P. *operculella; Phyllocnistis citrella, Phyllonorycter* spp. e.g. *P*. *blancardella, P. crataegella, P. issikii, P. ringoniella; Pieris* spp. e.g. *P. brassicae, P. rapae, P. napi; Pilocrocis tripunctata, Plathypena scabra, Platynota* spp. e.g. *P. flavedana, P. idaeusalis, P. stultana; Platyptilia carduidactyla, Plebejus argus, Plodia interpunctella, Plusia spp, Plutella maculipennis, Plutella xylostella, Pontia protodica, Prays* spp., *Prodenia* spp., *Proxenus lepigone, Pseudaletia* spp. e.g. *P. sequax, P. unipuncta; Pyrausta nubilalis, Rachiplusia nu, Richia albicosta, Rhizobius ventralis, Rhyacionia frustrana, Sabulodes aegrotata, Schizura concinna, Schoenobius* spp., *Schreckensteinia festaliella, Scirpophaga* spp. e.g. S. *incertulas, S. innotata; Scotia segetum, Sesamia* spp. e.g. S. *inferens, Seudyra subflava, Sitotroga cerealella, Sparganothis pilleriana, Spilonota lechriaspis, S. ocellina, Spodoptera (*=*Lamphygma)* spp. e.g. S. *cosmoides, S. eridania, S. exigua, S. frugiperda, S. latisfascia, S. littoralis, S. litura, S. omithogalli; Stigmella* spp., *Stomopteryx subsecivella, Strymon bazochii, Sylepta derogata, Synanthedon* spp. e.g. S. exitiosa, *Tecia solanivora, Telehin licus, Thaumatopoea pityocampa, Thaumatotibia (=Cryptophlebia) leucotreta, Thaumetopoea pityocampa, Thecla* spp., *Theresimima ampelophaga, Thyrinteina* spp, *Tildenia inconspicuella, Tinea* spp. e.g. *T. cloacella, T. pellionella; Tineola bisselliella, Tortrix* spp. e.g. *T. viridana; Trichophaga tapetzella, Trichoplusia* spp. e.g. *T. ni; Tuta (=Scrobipalpula) absoluta, Udea* spp. e.g. *U. rubigalis, U. rubigalis; Virachola* spp., *Yponomeuta padella, and Zeiraphera canadensis;* insects from the order of Coleoptera, e.g. *Acalymma vittatum, Acanthoscehdes obtectus, Adoretus* spp., *Agelastica alni, Agrilus* spp. e.g. *A. anxius, A. planipennis, A. sinuatus; Agriotes* spp. e.g. *A. fuscicollis, A. lineatus, A. obscurus; Alphitobius diaperinus, Amphimallus solstitialis, Anisandrus dispar, Anisoplia austriaca, Anobium punctatum, Anomala corpulenta, Anomala rufocuprea, Anoplophora* spp. e.g. *A. glabripennis; Anthonomus* spp. e.g. *A. eugenii, A. grandis, A. pomorum; Anthrenus* spp., *Aphthona euphoridae, Apion* spp., *Apogonia* spp., *Athous haemorrhoidalis, Atomaria* spp. e.g. *A. linearis; Attagenus* spp., *Aulacophora femoralis, Blastophagus piniperda, Blitophaga undata, Bruchidius obtectus, Bruchus* spp. e.g. *B. lentis, B. pisorum, B. rufimanus; Byctiscus betulae, Callidiellum rufipenne, Callopistria floridensis, Callosobruchus chinensis, Cameraria ohridella, Cassida nebulosa, Cerotoma trifurcata, Cetonia aurata, Ceuthorhynchus* spp. e.g. *C*. *assimilis, C. napi; Chaetocnema tibialis, Cleonus mendicus, Conoderus* spp. e.g. *C*. *vespertinus; Conotrachelus nenuphar, Cosmopolites* spp., *Costelytra zealandica, Crioceris asparagi, Cryptolestes ferrugineus, Cryptorhynchus lapathi, Ctenicera* spp. e.g. *C*. *destructor; Curculio* spp., *Cylindrocopturus* spp., *Cyclocephala* spp., *Dactylispa balyi, Dectes texanus, Dermestes* spp., *Diabrotica* spp. e.g. *D*. *undecimpunctata, D. speciosa, D. longicornis, D. semipunctata, D. virgifera; Diaprepes abbreviates, Dichocrocis* spp., *Dicladispa armigera, Diloboderus abderus, Diocalandra frumenti (Diocalandra stigmaticollis), Enaphalodes rufulus, Epilachna* spp. e.g. *E. varivestis, E. vigintioctomaculata; Epitrix* spp. e.g. *E. hirtipennis, E. similaris; Eutheola humilis, Eutinobothrus brasiliensis, Faustinus cubae, Gibbium psylloides, Gnathocerus cornutus, Hellula undalis, Heteronychus arator, Hylamorpha elegans, Hylobius abietis, Hylotrupes bajulus, Hypera* spp. e.g. *H. brunneipennis, H. postica; Hypomeces squamosus, Hypothenemus* spp., *Ips typographus, Lachnosterna consanguinea, Lasioderma serricorne, Latheticus oryzae, Lathridius* spp., *Lema* spp. e.g. *L. bilineata, L. melanopus; Leptinotarsa* spp. e.g. *L. decemlineata; Leptispa pygmaea, Limonius californicus, Lissorhoptrus oryzophilus, Lixus* spp., *Luperodes* spp., *Lyctus* spp. e.g. *L. bruneus; Liogenys fuscus, Macrodactylus* spp. e.g. *M. subspinosus; Maladera matrida, Megaplatypus mutates, Megascelis* spp., *Melanotus communis, Meligethes* spp. e.g. *M. aeneus; Melolontha* spp. e.g. *M. hippocastani, M. melolontha; Metamasius hemipterus, Microtheca* spp., *Migdolus* spp. e.g. *M. fryanus, Monochamus* spp. e.g. *M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetonia jucunda, Phaedon* spp. e.g. *P. brassicae, P. cochleariae; Phoracantha recurva, Phyllobius pyri, Phyllopertha horticola, Phyllophaga* spp. e.g. *P. helleri; Phyllotreta* spp. e.g. *P*. *chrysocephala, P. nemorum, P. striolata, P. vittula; Phyllopertha horticola, Popillia japonica, Premnotrypes* spp., *Psacothea hilaris, Psylliodes chrysocephala, Prostephanus truncates, Psylliodes* spp., *Ptinus* spp., *Pulga saltona, Rhizopertha dominica, Rhynchophorus* spp. e.g. *R*. *billineatus, R. ferrugineus, R. palmarum, R. phoenicis, R. vulneratus; Saperda candida, Scolytus schevyrewi, Scyphophorus acupunctatus, Sitona lineatus, Sitophilus* spp. e.g. *S*. *granaria, S*. *oryzae, S*. *zeamais; Sphenophorus* spp. e.g. *S*. *levis; Stegobium paniceum, Sternechus* spp. e.g. *S*. *subsignatus; Strophomorphus ctenotus, Symphyletes* spp., *Tanymecus* spp., *Tenebrio molitor, Tenebrioides mauretanicus, Tribolium* spp. e.g. *T. castaneum; Trogoderma* spp., *Tychius* spp., *Xylotrechus* spp. e.g. X. *pyrrhoderus; and, Zabrus* spp. *e.g. Z. tenebrioides;* insects from the order of Diptera e.g. *Aedes* spp. e.g. *A. aegypti, A. albopictus, A. vexans; Anastrepha ludens, Anopheles* spp. e.g. *A. albimanus, A. crucians, A. freeborni, A. gambiae, A. leucosphyrus, A. maculipennis, A. minimus, A. quadrimaculatus, A. sinensis; Bactrocera invadens, Bibio hortulanus, Calliphora erythrocephala, Calliphora vicina, Ceratitis capitata, Chrysomyia* spp. e.g. *C*. *bezziana, C. hominivorax, C. macellaria; Chrysops atlanticus, Chrysops discalis, Chrysops silacea, Cochliomyia* spp. e.g. *C*. *hominivorax; Contarinia* spp. e.g. *C*. *sorghicola; Cordylobia anthropophaga, Culex* spp. e.g. *C*. *nigripalpus, C. pipiens, C. quinquefasciatus, C. tarsalis, C. tritaeniorhynchus; Culicoides furens, Culiseta inornata, Culiseta melanura, Cuterebra* spp., *Dacus cucurbitae, Dacus oleae, Dasineura brassicae, Dasineura oxycoccana, Delia* spp. e.g. *D*. *antique, D. coarctata, D. platura, D. radicum; Dermatobia hominis, Drosophila* spp. e.g. *D. suzukii, Fannia* spp. e.g. *F. canicularis; Gastraphilus* spp. e.g. *G. intestinalis; Geomyza tipunctata, Glossina* spp. e.g. *G. fuscipes, G. morsitans, G. palpalis, G. tachinoides; Haematobia irritans, Haplodiplosis equestris, Hippelates* spp., Hylemyia spp. e.g. *H. platura; Hypoderma* spp. e.g. *H. lineata; Hyppobosca* spp., *Hydrellia philippina, Leptoconops torrens, Liriomyza* spp. e.g. *L. sativae, L. trifolii; Lucilia* spp. e.g. *L. caprina, L. cuprina, L. sericata; Lycoria pectoralis, Mansonia titillanus, Mayetiola* spp. e.g. *M. destructor; Musca* spp. e.g. *M. autumnalis, M. domestica; Muscina stabulans, Oestrus* spp. e.g. *O*. *ovis; Opomyza florum, Oscinella* spp. e.g. *O*. *frit; Orseolia oryzae, Pegomya hysocyami, Phlebotomus argentipes, Phorbia* spp. e.g. *P*. *antiqua, P. brassicae, P. coarctata; Phytomyza gymnostoma, Prosimulium mixtum, Psila* rosae, *Psorophora columbiae, Psorophora discolor, Rhagoletis* spp. e.g. *R. cerasi, R. cingulate, R. indifferens, R. mendax, R. pomonella; Rivellia quadrifasciata, Sarcophaga* spp. e.g. *S*. *haemorrhoidalis; Simulium vittatum, Sitodiplosis mosellana, Stomoxys* spp. e.g. *S*. *calcitrans; Tabanus* spp. e.g. *T. atratus, T. bovinus, T. lineola, T. similis; Tannia spp., Thecodiplosis japonensis, Tipula oleracea, Tipula paludosa,* and *Wohlfahrtia* spp;
insects from the order of Thysanoptera e.g., *Baliothrips biformis, Dichromothrips corbetti, Dichromothrips* ssp., *Echinothrips americanus, Enneothrips flavens, Frankliniella* spp. e.g. *F*. *fusca, F. occidentalis, F. tritici; Heliothrips* spp., *Hercinothrips femoralis, Kakothrips* spp., *Microcephalothrips abdominalis, Neohydatothrips samayunkur, Pezothrips kellyanus, Rhipiphorothrips cruentatus, Scirtothrips* spp. e.g. *S*. *citri, S*. *dorsalis, S. perseae; Stenchaetothrips spp, Taeniothrips cardamoni, Taeniothrips inconsequens, Thrips* spp. e.g. *T. imagines, T. hawaiiensis, T. oryzae, T. palmi, T. parvispinus, T. tabaci;*
insects from the order of Hemiptera e.g., *Acizzia jamatonica, Acrosternum* spp. e.g. *A. hilare;* Acyrthosipon spp. e.g. *A. onobrychis, A. pisum; Adelges laricis, Adelges tsugae, Adelphocoris* spp., e.g. *A. rapidus, A. superbus; Aeneolamia* spp., *Agonoscena* spp., *Aulacorthum solani, Aleurocanthus woglumi, Aleurodes* spp., *Aleurodicus disperses, Aleurolobus barodensis, Aleurothrixus* spp., *Amrasca* spp., *Anasa tristis, Antestiopsis* spp., *Anuraphis cardui, Aonidiella* spp., *Aphanostigma piri, Aphidula nasturtii, Aphis* spp. e.g. *A. craccivora, A. fabae, A. forbesi, A. gossypii, A. grossulariae, A. maidiradicis, A. pomi, A. sambuci, A. schneideri, A. spiraecola; Arboridia apicalis, Arilus critatus, Aspidiella* spp., *Aspidiotus* spp., *Atanus* spp., *Aulacaspis yasumatsui, Aulacorthum solani, Bactericera cockerelli* (*Paratrioza cockerelli), Bemisia* spp. e.g. *B*. *argentifolii, B. tabaci (Aleurodes tabaci); Blissus* spp. e.g. *B. leucopterus; Brachycaudus* spp. e.g. *B. cardui, B. helichrysi, B. persicae, B. prunicola; Brachycolus* spp., *Brachycorynella asparagi, Brevicoryne brassicae, Cacopsylla* spp. e.g. *C*. *fulguralis, C. pyricola (Psylla piri); Calligypona marginata, Calocoris* spp., *Campylomma livida, Capitophorus horni, Carneocephala fulgida, Cavelerius* spp., *Ceraplastes* spp., *Ceratovacuna lanigera, Ceroplastes ceriferus, Cerosipha gossypii, Chaetosiphon fragaefolii, Chionaspis tegalensis, Chlorita onukii, Chromaphis juglandicola, Chrysomphalus ficus, Cicadulina mbila, Cimex* spp. e.g. *C*. *hemipterus, C. lectularius; Coccomytilus halli, Coccus* spp. e.g. *C*. *hesperidum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus* spp., *Dasynus piperis, Dialeurodes* spp. e.g. *D. citrifolii; Dalbulus maidis, Diaphorina* spp. e.g. *D. citri; Diaspis* spp. e.g. *D. bromeliae; Dichelops furcatus, Diconocoris hewetti, Doralis* spp., *Dreyfusia nordmannianae, Dreyfusia piceae, Drosicha* spp., *Dysaphis* spp. e.g. *D. plantaginea, D. pyri, D. radicola; Dysaulacorthum pseudosolani, Dysdercus* spp. e.g. *D*. *cingulatus, D. intermedius; Dysmicoccus* spp., *Edessa* spp., *Geocoris* spp., *Empoasca* spp. e.g. *E. fabae, E. solana; Epidiaspis leperii, Eriosoma* spp. e.g. *E. lanigerum, E. pyricola; Erythroneura* spp., *Eurygaster* spp. e.g. *E. integriceps; Euscelis bilobatus, Euschistus* spp. e.g. *E. heros, E. impictiventris, E. servus; Fiorinia theae, Geococcus coffeae, Glycaspis brimblecombei, Halyomorpha* spp. e.g. *H. halys; Heliopeltis* spp., *Homalodisca vitripennis (=H. coagulata), Horcias nobilellus, Hyalopterus pruni, Hyperomyzus lactucae, Icerya* spp. e.g. *I. purchase; Idiocerus* spp., *Idioscopus* spp., *Laodelphax striatellus, Lecanium* spp., *Lecanoideus floccissimus, Lepidosaphes* spp. e.g. *L. ulmi; Leptocorisa* spp., *Leptoglossus phyllopus, Lipaphis erysimi, Lygus* spp. e.g. *L. hesperus, L. lineolaris, L. pratensis; Maconellicoccus hirsutus, Marchalina hellenica, Macropes excavatus, Macrosiphum* spp. e.g. *M.* rosae, *M. avenae, M. euphorbiae; Macrosteles quadrilineatus, Mahanarva fimbriolata, Megacopta cribraria, Megoura viciae, Melanaphis pyrarius, Melanaphis sacchari, Melanocallis (=Tinocallis) caryaefoliae, Metcafiella* spp., *Metopolophium dirhodum, Monellia costalis, Monelliopsis pecanis, Myzocallis coryli, Murgantia* spp., *Myzus* spp. e.g. *M. ascalonicus, M. cerasi, M. nicotianae, M. persicae, M. varians; Nasonovia ribis-nigri, Neotoxoptera formosana, Neomegalotomus spp, Nephotettix* spp. e.g. *N. malayanus, N. nigropictus, N. parvus, N. virescens; Nezara* spp. e.g. *N*. *viridula; Nilaparvata lugens, Nysius huttoni, Oebalus* spp. e.g. *O*. *pugnax; Oncometopia* spp., *Orthezia praelonga, Oxycaraenus hyalinipennis, Parabemisia myricae, Parlatoria* spp., *Parthenolecanium* spp. e.g. *P. corni, P. persicae; Pemphigus* spp. e.g. *P. bursarius, P. populivenae; Peregrinus maidis, Perkinsiella saccharicida, Phenacoccus* spp. e.g. *P*. *aceris, P. gossypii; Phloeomyzus passerinii, Phorodon humuli, Phylloxera* spp. e.g. *P. devastatrix, Piesma quadrata, Piezodorus* spp. e.g. *P. guildinii; Pinnaspis aspidistrae, Planococcus* spp. e.g. *P. citri, P. ficus; Prosapia bicincta, Protopulvinaria pyriformis, Psallus seriatus, Pseudacysta persea, Pseudaulacaspis pentagona, Pseudococcus* spp. e.g. *P. comstocki; Psylla* spp. e.g. *P*. *mali; Pteromalus* spp., *Pulvinaria amygdali, Pyrilla* spp., *Quadraspidiotus* spp., e.g. *Q*. *perniciosus; Quesada gigas, Rastrococcus* spp., *Reduvius senilis, Rhizoecus americanus, Rhodnius* spp., *Rhopalomyzus ascalonicus, Rhopalosiphum* spp. e.g. *R. pseudobrassicas, R. insertum, R. maidis, R. padi; Sagatodes* spp., *Sahlbergella singularis, Saissetia* spp., *Sappaphis mala, Sappaphis mali, Scaptocoris* spp., *Scaphoides titanus, Schizaphis graminum, Schizoneura lanuginosa, Scotinophora* spp., *Selenaspidus articulatus, Sitobion avenae, Sogata* spp., *Sogatella furcifera, Solubea insularis, Spissistilus festinus (=Stictocephala festina), Stephanitis nashi, Stephanitis pyrioides, Stephanitis takeyai, Tenalaphara malayensis, Tetraleurodes perseae, Therioaphis maculate, Thyanta* spp. e.g. *T. accerra, T. perditor; Tibraca* spp., *Tomaspis* spp., *Toxoptera* spp. e.g. *T. aurantii; Trialeurodes* spp. e.g. *T. abutilonea, T. ricini, T. vaporariorum; Triatoma* spp., *Trioza* spp., *Typhlocyba* spp., *Unaspis* spp. e.g. *U. citri, U. yanonensis; and Viteus vitifolii,*
Insects from the order Hymenoptera e.g. *Acanthomyops interjectus, Athalia* rosae, *Atta* spp. *e.g. A. capiguara, A. cephalotes, A. cephalotes, A. laevigata, A. robusta, A. sexdens, A. texana, Bombus* spp., *Brachymyrmex* spp., *Camponotus* spp. e.g. *C*. *floridanus, C. pennsylvanicus, C. modoc; Cardiocondyla nuda, Chalibion sp, Crematogaster* spp., *Dasymutilla occidentalis, Diprion* spp., *Dolichovespula maculata, Dorymyrmex* spp., *Dryocosmus kuriphilus, Formica* spp., *Hoplocampa* spp. e.g. *H. minuta, H. testudinea; Iridomyrmex humilis,* Lasius spp. e.g. *L. niger, Linepithema humile, Liometopum* spp., *Leptocybe invasa, Monomorium* spp. e.g. *M. pharaonis, Monomorium, Nylandria fulva, Pachycondyla chinensis, Paratrechina longicornis, Paravespula* spp., e.g. *P. germanica, P. pennsylvanica, P. vulgaris; Pheidole* spp. e.g. *P. megacephala; Pogonomyrmex* spp. e.g. *P. barbatus, P. californicus, Polistes rubiginosa, Prenolepis impairs, Pseudomyrmex gracilis, Schelipron* spp., *Sirex cyaneus, Solenopsis* spp. e.g. S. *geminata, S.invicta, S. molesta, S. richteri, S. xyloni, Sphecius speciosus, Sphex* spp., *Tapinoma* spp. e.g. *T. melanocephalum, T. sessile; Tetramorium* spp. e.g. *T. caespitum, T. bicarinatum, Vespa* spp. e.g. *V. crabro; Vespula* spp. e.g. *V. squamosal; Wasmannia auropunctata, Xylocopa* sp;
Insects from the order Orthoptera e.g. *Acheta domesticus, Calliptamus italicus, Chortoicetes terminifera, Ceuthophilus* spp., *Diastrammena asynamora, Dociostaurus maroccanus, Gryllotalpa* spp. e.g. *G. africana, G. gryllotalpa; Gryllus* spp., *Hieroglyphus daganensis, Kraussaria angulifera, Locusta* spp. e.g. *L. migratoria, L. pardalina; Melanoplus* spp. e.g. *M. bivittatus, M. femurrubrum, M. mexicanus, M. sanguinipes, M. spretus; Nomadacris septemfasciata, Oedaleus senegalensis, Scapteriscus* spp., *Schistocerca* spp. e.g. S. *americana, S. gregaria, Stemopelmatus* spp., *Tachycines asynamorus,* and *Zonozerus variegatus;*
Pests from the Class Arachnida e.g. Acari,e.g. of the families Argasidae, Ixodidae and Sarcoptidae, e.g. *Amblyomma* spp. (e.g. *A. americanum, A. variegatum, A. maculatum),* Argas spp. e.g. *A. persicu), Boophilus* spp. e.g. *B. annulatus, B. decoloratus, B. microplus, Dermacentor* spp. e.g. *D.silvarum, D. andersoni, D. variabilis, Hyalomma* spp. e.g. *H. truncatum, Ixodes* spp. e.g. *I. ricinus, I. rubicundus, I. scapularis, I. holocyclus, I. pacificus, Rhipicephalus sanguineus, Ornithodorus* spp. e.g. *O*. *moubata, O. hermsi, O. turicata, Ornithonyssus bacoti, Otobius megnini, Dermanyssus gallinae, Psoroptes* spp. e.g. *P*. *ovis, Rhipicephalus* spp. e.g. *R. sanguineus, R. appendiculatus, Rhipicephalus evertsi, Rhizoglyphus* spp., *Sarcoptes* spp. e.g.S. *Scabiei;* and Family Eriophyidae including *Aceria* spp. e.g. *A. sheldoni, A. anthocoptes, Acallitus* spp., *Aculops* spp. e.g. *A. lycopersici, A. pelekassi; Aculus* spp. e.g. *A. schlechtendali; Colomerus vitis, Epitrimerus pyri, Phyllocoptruta oleivora; Eriophytes ribis* and *Eriophyes* spp. e.g. *Eriophyes sheldoni;* Family Tarsonemidae including *Hemitarsonemus* spp., *Phytonemus pallidus and Polyphagotarsonemus latus, Stenotarsonemus* spp. *Steneotarsonemus spinki;* Family Tenuipalpidae including Brevipalpus spp. e.g. *B. phoenicis;* Family Tetranychidae including *Eotetranychus* spp., *Eutetranychus* spp., *Oligonychus* spp., *Petrobia latens, Tetranychus* spp. e.g. *T. cinnabarinus, T. evansi, T. kanzawai, T, pacificus, T. phaseulus, T. telarius* and *T. urticae; Bryobia praeti*osa; *Panonychus* spp. e.g. *P. ulmi, P. citri; Metatetranychus* spp. and *Oligonychus* spp. e.g. O. *pratensis, O. perseae, Vasates lycopersici; Raoiella indica, Family* Carpoglyphidae including *Carpoglyphus* spp.; *Penthaleidae* spp. e.g. *Halotydeus destructor,* Family Demodicidae with species e.g. *Demodex* spp.; Family Trombicidea including *Trombicula* spp.; Family Macronyssidae including *Ornothonyssus* spp.; Family Pyemotidae including *Pyemotes tritici; Tyrophagus putrescentiae;* Family Acaridae including *Acarus siro;* Family Araneida including *Latrodectus mactans, Tegenaria agrestis, Chiracanthium sp, Lycosa sp Achaearanea tepidariorum* and *Loxosceles reclusa;*
Pests from the Phylum Nematoda, e.g. plant parasitic nematodes e.g. root-knot nematodes, *Meloidogyne* spp. e.g. *M. hapla, M. incognita, M. javanica;* cyst-forming nematodes, *Globodera* spp. e.g. *G. rostochiensis; Heterodera* spp. e.g. *H. avenae, H. glycines, H. schachtii, H. trifolii;* Seed gall nematodes, *Anguina* spp.; Stem and foliar nematodes, *Aphelenchoides* spp. e.g. *A. besseyi;* Sting nematodes, *Belonolaimus* spp. e.g. *B. longicaudatus;* Pine nematodes, *Bursaphelenchus* spp. e.g. *B. lignicolus, B. xylophilus;* Ring nematodes, *Criconema* spp., *Criconemella* spp. e.g. *C. xenoplax* and *C*. *ornata;* and, *Criconemoides* spp. e.g. *Criconemoides informis; Mesocriconema* spp.; Stem and bulb nematodes, *Ditylenchus* spp. e.g. *D*. *destructor, D. dipsaci;* Awl nematodes, *Dolichodorus* spp.; Spiral nematodes, *Heliocotylenchus multicinctus;* Sheath and sheathoid nematodes, *Hemicycliophora* spp. and *Hemicriconemoides* spp.; *Hirshmanniella* spp.; Lance nematodes, *Hoploaimus* spp.; False rootknot nematodes, *Nacobbus* spp.; Needle nematodes, *Longidorus* spp. e.g. *L. elongatus;* Lesion nematodes, *Pratylenchus* spp. e.g. *P*. *brachyurus, P. neglectus, P. penetrans, P. curvitatus, P. goodeyi;* Burrowing nematodes, *Radopholus* spp. e.g. *R. similis; Rhadopholus* spp.; *Rhodopholus* spp.; Reniform nematodes, *Rotylenchus* spp. e.g. *R. robustus, R. reniformis; Scutellonema* spp.; Stubby-root nematode, *Trichodorus* spp. e.g. *T. obtusus, T. primitivus; Paratrichodorus* spp. e.g. *P. minor;* Stunt nematodes, *Tylenchorhynchus* spp. e.g. *T. claytoni, T. dubius;* Citrus nematodes, *Tylenchulus* spp. e.g. *T. semipenetrans;* Dagger nematodes, *Xiphinema* spp.; and other plant parasitic nematode species;
Insects from the order Blattodea e.g. *Macrotermes* spp. e.g. *M. natalensis; Cornitermes cumulans, Procornitermes* spp., *Globitermes sulfureus, Neocapritermes* spp. e.g. *N. opacus, N. parvus; Odontotermes spp., Nasutitermes* spp. e.g. *N. corniger, Coptotermes* spp. e.g. *C*. *formosanus, C. gestroi, C. acinaciformis; Reticulitermes* spp. e.g. *R. hesperus, R. tibialis, R. speratus, R. flavipes, R. grassei, R. lucifugus, R. virginicus; Heterotermes* spp. e.g. *H. aureus, H. longiceps, H. tenuis; Cryptotermes* spp. e.g. *C*. *brevis, C. cavifrons; Incisitermes* spp. e.g. *I. minor, I. snyderi; Marginitermes hubbardi, Kalotermes flavicollis, Neotermes* spp. e.g. *N. castaneus, Zootermopsis* spp. *e.g. Z. angusticollis, Z. nevadensis, Mastotermes* spp. e.g. *M. darwiniensis; Blatta* spp. e.g. *B. orientalis, B. lateralis; Blattella* spp. e.g. *B. asahinae, B. germanica; Rhyparobia maderae, Panchlora nivea, Periplaneta* spp. e.g. *P. americana, P. australasiae, P. brunnea, P. fuliginosa, P. japonica; Supella longipalpa, Parcoblatta pennsylvanica, Eurycotis floridana, Pycnoscelus surinamensis,*
Insects from the order Siphonoptera, e.g. *Cediopsylla simples, Ceratophyllus* spp., *Ctenocephalides* spp., e.g. *C*. *felis, C. canis, Xenopsylla cheopis, Pulex irritans, Trichodectes canis, Tunga penetrans,* and *Nosopsyllus fasciatus,*
Insects from the order Thysanura, e.g. *Lepisma saccharina, Ctenolepisma urbana,* and *Thermobia domestica,*
Pests from the class Chilopoda, e.g. *Geophilus* spp., Scutigera spp. e.g. *Scutigera coleoptrata;*
Pests from the class Diplopoda, e.g. *Blaniulus guttulatus, Julus* spp., *Narceus* spp.,
Pests from the class Symphyla, e.g. *Scutigerella immaculata,*
Insects from the order Dermaptera, e.g. *Forficula auricularia,*
Insects from the order Collembola, e.g. *Onychiurus* spp., e.g. *Onychiurus armatus,*
Pests from the order Isopoda, e.g., *Armadillidium vulgare, Oniscus asellus, Porcellio scaber,* Insects from the order Phthiraptera, e.g. *Damalinia* spp., Pediculus spp. e.g. *Pediculus humanus capitis, Pediculus humanus corporis, Pediculus humanus humanus; Pthirus pubis,* Haematopinus spp. e.g. *Haematopinus eurysternus, Haematopinus suis;* Linognathus spp. e.g. *Linognathus vituli; Bovicola bovis, Menopon gallinae, Menacanthus stramineus* and *Solenopotes capillatus, Trichodectes* spp.,
Further pest species which may be controlled by compounds I include: from the Phylum Mollusca, class Bivalvia, e.g., *Dreissena* spp.; class Gastropoda, e.g., *Arion* spp., *Biomphalaria* spp., *Bulinus* spp., *Deroceras* spp., *Galba* spp., *Lymnaea* spp., *Oncomelania* spp., *Pomacea canaliclata, Succinea* spp.; from the class of the helminths, e.g., *Ancylostoma duodenale, Ancylostoma ceylanicum, Acylostoma braziliensis, Ancylostoma* spp., *Ascaris lubricoides, Ascaris spp., Brugia malayi, Brugia timori, Bunostomum* spp., *Chabertia* spp., *Clonorchis* spp., *Cooperia* spp., *Dicrocoelium* spp., *Dictyocaulus filaria, Diphyllobothrium latum, Dracunculus medinensis, Echinococcus granulosus, Echinococcus multilocularis, Enterobius vermicularis, Faciola* spp., Haemonchus spp. e.g. *Haemonchus contortus; Heterakis* spp., *Hymenolepis nana, Hyostrongulus* spp., *Loa Loa, Nematodirus* spp., *Oesophagostomum* spp., *Opisthorchis* spp., *Onchocerca volvulus, Ostertagia* spp., *Paragonimus* spp., *Schistosomen* spp., *Strongyloides fuelleborni, Strongyloides stercora lis, Stronyloides* spp., *Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.*
The compounds of the invention are particularly suitable for efficiently combating insects from the sub-order of Auchenorrhyncha, e.g. *Amrasca biguttula, Empoasca spp., Nephotettix virescens, Sogatella furcifera, Mahanarva spp., Laodelphax striatellus, Nilaparvata lugens, Diaphorina citri;*
Lepidoptera, e.g. *Helicoverpa spp., Heliothis virescens, Lobesia botrana, Ostrinia nubilalis, Plutella xylostella, Pseudoplusia includens, Scirpophaga incertulas, Spodoptera spp., Trichoplusia ni, Tuta absoluta, Cnaphalocrocis medialis, Cydia pomonella, Chilo suppressalis, Anticarsia gemmatalis, Agrotis ipsilon, Chrysodeixis includens;*
True bugs, e.g. *Lygus spp.,* Stink bugs such as *Euschistus spp., Halyomorpha halys, Nezara viridula, Piezodorus guildinii, Dichelops furcatus;*
Thrips, e.g. *Frankliniella spp., Thrips spp., Dichromothrips corbettii;*
Aphids, e.g. *Acyrthosiphon pisum, Aphis spp., Myzus persicae, Rhopalosiphum spp., Schizaphis graminum, Megoura viciae;*
Whiteflies, e.g. *Trialeurodes vaporariorum, Bemisia spp.;*
Coleoptera, e.g. *Phyllotreta spp., Melanotus spp., Meligethes aeneus, Leptinotarsa decimlineata, Ceutorhynchus spp., Diabrotica spp., Anthonomus grandis, Atomaria linearia, Agriotes spp., Epilachna spp.;*
Flies, e.g. *Delia spp., Ceratitis capitate, Bactrocera spp., Liriomyza spp.;*
Mosquitoes (Diptera), e.g. *Aedes aegypti, A. albopictus, A. vexans, Anastrepha ludens, Anopheles maculipennis, A. crucians, A. albimanus, A. gambiae, A. freeborni, A. leucosphyrus, A. minimus, A. quadrimaculatus;*
Coccoidea, e.g. *Aonidiella aurantia, Ferrisia virgate;*
Anthropods of class Arachnida (Mites), e.g. *Penthaleus major, Tetranychus spp.;*
Nematodes, e.g. *Heterodera glycines, Meloidogyne sp., Pratylenchus spp., Caenorhabditis elegans.*

The compounds of the invention are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a method of treating or protecting animals against infestation and infection by parasites, which comprises orally, topically or parenterally administering or applying to the animals a parasiticidally effective amount of a compound of the invention.

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound of the invention.

The compounds of the invention are further suitable for use in combating or controlling parasites in and on animals. Furthermore, the invention relates to a method of combating or controlling parasites in and on animals, which comprises contacting the parasites with a parasitically effective amount of a compound of the invention.

The invention also relates to the non-therapeutic use of compounds of the invention for controlling or combating parasites. Moreover, the invention relates to a non-therapeutic method of combating or controlling parasites, which comprises applying to a locus a parasiticidally effective amount of a compound of the invention.

The compounds of the invention can be effective through both contact (via soil, glass, wall, bed net, carpet, blankets or animal parts) and ingestion (e.g. baits). Furthermore, the compounds of the invention can be applied to any and all developmental stages.

The compounds of the invention can be applied as such or in form of compositions comprising the compounds of the invention.

The compounds of the invention can also be applied together with a mixing partner, which acts against pathogenic parasites, e.g. with synthetic coccidiosis compounds, polyetherantibiotics e.g. Amprolium, Robenidin, Toltrazuril, Monensin, Salinomycin, Maduramicin, Lasalocid, Narasin or Semduramicin, or with other mixing partners as defined above, or in form of compositions comprising said mixtures.

The compounds of the invention and compositions comprising them can be applied orally, parenterally or topically, e.g. dermally. The compounds of the invention can be systemically or non-systemically effective.

The application can be carried out prophylactically, therapeutically or non-therapeutically. Furthermore, the application can be carried out preventively to places at which occurrence of the parasites is expected.

As used herein, the term "contacting" includes both direct contact (applying the compounds/compositions directly on the parasite, including the application directly on the animal or excluding the application directly on the animal, e.g. at its locus for the latter) and indirect contact (applying the compounds/compositions to the locus of the parasite). The contact of the parasite through application to its locus is an example of a non-therapeutic use of the compounds of the invention.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include lice, biting lice, ticks, nasal bots, keds, biting flies, muscoid flies, flies, myiasitic fly larvae, chiggers, gnats, mosquitoes and fleas.

The compounds of the invention are especially useful for combating parasites of the following orders and species, respectively:
fleas (Siphonaptera), e.g. *Ctenocephalides felis, C. canis, Xenopsylla cheopis, Pulex irritans, Tunga penetrans,* and *Nosopsyllus fasciatus;* cockroaches (Blattaria - Blattodea), e.g. *Blattella germanica, B. asahinae, Periplaneta americana, P. japonica, P. brunnea, P. fuligginosa,* P. *australasiae,* and *Blatta orientalis;* flies, mosquitoes (Diptera), e.g. *Aedes aegypti, A. albopictus, A. vexans, Anastrepha ludens, Anopheles maculipennis, A. crucians, A. albimanus, A. gambiae, A. freeborni, A. leucosphyrus, A. minimus, A. quadrimaculatus, Calliphora vicina, Chrysomya bezziana, C. hominivorax, C. macellaria, Chrysops discalis, C. silacea, C. atlanticus, Cochliomyia hominivorax, Cordylobia anthropophaga, Culicoides furens, Culex pipiens, C. nigripalpus, C. quinquefasciatus, C. tarsalis, Culiseta inornata, C. melanura, Dermatobia hominis, Fannia canicularis, Gasterophilus intestinalis, Glossina morsitans, G. palpalis, G. fuscipes, G. tachinoides, Haematobia irritans, Haplodiplosis equestris, Hippelates spp., Hypoderma lineata, Leptoconops torrens, Lucilia caprina, L. cuprina, L. sericata, Lycoria pectoralis, Mansonia spp., Musca domestica, M. stabulans, Oestrus ovis, Phlebotomus argentipes, Psorophora columbiae, P. discolor, Prosimulium mixtum, Sarcophaga spp., S. haemorrhoidalis, Simulium vittatum, Stomoxys calcitrans, Tabanus bovinus, T. atratus, T. lineola,* and *T. similis;* lice (Phthiraptera), e.g. *Pediculus humanus capitis, P. humanus humanus, Pthirus pubis, Haematopinus eurysternus, H. suis, Linognathus vituli, Bovicola bovis, Menopon gallinae, Menacanthus stramineus,* and *Solenopotes capillatus;* ticks and parasitic mites (Parasitiformes): ticks (Ixodida), e.g. *Ixodes scapularis, I. holocyclus, I. pacificus, Rhiphicephalus sanguineus, Dermacentor andersoni, D. variabilis, Amblyomma americanum, A. maculatum, Ornithodorus hermsi, O. turicata* and parasitic mites (Mesostigmata), e.g. *Ornithonyssus bacoti, Dermanyssus gallinae;* Actinedida (Prostigmata) and Acaridida (Astigmata), e.g. *Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp.,* and *Laminosioptes spp;* Bugs (Heteropterida): *Cimex lectularius, C. hemipterus, Reduvius senilis, Triatoma spp., Rhodnius ssp., Panstrongylus ssp.,* and *Arilus critatus;* Anoplurida, e.g. *Haematopinus spp., Linognathus spp., Pediculus spp., Phtirus spp.,* and *Solenopotes spp.;* Mallophagida (suborders Arnblycerina and Ischnocerina), e.g. *Trimenopon spp., Menopon spp., Trinoton spp., Bovicola spp., Werneckiella spp., Lepikentron spp., Trichodectes spp.,* and *Felicola spp.;* Roundworms Nematoda: Wipeworms and Trichinosis (Trichosyringida), e.g. Trichinellidae *(Trichinella spp.), (*Trichuridae*) Trichuris spp., Capillaria spp.;* Rhabditida, e.g. *Rhabditis spp., Strongyloides spp., Helicephalobus spp.;* Strongylida, e.g. *Strongylus spp., Ancylostoma spp., Necator americanus, Bunostomum spp. (Hookworm), Trichostrongylus spp., Haemonchus contortus, Ostertagia spp., Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp., Aleurostrongylus abstrusus,* and *Dioctophyma renale;* Intestinal roundworms (Ascaridida), e.g. *Ascaris lumbricoides, Ascaris suum, Ascaridia galli, Parascaris equorum, Enterobius vermicularis (Threadworm), Toxocara canis, Toxascaris leonine, Skrjabinema spp.,* and *Oxyuris equi;* Camallanida, e.g. *Dracunculus medinensis* (guinea worm); Spirurida, e.g. *Thelazia spp., Wuchereria spp., Brugia spp., Onchocerca spp., Dirofilari spp.a, Dipetalonema spp., Setaria spp., Elaeophora spp., Spirocerca lupi,* and *Habronema spp.;* Thorny headed worms (Acanthocephala), e.g. *Acanthocephalus spp., Macracanthorhynchus hirudinaceus* and *Oncicola spp.;* Planarians (Plathelminthes): Flukes (Trematoda), e.g. *Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp.,* and *Nanocyetes spp.;* Cercomeromorpha, in particular Cestoda (Tapeworms), e.g. *Diphyllobothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp.,* and *Hymenolepis spp..*

The term "animal" includes warm-blooded animals (including humans) and fish. Preferred are mammals, e.g. cattle, sheep, swine, camels, deer, horses, pigs, poultry, rabbits, goats, dogs and cats, water buffalo, donkeys, fallow deer and reindeer, and also in fur-bearing animals e.g. mink, chinchilla and raccoon, birds e.g. hens, geese, turkeys and ducks and fish e.g. fresh- and salt-water fish e.g. trout, carp and eels. Particularly preferred are domestic animals, e.g. dogs or cats.

Generally, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions e.g. desired parasiticidal effect and duration, target species, mode of application.

Generally, it is favorable to apply the compounds of the invention in total amounts of 0.5 mg/kg to 100 mg/kg per day, preferably 1 mg/kg to 50 mg/kg per day.

For oral administration to warm-blooded animals, the compounds I may be formulated as animal feeds, animal feed premixes, animal feed concentrates, pills, solutions, pastes, suspensions, drenches, gels, tablets, boluses and capsules. In addition, the compounds I may be administered to the animals in their drinking water. For oral administration, the dosage form chosen should provide the animal with 0.01 mg/kg to 100 mg/kg of animal body weight per day of the compounds I, preferably with 0.5 mg/kg to 100 mg/kg of animal body weight per day.

Alternatively, the compounds I may be administered to animals parenterally, e.g., by intraruminal, intramuscular, intravenous or subcutaneous injection. The compounds I may be dispersed or dissolved in a physiologically acceptable carrier for subcutaneous injection. Alternatively, the compounds I may be formulated into an implant for subcutaneous administration. In addition, the compounds I may be transdermally administered to animals. For parenteral administration, the dosage form chosen should provide the animal with 0.01 mg/kg to 100 mg/kg of animal body weight per day of the compounds I.

The compounds I may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds I. In addition, the compounds I may be formulated as ear tags for animals, particularly quadrupeds e.g. cattle and sheep.

Suitable preparations are:
- Solutions e.g. oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations e.g. powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further auxiliaries e.g. acids, bases, buffer salts, preservatives, and solubilizers. Suitable auxiliaries for injection solutions are known in the art. The solutions are filtered and filled sterile.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on. Solutions for use on the skin are prepared according to the state of the art and according to what is described above for injection solutions, sterile procedures not being necessary.

Gels are applied to or spread on the skin or introduced into body cavities. Gels are prepared by treating solutions which have been prepared as described in the case of the injection solutions with sufficient thickener that a clear material having an ointment-like consistency results. Suitable thickeners are known in the art.

Pour-on formulations are poured or sprayed onto limited areas of the skin, the active compound penetrating the skin and acting systemically. Pour-on formulations are prepared by dissolving, suspending or emulsifying the active compound in suitable skin-compatible solvents or solvent mixtures. If appropriate, other auxiliaries e.g. colorants, bioabsorption-promoting substances, antioxidants, light stabilizers, adhesives are added. Suitable such auxiliaries are known in the art.

Emulsions can be administered orally, dermally or as injections. Emulsions are either of the water-in-oil type or of the oil-in-water type. They are prepared by dissolving the active compound either in the hydrophobic or in the hydrophilic phase and homogenizing this with the solvent of the other phase with the aid of suitable emulsifiers and, if appropriate, other auxiliaries e.g. colorants, absorption-promoting substances, preservatives, antioxidants, light stabilizers, viscosity-enhancing substances. Suitable hydrophobic phases (oils), suitable hydrophilic phases, suitable emulsifiers, and suitable further auxiliaries for emulsions are known in the art.

Suspensions can be administered orally or topically/dermally. They are prepared by suspending the active compound in a suspending agent, if appropriate with addition of other auxiliaries e.g. wetting agents, colorants, bioabsorption-promoting substances, preservatives, antioxidants, light stabilizers. Suitable suspending agents, and suitable other auxiliaries for suspensions including wetting agents are known in the art.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form. Suitable auxiliaries for this purpose are known in the art.

The compositions which can be used in the invention can comprise generally from about 0.001 to 95% of the compound of the invention.

Ready-to-use preparations contain the compounds acting against parasites, preferably ectoparasites, in concentrations of 10 ppm to 80% by weight, preferably from 0.1 to 65% by weight, more preferably from 1 to 50% by weight, most preferably from 5 to 40% by weight.

Preparations which are diluted before use contain the compounds acting against ectoparasites in concentrations of 0.5 to 90% by weight, preferably of 1 to 50% by weight.

Furthermore, the preparations comprise the compounds of formula I against endoparasites in concentrations of 10 ppm to 2% by weight, preferably of 0.05 to 0.9% by weight, very particularly preferably of 0.005 to 0.25% by weight.

Topical application may be conducted with compound-containing shaped articles e.g. collars, medallions, ear tags, bands for fixing at body parts, and adhesive strips and foils.

Generally, it is favorable to apply solid formulations which release compounds of the invention in total amounts of 10 mg/kg to 300 mg/kg, preferably 20 mg/kg to 200 mg/kg, most preferably 25 mg/kg to 160 mg/kg body weight of the treated animal in the course of three weeks.

### A. Preparation examples

The compounds were characterized by melting point determination, by NMR spectroscopy or by the mass-to-charge ratio ([m/z]) and retention time (RT; [min]), as determined by mass spectrometry (MS) coupled with HPLC analysis (HPLC-MS = high performance liquid chromatography-coupled mass spectrometry) or LC analysis (LC-MS = liquid chromatography-coupled mass spectrometry).

Method A: HPLC: Shimadzu Nexera UHPLC + Shimadzu LCMS-2020, ESI; Column: Phenomenex Kinetex 1.7µm XB-C18 100A, 2.1x50mm; Mobile phase: A: water + 0.1%TFA; B: ACN; Temperature: 60°C; Gradient: 5% B to 100% B in 1.5min; 100% B 0.25min; Flow: 0.8mL/min to 1.0mL/min in 1.51min; MS: ESI positive; Mass range (m/z): 100-700.

Method B: LC: Shimadzu LC-30AD, ESI; Column: Kinetex EVO C18 5µm 2.1x30mm; Mobile phase: A: water + 0.04% TFA; B: ACN + 0.02% TFA; Temperature: 40°C; Gradient: 5% B to 100% B in 2.5min; 100% B to 5% B in 0.02min; 5% B for 0.5min; Flow: 0.8mL/min; MS: ESI positive; Mass range: 100-2000.

Method C: HPLC/MS: Agilent 1200 HPLC MSD:1956A single quadrupole MSD, ES-API; Column: Luna C18 2.0x50mm 5µm; Mobile phase: A: 0.04% TFA in water; B: 0.02% TFA in ACN; Temperature: 40°C; Gradient: 5% B for 0.4min; 5% B to 95% B in 2.6 min; 95% B for 1 min; 95% B to 5% B in 0.01min; 5% B for 0.5min; Flow: 1.0mL/min; MS: ES-API positive; Mass range: 50-1500.

### Example 1: preparation of N-[1-[3-(5-methyl-1,2,4-oxadiazol-3-yl)pyrazin-2-yl]ethyl]-3,5-bis(trifluoromethyl)benzamide (I-1)

### Step 1: preparation of 1-(3-chloropyrazin-2-yl)ethanamine

To a mixture of 1-(3-chloropyrazin-2-yl)ethanone (10g, 63.8mmol) and NH₄OAc (60.4g, 957.8mmol) in EtOH (500mL) were added NaBH₃CN (12.04g, 191.4mmol) and NH₃/MeOH (150mL, 7N) at 30°C. The mixture was stirred at 50°C until completion was determined by LCMS (16h). The reaction mixture was quenched with H₂O (100mL) and concentrated to remove MeOH and EtOH. 6N aq. NaOH was added to adjust the pH of the residue to 11 and the resulting mixture was extracted with EtOAc (3x100mL). The combined organic layers were dried over Na₂SO₄, filtered, and concentrated. The crude product was purified by preparative HPLC (NH₄HCO₃) to deliver 1-(3-chloropyrazin-2-yl)ethanamine (10g, 50% yield) as a white solid.

¹H-NMR (400MHz, DMSO-*d₆*): δ = 8.70 (d, J=2.4Hz, 1H), 8.47 (d, J=2.5Hz, 1H), 4.50 (q, J=6.7Hz, 1H), 1.37 (d, J=6.8Hz, 3H).

### Step 2: preparation of N-[1-(3-chloropyrazin-2-yl)ethyl]-3,5-bis(trifluoromethyl)benzamide

To a mixture of 3,5-bis(trifluoromethyl)benzoic acid (19.65g, 76.14mmol) and chloro-N,N,N',N'-tetramethylformamidinium hexafluorophosphate (21.36g, 76.14mmol) in MeCN (200mL) were added *N*-methylimidazole (16.7g, 203.2mmol) and 1-(3-chloropyrazin-2-yl)ethanamine (8.0g, 50.8mmol) at 30°C. The reaction mixture was stirred until completion was determined by TLC (PE:EtOAc=3:1, Rf =0.45; 16h). The reaction mixture was quenched with H₂O (50mL) and extracted with EtOAc (3×100mL). The combined organic layers were dried over Na₂SO₄, filtered, and concentrated. The crude product was purified by chromatographic column on silica gel (eluent: 12% EtOAc in PE) to deliver N-[1-(3-chloropyrazin-2-yl)ethyl]-3,5-bis(trifluoromethyl)benzamide (13g, 43% yield) as a yellow solid.

¹H-NMR (400MHz, DMSO-*d₆*): δ = 9.53 (d, J=6.8 Hz, 1H), 8.65 (d, J=2.5Hz, 1H), 8.55 (s, 2H), 8.46 (d, J=2.5Hz, 1H), 8.33 (s, 1H), 5.53 (dq, J=6.8Hz, J=6.9Hz, 1H), 1.56 (d, J= 6.9Hz, 3H).

### Step 3: preparation of N-[1-(3-cyanopyrazin-2-yl)ethyl]-3,5-bis(trifluoromethyl)benzamide

To a solution of N-[1-(3-chloropyrazin-2-yl)ethyl]-3,5-bis(trifluoromethyl)benzamide (1.0g, 2.51mmol) in DMF (10mL) were added Zn(CN)₂ (330mg, 2.76mmol) and Pd(PPh₃)₄ (350mg, 0.30mmol) at 20°C, and the resulting mixture was purged with N₂ for 3 min. The vial was sealed and subjected to microwave irradiation (Biotage Smith Synthesis, 130°C, 10min). Completion was determined by TLC (PE:EtOAc=3:1, Rf =0.4). The reaction mixture was filtered through a celite pad, the filter cake was washed with EtOAc (50mL), and the filtrate was concentrated. The crude product was purified by chromatographic column on silica gel (17% EtOAc in PE) to deliver N-[1-(3-cyanopyrazin-2-yl)ethyl]-3,5-bis(trifluoromethyl)benzamide (2.6g, 45% yield) as a yellow solid.

¹H-NMR (400MHz, CDCl₃): δ 8.77 (d, J=2.3Hz, 1H), 8.70 (d, J=2.4Hz, 1H), 8.28 (s, 2H), 8.04 (s, 1H), 7.37-7.28 (m, 1H), 5.84-5.73 (m, 1H), 1.74 (d, J=6.8Hz, 3H).

### Step 4: preparation of N-[1-[3-[N'-hydroxycarbamimidoyl]pyrazin-2-yl]ethyl]-3,5-bis(trifluoromethyl)benzamide

To a solution of N-[1-(3-cyanopyrazin-2-yl)ethyl]-3,5-bis(trifluoromethyl)benzamide (1.1g, 2.8mmol) in EtOH (20mL) were added HONH₂•HCl (238mg, 3.36mmol) and triethylamine (575mg, 5.6mmol) at 30°C. The resulting mixture was stirred at 80°C for 2.5h, until completion was determined by TLC (PE:EtOAc=3:1, Rf=0.3). The reaction mixture was quenched with H₂O (10mL) and extracted with EtOAc (3x20mL). The combined organic layers were dried over Na₂SO₄, filtered, and concentrated. The crude product was purified by chromatographic column on silica gel (25% EtOAc in PE) to deliver N-[1-[3-[N'-hydroxycarbamimidoyl]pyrazin-2-yl]ethyl]-3,5-bis(trifluoromethyl)benzamide (600mg, 51% yield) as a white solid.

¹H-NMR (400MHz, DMSO-*d₆*) δ = 10.21 (s, 1H), 9.42 (d, J=7.2Hz, 1H), 8.62 (d, J=2.4Hz, 1H), 8.57 (d, J=2.3Hz, 1H), 8.54 (s, 2H), 8.30 (s, 1H), 6.12 (dq, J=6.8Hz, J=7.2Hz, 1H), 5.96 (s, 2H), 1.54 (d, J=6.8Hz, 3H).

LCMS: calculated mass: 422.1; observed mass: 422.0.

### Step 5: preparation of N-[1-[3-(5-methyl-1,2,4-oxadiazol-3-yl)pyrazin-2-yl]ethyl]-3,5-bis(trifluoromethyl)benzamide (I-1)

To a mixture of N-[1-[3-[N'-hydroxycarbamimidoyl]pyrazin-2-yl]ethyl]-3,5-bis(trifluoromethyl)benzamide (400mg, 0.95mmol) and 1,1,1-trimethoxyethane (8 mL) was added TsOH (245mg, 1.4mmol) at 30°C. The resulting mixture was stirred at 80°C for 16h, until completion was determined by TLC (PE/EtOAc=1:1, Rf=0.4). The reaction mixture was quenched with H₂O (30mL) and extracted with EtOAc (3×50mL). The combined organic layers were dried over Na₂SO₄, filtered, and the filtrate was concentrated. The crude product was purified by column chromatography on silica gel (27% EtOAc in PE) to deliver N-[1-[3-(5-methyl-1,2,4-oxadiazol-3-yl)pyrazin-2-yl]ethyl]-3,5-bis(trifluoromethyl)benzamide (I-1, 195mg, 46% yield) as a yellow oil.

¹H-NMR (400MHz, CDCl₃) δ = 8.78 (d, J=2.3Hz, 1H), 8.73 (d, J=2.3Hz, 1H), 8.27 (s, 2H), 8.02 (s, 1H), 7.70 (br d, J=7.3Hz, 1H), 6.37-6.26 (m, 1H), 2.78 (s, 3H), 1.68 (d, J=6.6Hz, 3H). LCMS: calculated mass: 446.1; observed mass: 446.1.

### Example 2: preparation of 3-[3-[1-[[3,5-bis(trifluoromethyl)benzoyl]amino]ethyl]pyrazin-2-yl]-N-ethyl-N-methyl-1,2,4-oxadiazole-5-carboxamide (I-2)

### Step 1: preparation of methyl 3-[3-[1-[[3,5-bis(trifluoromethyl)benzoyl]amino]ethyl]pyrazin-2-yl]-1,2,4-oxadiazole-5-carboxylate

To a solution of N-[1-[3-[(Z)-N'-hydroxycarbamimidoyl]pyrazin-2-yl]ethyl]-3,5-bis(trifluoromethyl)benzamide (500mg, 1.19mmol) in dioxane (3mL) and CHCl₃ (3mL) were added triethylamine (360mg, 3.56mmol) and subsequently methyl 2-chloro-2-oxo-acetate (436mg, 3.56mmol) dropwise at 0°C. The resulting mixture was stirred at 20°C for 4h, until disappearance of the starting material was determined by TLC (PE/EtOAc=1:1, Rf=0.5). Subsequently, the mixture was stirred at 80°C for further 16h until completion was determined by TLC (PE/EtOAc=1:1, Rf=0.5). The reaction mixture was quenched with H₂O (30mL) and extracted with EtOAc (50mL × 3). The combined organic layers were dried over Na₂SO₄, filtered, and the filtrate was concentrated. The crude product was purified by column chromatography on silica gel (25% EtOAc in PE) to deliver methyl 3-[3-[1-[[3,5-bis(trifluoromethyl)benzoyl]amino]ethyl]pyrazin-2-yl]-1,2,4-oxadiazole-5-carboxylate (375mg, 64% yield) as a yellow solid.

¹H-NMR (400MHz, CDCl₃) δ = 8.82 (d, J=2.4 Hz, 1H), 8.78 (d, J=2.4 Hz, 1H), 8.25 (s, 2H), 8.03 (s, 1H), 7.59 (br d, J=7.9 Hz, 1H), 6.29-6.22 (m, 1H), 4.14 (s, 3H), 1.71 (d, J=6.7 Hz, 3H).

### Step 2: preparation of 3-[3-[1-[[3,5-bis(trifluoromethyl)benzoyl]amino]ethyl]pyrazin-2-yl]-N-ethyl-N-methyl-1,2,4-oxadiazole-5-carboxamide (I-2)

To a solution of methyl 3-[3-[1-[[3,5-bis(trifluoromethyl)benzoyl]amino]ethyl]pyrazin-2-yl]-1,2,4-oxadiazole-5-carboxylate (275mg, 0.56mmol) in EtOH (1.5mL) was added N-methylethanamine (1.5mL) at 0°C. The resulting mixture was stirred at 0°C for 2h, until completion was determined by TLC (PE/EtOAc=1:1, Rf=0.5). The volatiles were removed under reduced pressure and the residue subjected to column chromatography on silica gel (23% EtOAc in PE) to furnish 3-[3-[1-[[3,5-bis(trifluoromethyl)benzoyl]amino]ethyl]pyrazin-2-yl]-N-ethyl-N-methyl-1,2,4-oxadiazole-5-carboxamide (I-2, 200mg, 35% yield) as a yellow solid.

¹H-NMR (400MHz, CDCl₃) δ = 8.82-8.76 (m, 2H), 8.26 (s, 2H), 8.02 (s, 1H), 7.67 (br m, 1H), 6.37-6.28 (m, 1H), 3.78-3.58 (m, 2H), 3.35-3.18 (m, 3H), 1.69 (m, 3H), 1.42-1.27 (m, 3H). LCMS: calculated mass: 517.1; observed mass: 517.1.

### Example 3: preparation of N-(cyclopropylmethyl)-N-[1-[3-(5-methyl-1,2,4-oxadiazol-3-yl)pyra-zin-2-yl]ethyl]-3,5-bis(trifluoromethyl)benzamide (I-3)

### Step 1: preparation of 1-(3-chloropyrazin-2-yl)-N-(cyclopropylmethyl)ethanamine

To a solution of 1-(3-chloropyrazin-2-yl)ethanone (5g, 31.93mmol) and cyclopropylmethanamine (2.5g, 35.13mmol) in THF (50mL) was added Ti(OEt)₄ (10.93g, 47.90mmol) at 20°C under N₂ and the resulting mixture was stirred at 80°C for 16h, after which time quantitative formation of the imine intermediate was determined by TLC (PE:EtOAc=3:1, Rf=0.8). After cooling the reaction mixture to room temperature, EtOH (10mL) and NaBH₃CN (4.02g, 63.86mmol) were added. The resulting mixture was stirred at 28°C for 16h, at which time completion was determined by LCMS. After addition of H₂O (30mL), the pH of the mixture was adjusted to 11 by addition of aq. NaOH (6N), and the mixture was extracted with EtOAc (3×80mL). The combined organic layers were dried over Na₂SO₄, filtered, and concentrated to furnish 1-(3-chloropyrazin-2-yl)-N-(cyclopropylmethyl)ethanamine (5.7g, crude) as a yellow oil. The crude product was employed in the next step without further purification.

¹H-NMR (400MHz, DMSO-*d₆*): δ = 8.68 (d, J=2.4Hz, 1H), 8.40 (d, J=2.4 Hz, 1H), 4.24 (q, *J*=6.7Hz, 1H), 2.35 (dd, J=6.2Hz, J=11.7Hz, 1H), 2.05 (dd, *J*=7.1Hz, J=11.7Hz, 1H), 1.26 (d, *J*=6.8Hz, 3H), 0.82-0.71 (m, 1H), 0.36-0.28 (m, 2H), 0.01--0.10 (m, 2H).

### Step 2: preparation of N-[1-(3-chloropyrazin-2-yl)ethyl]-N-(cyclopropylmethyl)-3,5-bis(trifluoromethyl)benzamide

To a mixture of 3,5-bis(trifluoromethyl)benzoic acid (6.3g, 24.56mmol) and chloro-N,N,N',N',-tetramethylformamidinium hexafluorophosphate (6.9g, 24.56mmol) in MeCN (100mL) was added N-methylimidazole (6.1g, 24.56mmol) and 1-(3-chloropyrazin-2-yl)-N-(cyclopropylmethyl)ethanamine (5.2g, 24.56mmol) at 30°C. The resulting mixture was stirred at 30°C for 16h, after which time completion was determined by TLC (PE: EtOAc=3:1, Rf=0.5). The reaction mixture was poured into H₂O (20mL), and the resulting mixture was extracted with EtOAc (3x50mL). The combined organic layers were washed with brine (20mL), dried over Na₂SO₄, filtered, and concentrated. The crude product was purified by chromatographic column on silica gel (5% EtOAc in PE) to deliver N-[1-(3-chloropyrazin-2-yl)ethyl]-N-(cyclopropylmethyl)-3,5-bis(trifluoromethyl)benzamide (4.8g, 43% yield) as a yellow solid.

¹H-NMR (400MHz, DMSO-d₆): δ = 8.71 (d, J=2.3Hz, 1H), 8.51 (d, J=1.9Hz, 1H), 8.25 (s, 1H), 8.10 (s, 2H), 6.13-4.86 (m, 1H), 3.15-2.86 (m, 2H), 1.66 (br s, 3H), 0.38-0.00 (m, 3H), 0.13-0.67 (m, 2H).

### Step 3: preparation of N-[1-(3-cyanopyrazin-2-yl)ethyl]-N-(cyclopropylmethyl)-3,5-bis(trifluoromethyl)benzamide

To a solution of N-[1-(3-chloropyrazin-2-yl)ethyl]-N-(cyclopropylmethyl)-3,5-bis(trifluoromethyl)benzamide (1.0g, 2.21mmol) in DMF (10mL) were added Zn(CN)₂ (286mg, 2.43mmol) and Pd(PPh₃)₄ (307mg, 0.27mmol) at 20°C. The resulting mixture was purged with N₂ for 3 min. The vial was sealed and subjected to microwave irradiation on a Biotage Smith Synthesis at 130°C for 20min, after which time completion was determined by TLC (PE: EtOAc=3:1, Rf=0.3). The mixture was filtered over diatomite and the filter cake was washed with EtOAc (50 mL). The filtrate was concentrated. The crude product was purified by column chromatography on silica gel (16% EtOAc in PE) to deliver N-[1-(3-cyanopyrazin-2-yl)ethyl]-N-(cyclopropylmethyl)-3,5-bis(trifluoromethyl)benzamide (3.9g, 85% yield) as a yellow solid.

¹H-NMR (400MHz, CDCl₃): δ = 8.78 (d, J=2.4Hz, 1H), 8.64 (d, J=2.3 Hz, 1H), 7.99-7.93 (m, 3H), 5.82-5.45 (m, 1H), 3.42 (dd, J=5.8Hz, J=15.3Hz, 1H), 3.31-3.22 (m, 1H), 1.91 (br d, J=6.5Hz, 3H), 0.98-0.88 (m, 1H), 0.60-0.44 (m, 2H), 0.06 (br d, J=15.5Hz, 1H), -0.09--0.20 (m, 1H).

### Step 4: preparation of N-(cyclopropylmethyl)-N-[1-[3-[(Z)-N'-hydroxycarbamimidoyl]pyrazin-2-yl]ethyl]-3,5-bis(trifluoromethyl)benzamide

To a mixture of N-[1-(3-cyanopyrazin-2-yl)ethyl]-N-(cyclopropylmethyl)-3,5-bis(trifluoromethyl)benzamide (3.43g, 7.75mmol) in EtOH (35mL) were added HONH₂xHCl (652mg, 9.38mmol) and triethylamine (1.6g, 15.81mmol) at 30°C. The mixture was stirred at 80°C for 2.5h, after which time completion was determined by TLC (PE: EtOAc=1:1, Rf=0.6). The reaction mixture was quenched with H₂O (20mL) and the resulting solution was extracted with EtOAc (3×50mL). The combined organic layers were dried over Na₂SO₄, filtered, and concentrated. The crude product was purified by chromatographic column on silica gel (25% EtOAc in PE) to furnish N-(cyclopropylmethyl)-N-[1-[3-[(Z)-N'-hydroxycarbamimidoyl]pyrazin-2-yl]ethyl]-3,5-bis(trifluoromethyl)benzamide (3.7g, 95% yield) as a white solid.

¹H-NMR (400MHz, DMSO-d₆): δ = 9.68 (s, 1H), 8.64 (d, J=2.3 Hz, 1H), 8.57 (d, J=2.3 Hz, 1H), 8.05 (s, 1H), 7.86 (br s, 2H), 6.08-5.98 (m, 1H), 5.63 (br s, 2H), 3.41-3.29 (m, 1H), 3.28-3.19 (m, 1H), 1.69 (d, J=6.8 Hz, 3H), 0.98-0.86 (m, 1H), 0.45-0.32 (m, 2H), 0.08--0.13 (m, 2H).

### Step 5: preparation of N-(cyclopropylmethyl)-N-[1-[3-(5-methyl-1,2,4-oxadiazol-3-yl)pyrazin-2-yl]ethyl]-3,5-bis(trifluoromethyl)benzamide (I-3)

To a solution of N-(cyclopropylmethyl)-N-[1-[3-[(Z)-N'-hydroxycarbamimidoyl]pyrazin-2-yl]ethyl]-3,5-bis(trifluoromethyl)benzamide (600mg, 1.26mmol) in 1,1,1-trimethoxyethane (6 mL) was added TsOH (22mg, 0.12mmol) at 25°C. The resulting mixture was stirred at 80°C for 6h after which time completion was determined by LCMS. The reaction mixture was quenched with water (10mL) and extracted with EtOAc (3×20mL). The combined organic layer was washed with brine (10mL), dried over Na₂SO₄, filtered, and concentrated. The crude product was purified by preparative HPLC (NH₄HCO₃, MeCN-H₂O) to deliver N-(cyclopropylmethyl)-N-[1-[3-(5-methyl-1,2,4-oxadiazol-3-yl)pyrazin-2-yl]ethyl]-3,5-bis(trifluoromethyl)benzamide (350mg, 56% yield) as a thick yellow oil.

¹H-NMR (400MHz, DMSO-d₆): δ = 8.87 (d, J=2.3Hz, 1H), 8.77 (d, J=2.4Hz, 1H), 8.09 (s, 1H), 7.83 (s, 2H), 5.91 (br d, 1H), 3.23 (br s, 2H), 2.62 (s, 3H), 1.72 (d, J=7.0Hz, 3H), 0.89-0.68 (m, 1H), 0.46-0.18 (m, 2H), 0.07--0.23 (m, 2H).

### Example 4: preparation of 3-[3-[1-[[3,5-bis(trifluoromethyl)benzoyl]-(cyclopropylmethyl)amino]ethyl]pyrazin-2-yl]-N-methyl-1,2,4-oxadiazole-5-carboxamide (I-4)

### Step 1: preparation of methyl 3-[3-[1-[[3,5-bis(trifluoromethyl)benzoyl]-(cyclopropylmethyl)amino]ethyl]pyrazin-2-yl]-1,2,4-oxadiazole-5-carboxylate

To a solution of N-(cyclopropylmethyl)-N-[1-[3-[(Z)-N'-hydroxycarbamimidoyl]pyrazin-2-yl]ethyl]-3,5-bis(trifluoromethyl)benzamide (530mg, 1.13mmol) in 1,4-dioxane (3mL) and CHCl₃ (3mL) was added triethylamine (337.58mg, 3.35mmol). Subsequently, methyl 2-chloro-2-oxo-acetate (407.3mg, 3.35mmol) was added dropwise at 0°C under N₂. The resulting mixture was stirred at 25°C for 12h, and then stirred at 80°C for 32h, after which time completion was determined by LCMS. The reaction mixture was quenched with water (10mL) and extracted with EtOAc (2×20mL). The combined organic layer was washed with brine (30mL), dried over Na₂SO₄, filtered, and concentrated. The crude product was purified by chromatographic column on silica gel (EtOAc in PE = 0% to 100%) to deliver methyl 3-[3-[1-[[3,5-bis(trifluoromethyl)benzoyl]-(cyclopropylmethyl)amino]ethyl]pyrazin-2-yl]-1,2,4-oxadiazole-5-carboxylate (190mg, 31% yield) as a thick yellow oil.

¹H-NMR (400MHz, CDCl₃): δ = 8.78-8.79 (m, 1H), 8.76 (s, 1H), 7.79-7.89 (m, 3H), 6.14-6.36 (m, 1H), 4.07 (s, 3H), 3.24-3.56 (m, 1H), 2.91-3.23 (m, 1H), 1.81-1.82 (m, 3H), 0.65-0.83 (m, 1H), 0.32-0.48 (m, 4H).

### Step 2: preparation of 3-[3-[1-[[3,5-bis(trifluoromethyl)benzoyl]-(cyclopropylmethyl)amino]ethyl]pyrazin-2-yl]-N-methyl-1,2,4-oxadiazole-5-carboxamide (I-4)

To a solution of 3-[3-[1-[[3,5-bis(trifluoromethyl)benzoyl]-(cyclopropylmethyl)amino]ethyl]pyrazin-2-yl]-1,2,4-oxadiazole-5-carboxylate (118mg, 0.218mmol) in THF (3mL) was added MeNH₂ (30% in EtOH, 67mg, 0.653mmol) at 25°C. The resulting mixture was stirred at 25°C for 6h, after which time completion was determined by TLC (PE:EtOAc = 1:1). The reaction mixture was concentrated and the residue purified by preparative TLC (PE:EtOAc=1:1) to deliver 3-[3-[1-[[3,5-bis(trifluoromethyl)benzoyl]-(cyclopropylmethyl)amino]ethyl]pyrazin-2-yl]-N-methyl-1,2,4-oxadiazole-5-carboxamide (110mg, 93% yield) as a white solid.

¹H-NMR (400MHz, DMSO-d₆): δ = 9.11 (br s, 1H), 8.91 (d, J=2.32Hz, 1H), 8.81 (d, J=2.32Hz, 1H), 8.05 (s, 1H), 7.83 (s, 2H), 6.03 (br dd, 1H), 3.20-3.31 (m, 2H), 2.84 (d, J=4.77Hz, 3H), 1.74 (d, J=6.85Hz, 3H), 0.68-0.84 (m, 1H), 0.19-0.43 (m, 2H), -0.33--0.03 (m, 2H).

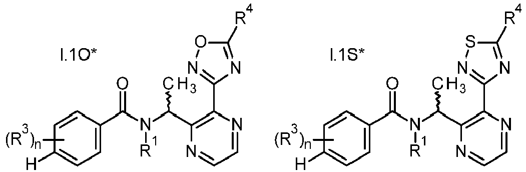

**Table I - compounds of formula I**

| No. | Formula | R¹ | R³ | R⁴ | Method | phys. data (HPLC RT [min]) | M+H [m/z] |
|---|---|---|---|---|---|---|---|
| I-1 | I.1O* | H | 3,5-(CF₃)₂ | CH₃ | B | 1.586 | 446.1 |
| I-2 | I.1O* | H | 3,5-(CF₃)₂ | C(O)N(CH₃)C₂H₅ | B | 1.633 | 517.1 |
| I-3 | I.1O* | CH₂-cC₃H₅ | 3,5-(CF₃)₂ | CH₃ | C | 2.962 | 500.0 |
| I-4 | I.1O* | CH₂-cC₃H₅ | 3,5-(CF₃)₂ | C(O)N(CH₃)C₂Hₛ | B | 1.627 | 543.2 |
| I-5 | I.1S* | H | 3-Cl,5-(SO₂CH₃) | C(O)NH(CH₃) | C | 2.133 | 480.9 |

### Biological examples

If not otherwise specified, the test solutions were prepared as follow:
The active compound was dissolved at the desired concentration in a mixture of 1:1 (vol:vol) distilled water: acetone. The test solution was prepared on the day of use.

The activity of the compounds of formula I of the present invention can be demonstrated and evaluated by the following biological tests.

### B.1 Diamond back moth (Plutella xylostella)

The active compound was dissolved at the desired concentration in a mixture of 1:1 (vol:vol) distilled water: acetone. Surfactant (Kinetic HV) was added at a rate of 0.01% (vol/vol). The test solution was prepared on the day of use.

Leaves of cabbage were dipped in test solution and air-dried. Treated leaves were placed in petri dishes lined with moist filter paper and inoculated with ten 3^{rd} instar larvae. Mortality was recorded 72 hours after treatment. Feeding damages were also recorded using a scale of 0-100%.

In this test, compounds I-1, I-2, and I-3, resp., at 300 ppm showed at least 75% mortality in comparison with untreated controls.

### B.2 Green Peach Aphid (Myzus persicae)

For evaluating control of green peach aphid *(Myzus persicae)* through systemic means the test unit consisted of 96-well-microtiter plates containing liquid artificial diet under an artificial mem brane.

The compounds were formulated using a solution containing 75% v/v water and 25% v/v DMSO. Different concentrations of formulated compounds were pipetted into the aphid diet, using a custom built pipetter, at two replications.

After application, 5-8 adult aphids were placed on the artificial membrane inside the microtiter plate wells. The aphids were then allowed to suck on the treated aphid diet and incubated at about 23 ± 1°C and about 50 ± 5 % relative humidity for 3 days. Aphid mortality and fecundity was then visually assessed.

In this test, compounds I-1 and I-3, resp., at 2500 ppm showed at least 75% mortality in comparison with untreated controls.

### B.3 Tobacco budworm (Heliothis virescens)

For evaluating control of tobacco budworm *(Heliothis virescens)* the test unit consisted of 96-well-microtiter plates containing an insect diet and 15-25 *H. virescens* eggs.

The compounds were formulated using a solution containing 75% v/v water and 25% v/v DMSO. Different concentrations of formulated compounds were sprayed onto the insect diet at 10 µl using a custom-built micro atomizer, at two replications.

After application, microtiter plates were incubated at about 28 ± 1°C and about 80 ± 5 % relative humidity for 5 days. Egg and larval mortality was then visually assessed.

In this test, compounds 1-1, I-2, and I-4, resp., at 2500 ppm showed at least 75% mortality in comparison with untreated controls.

### B.4 Boll weevil (Anthonomus grandis)

For evaluating control of boll weevil *(Anthonomus grandis)* the test unit consisted of 96-well-microtiter plates containing an insect diet and 5-10 *A. grandis* eggs.

The compounds were formulated using a solution containing 75% v/v water and 25% v/v DMSO. Different concentrations of formulated compounds were sprayed onto the insect diet at 5µl, using a custom-built micro atomizer, at two replications.

After application, microtiter plates were incubated at about 25 ± 1°C and about 75 ± 5 % relative humidity for 5 days. Egg and larval mortality was then visually assessed.

In this test, compounds 1-1, I-2, I-3, and I-4, resp., at 2500 ppm showed at least 75% mortality in comparison with untreated controls.

### B.5. Southern armyworm (Spodoptera eridania), 2nd instar larvae

The active compounds were formulated by a Tecan liquid handler in 100% cyclohexanone as a 10,000 ppm solution supplied in tubes. The 10,000 ppm solution was serially diluted in 100% cyclohexanone to make interim solutions. These served as stock solutions for which final dilutions were made by the Tecan in 50% acetone:50% water (v/v) into 10 or 20ml glass vials. A nonionic surfactant (Kinetic^{®}) was included in the solution at a volume of 0.01% (v/v). The vials were then inserted into an automated electrostatic sprayer equipped with an atomizing nozzle for application to plants/insects. Lima bean plants (variety Sieva) were grown 2 plants to a pot and selected for treatment at the 1st true leaf stage. Test solutions were sprayed onto the foliage by an auto-mated electrostatic plant sprayer equipped with an atomizing spray nozzle. The plants were dried in the sprayer fume hood and then removed from the sprayer. Each pot was placed into perforated plastic bags with a zip closure. Ten to 11 armyworm larvae were placed into the bag and the bags zipped closed. Test plants were maintained in a growth room at about 25°C and about 20-40% relative humidity for 4 days, avoiding direct exposure to fluorescent light (14:10 light:dark photoperiod) to prevent trapping of heat inside the bags. Mortality and reduced feeding were assessed 4 days after treatment, compared to untreated control plants.

In this test, compounds I-1 and I-3, resp., at 300 ppm showed at least 75% mortality in comparison with untreated controls.

### B.6 Orchid thrips (Dichromothrips corbetti)

*Dichromothrips corbetti* adults used for bioassay were obtained from a colony maintained continuously under laboratory conditions. For testing purposes, the test compound is diluted in a 1:1 mixture of acetone:water (vol:vol), plus Kinetic HV at a rate of 0.01% v/v.

Thrips potency of each compound was evaluated by using a floral-immersion technique. All petals of individual, intact orchid flowers were dipped into treatment solution and allowed to dry in Petri dishes. Treated petals were placed into individual re-sealable plastic along with about 20 adult thrips. All test arenas were held under continuous light and a temperature of about 28°C for duration of the assay. After 3 days, the numbers of live thrips were counted on each petal. The percent mortality was recorded 72 hours after treatment.

In this test, compound I-3 at 300 ppm showed at least 75% mortality in comparison with untreated controls.

## Claims

1. Compounds of formula I 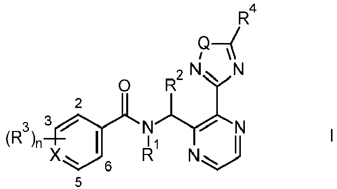 wherein
R¹ is H, OH, C₁-C₆-alkyl, C₁-C₆-haloalkyl, C₃-C₆-cycloalkyl, C₃-C₆-halocycloalkyl, C₁-C₅-alkoxy, C₁-C₄-alkyl-C₃-C₆-cycloalkyl, C₁-C₄-alkyl-C₃-C₆-halocycloalkyl, which groups are unsubstituted, or partially or fully substituted with R¹¹;
R¹¹ is halogen, CN, NO₂, NR¹²R¹³, C(O)NH₂, C(S)NH₂, C(O)OH, OR¹⁴, Si(CH₃)₃; C₁-C₆-alkyl; C₁-C₆-haloalkyl; C₂-C₆-alkenyl; C₂-C₆-haloalkenyl; C₂-C₆-alkynyl; C₂-C₆-haloalkynyl; C₃-C₄-cycloalkyl-C₁-C₂-alkyl, which ring is unsubstituted or substituted with 1 or 2 halogen; 3- to 6-membered heterocyclyl, 5- or 6-membered hetaryl, or phenyl, which rings are unsubstituted or substituted with halogen, C₁-C₃-haloalkyl, and/or CN;
R¹², R¹³ are independently from each other H, C₁-C₄-alkyl, C₁-C₄-alkoxy, C₁-C₄-haloalkoxy, C₁-C₄-haloalkyl, C₃-C₆-cycloalkyl, C(O)-C₁-C₄-alkyl, C(O)-C₁-C₄-haloalkyl, C(O)-C₃-C₄-cycloalkyl, C(O)-C₃-C₄-halocycloalkyl, C(O)NH-C₁-C₄-alkyl, C(O)NH-C₁-C₄-haloalkyl, C(O)N(C₁-C₄-alkyl)-C₁-C₄-alkyl, C(O)N(C₁-C₄-haloalkyl)-C₁-C₄-alkyl, C(O)N(C₁-C₄-haloalkyl)-C₁-C₄-haloalkyl, C(O)NH-C₁-C₄-alkoxy, C(O)NH-C₁-C₄-haloalkoxy, C(O)NH-C₁-C₄-alkoxy-C₁-C₄-alkyl, C(O)NH-C₁-C₄-alkoxy-C₁-C₄-haloalkyl; C(O)NH-phenyl, C(O)NH-3-6-membered heterocyclyl or 5- or 6-membered hetaryl, C(O)NH-C₁-C₄-alkyl-phenyl, C(O)NH-C₁-C₄-alkyl-3-6-membered heterocyclyl or 5- or 6-membered hetaryl, which rings are unsubstituted or substituted with halogen, C₁-C₃-haloalkyl, and/or CN; S(O)m-C₁-C₄-haloalkyl, S(O)ₘ-C₃-C₆-cycloalkyl, S(O)ₘ-C₃-C₆-halocycloalkyl; 3- to 6-membered heterocyclyl, 5- or 6-membered hetaryl, or phenyl, which rings are unsubstituted or substituted with halogen, C₁-C₃-haloalkyl, and/or CN;
or R¹² and R¹³ together with the nitrogen atom they are bound to form a 3-6 membered saturated, partially or fully unsaturated heterocycle, which may contain 1 or 2 additional heteroatoms selected from N, O, and S, wherein S may be oxidized, and which heterocycle is unsubstituted or substituted with R³;
or R¹² and R¹³ together with the nitrogen atom they are bound to form a group N=S(=O)R^{14a}R^{14b}, wherein R^{14a} and R^{14b} are defined as R¹⁴;
R¹⁴ is H, C₁-C₄-alkyl, C₁-C₄-haloalkyl, C₃-C₆-cycloalkyl, C₃-C₆-halocycloalkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halo-cycloalkyl, or phenyl which is unsubstituted or partially or fully substituted with R³;
R² m is 0, 1, or 2; is H, CN, C₁-C₃-alkyl, C₁-C₃-haloalkyl, C₂-C₃-alkenyl, or C₂-C₃-alkynyl;
R³ is halogen, CN, NO₂, C₁-C₄-alkyl, C₃-C₆-cycloalkyl, C₁-C₆-haloalkyl, C₁-C₆-halocycloalkyl, OR¹⁴, S(O)ₘ-R¹⁴; wherein rings are unsubstituted or substituted with R¹¹;
n is 0, 1, 2, or 3;
R⁴ is H, halogen, C₁-C₆-alkyl, C₃-C₆-cycloalkyl, C₁-C₆-haloalkyl, C₁-C₆-halocycloalkyl, C₂-C₄-alkenyl, C₂-C₄-haloalkenyl, C₂-C₄-alkynyl, each optionally substituted with R⁴¹; S(O)m-C₁-C₄-alkyl, S(O)m-C₁-C₄-haloalkyl, S(O)ₘ-C₃-C₆-cycloalkyl, S(O)ₘ-C₃-C₆-halocycloalkyl, S(O)₂NR¹²R¹³, NR¹²R¹³, C(O)NR¹²R¹³, C(O)OR¹⁴, 3- to 6-membered heterocyclyl, 5- or 6-membered hetaryl, or phenyl, which rings are unsubstituted or substituted with R³;
R⁴¹ is H, OR¹⁵, NR¹²R¹³, C₁-C₄-alkyl, C₁-C₄-haloalkyl, C₃-C₆-cycloalkyl, C(O)-C₁-C₄-alkyl, C(O)-C₁-C₄-haloalkyl, C(O)-C₃-C₄-cycloalkyl, C(O)-C₃-C₄-halocycloalkyl, C(O)NH-C₁-C₄-alkyl, C(O)NH-C₁-C₄-haloalkyl, C(O)N(C₁-C₄-alkyl)-C₁-C₄-alkyl, C(O)N(C₁-C₄-haloalkyl)-C₁-C₄-alkyl, C(O)N(C₁-C₄-haloalkyl)-C₁-C₄-haloalkyl, C(O)NH-C₁-C₄-alkoxy, C(O)NH-C₁-C₄-haloalkoxy, C(O)NH-C₁-C₄-alkoxy-C₁-C₄-alkyl, C(O)NH-C₁-C₄-alkoxy-C₁-C₄-haloalkyl; C(O)NH-phenyl, C(O)NH-3-6-membered heterocyclyl or 5- or 6-membered hetaryl, C(O)NH-C₁-C₄-alkyl-phenyl, C(O)NH-C₁-C₄-alkyl-3-6-membered heterocyclyl or 5- or 6-membered hetaryl, which rings are unsubstituted or substituted with halogen, C₁-C₃-haloalkyl, and/or CN; S(O)ₘ-C₁-C₄-haloalkyl, S(O)ₘ-C₃-C₄-cycloalkyl, S(O)ₘ-C₃-C₄-halocycloalkyl; 3- to 6-membered heterocyclyl, 5- or 6-membered hetaryl, or phenyl, which rings are unsubstituted or substituted with halogen, C₁-C₃-haloalkyl, and/or CN;
R¹⁵ is H, C₁-C₄-alkyl, or C₁-C₄-haloalkyl, C₃-C₆-cycloalkyl, C₁-C₆-halocycloalkyl, which carbon chains are unsubstituted or partially or fully substituted with R¹¹; or 3- to 6-membered heterocyclyl, 5- or 6-membered hetaryl, or phenyl, which rings are unsubstituted or substituted with R³;
Q is O or S;
and the N-oxides, stereoisomers, and agriculturally or veterinarily acceptable salts thereof.

2. Compounds of formula I according to claim 1, wherein R¹ is H or CH₂-cC₃H₅.

3. Compounds of formula I according to claim 1 or 2, wherein R² is CH₃.

4. Compounds of formula I according to any of claim 1 to 3, wherein R³ is halogen, CN, C₁-C₄-haloalkyl, C₁-C₄-hal0alkoxy, C₃-C₄-cycloalkyl unsubstituted or substituted with one or more CN, C₃-C₄-halocycloalkyl, S(O)ₘ-C₁-C₄-alkyl, S(O)ₘ-C₁-C₄-haloalkyl, S(O)ₘ-C₃-C₄-cycloalkyl, S(O)ₘ-C₃-C₄-halocycloalkyl, or S(O)ₘ-R¹⁴, wherein R¹⁴ is phenyl, which is partially substituted with R¹¹.

5. Compounds of formula I according to any of claim 1 to 4, wherein n is 2 and R³ is in positions 3 and 5.

6. Compounds of formula I according to any one of claims 1 to 5, wherein X is CH.

7. Compounds of formula I according to any one of claims 1 to 6, wherein R⁴ is H, C₁-C₃-alkyl, C₁-C₃-haloalkyl, C₁-C₄-alkoxy-C₁-C₄-alkyl, C(O)NH-C₁-C₆-alkyl, C(O)N(C₁-C₆-alkylh, S(O)ₘ-C₁-C₄-alkyl, or phenyl unsubstituted or substituted with one or more groups R³ as defined in claim 4.

8. Compounds of formula I according to any one of the preceding claims, which correspond to formula I.O

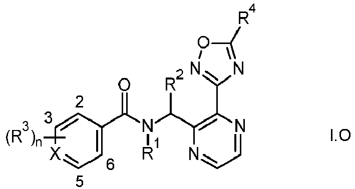

9. Compounds of formula I according to any one of claims 1 to 7, which correspond to formula I.S

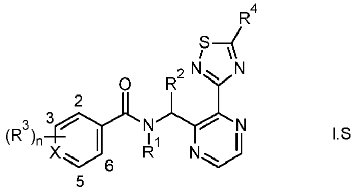

10. Compounds of formula I according to any one of the preceding claims, which consist mainly of the isomer I.A.

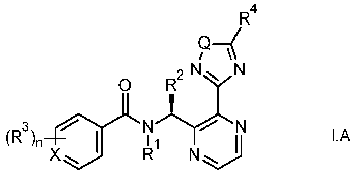

11. An agricultural or veterinary composition comprising at least one compound according to any one of claims 1 to 10 and/or at least one agriculturally or veterinarily acceptable salt thereof, and at least one inert liquid and/or solid agriculturally or veterinarily acceptable carrier.

12. An agricultural composition for combating animal pests comprising at least one compound as defined in any of claims 1 to 10 and at least one inert liquid and/or solid acceptable carrier and, if desired, at least one surfactant.

13. A method for combating or controlling invertebrate pests, which method comprises contacting said pest or its food supply, habitat or breeding grounds with a pesticidally effective amount of at least one compound as defined in any one of claims 1 to 10.

14. A method for protecting growing plants from attack or infestation by invertebrate pests, which method comprises contacting a plant, or soil or water in which the plant is growing, with a pesticidally effective amount of at least one compound as defined in any of claims 1 to 10.

15. Seed comprising a compound as defined in any of claims 1 to 10, or the enantiomers, diastereomers or salts thereof, in an amount of from 0.1 g to 10 kg per 100 kg of seed.

16. A method for treating or protecting an animal from infestation or infection by invertebrate pests which comprises bringing the animal in contact with a pesticidally effective amount of at least one compound of the formula I as defined in any of claims 1 to 10, a stereoisomer thereof and/or at least one veterinarily acceptable salt thereof.
